# EUROPÄISCHE PATENTSCHRIFT

(11) **EP 4 153 087 B1**
(45) Veröffentlichungstag und Bekanntmachung des Hinweises auf die Patenterteilung: **24.04.2024**
(21) Anmeldenummer: 20727270.9
(22) Anmeldetag: 19.05.2020
(51) Int. Cl.: A61C 1/08

(54) **POSITIONIERUNGSELEMENT ZUM POSITIONIEREN UND FIXIEREN EINER BOHRSCHABLONE**
POSITIONING ELEMENT FOR POSITIONING AND FIXING A DRILLING TEMPLATE
ÉLÉMENT DE POSITIONNEMENT POUR POSITIONNER ET FIXER UN GABARIT DE PERÇAGE

(43) Veröffentlichungstag der Anmeldung: 29.03.2023
(73) Patentinhaber: Exocad GmbH, 64293 Darmstadt (DE)
(72) Erfinder: RÜHL, Sebastian, 64293 Darmstadt (DE); SCHNITZSPAN, Paul, 64293 Darmstadt (DE)
(74) Vertreter: Richardt Patentanwälte PartG mbB
(86) Internationale Anmeldenummer: PCT/EP2020/064001
(87) Internationale Veröffentlichungsnummer: WO 2021/233531

(56) Entgegenhaltungen:
- DE-U1-202013 101 234
- US-A1- 2017 209 235
- US-A9- 2018 028 277

## Beschreibung

Die Erfindung betrifft ein Verfahren zum Herstellen eines Positionierungselements zum Fixieren einer Bohrschablone auf einer Gingiva eines Kiefers eines Patienten und eine Kombination aus einem entsprechenden Positionierungselement und einer Bohrschablone.

In der Implantologie werden anhand anatomischer Gegebenheiten, insbesondere des Kiefers, im Vorfeld die genauen Positionen geplant, an denen Implantate gesetzt werden sollen. Um Implantatbohrungen exakt an den für die Implantate geplanten Positionen einbringen zu können, kommen Bohrschablone zum Einsatz. Diese Bohrschablonen legen die Positionen der einzubringenden Implantatbohrungen gemäß der vorangehenden Planung fest.

Die von der Bohrschablone für Implantatbohrungen festgelegten Positionen sind jedoch abhängig von der Position der Bohrschablone auf dem Kiefer des Patienten. Sitzt eine Bohrschablone auf der weichen, nachgiebigen Gingiva, d.h. dem Zahnfleisch, des Kiefers auf, besteht die Gefahr, dass es der Bohrschablone zumindest teilweise an einem ausreichenden Halt fehlt. So kann der bereitgestellte Halt unzureichend für eine präzise Positionierung der einzubringenden Implantatbohrungen, beispielsweise im Submillimeterbereich. Verschiebt sich die Bohrschablone, d.h. stimmt ihre tatsächliche Position auf dem Kiefer des Patienten nicht mit der Position überein, für welche die Bohrschablone konfiguriert wurde, stimmen auch die von der Bohrschablone festgelegten Positionen für die Implantatbohrungen nicht mehr mit den geplanten Positionen überein.

Die US 2017/209235 A1 beschreibt eine Bohrerführungsanordnung, welche zum Führen eines Bohrers in den Kieferknochen eines Patienten während einer Zahnoperation verwendet wird. Die Bohrerführungsanordnung, umfasst mindestens eine durchgehende Führungsbohrung zum Führen des Bohrers und mindestens eine Befestigung zum Befestigen eines Teils der Bohrerführungsanordnung am Kiefer des Patienten während der Operation. Die Bohrerführungsanordnung umfasst einen ersten Teil, der die mindestens eine erste durchgehende Führungsbohrung, die mindestens eine erste Befestigung und mindestens einen ersten Referenzbereich zum Beziehen des ersten Teils auf ein erstes anatomisches Merkmal des Patienten, einen zweiten Teil, der einen zweiten Referenzbereich zum Beziehen des zweiten Teils auf ein zweites anatomisches Merkmal des Patienten enthält, und eine Führungsstruktur zum Führen des ersten Teils und des zweiten Teils in eine gewünschte Konfiguration.

Der Erfindung liegt die Aufgabe zugrunde, Verfahren und Mittel für ein verbessertes Fixieren einer Bohrschablone auf einer Gingiva eines Kiefers eines Patienten zu schaffen.

Die der Erfindung zugrunde liegende Aufgabe wird jeweils mit den Merkmalen der unabhängigen Patentansprüche gelöst. Ausführungsformen der Erfindung sind in den abhängigen Patentansprüchen angegeben.

Beispielsweise wird ein Verfahren zum Herstellen eines ersten Positionierungselements zum Fixieren einer ersten Bohrschablone auf einer Gingiva eines Kiefers eines Patienten in einer ersten Position relativ zu einem Kieferknochen des Kiefers bereitgestellt. Das erste Positionierungselement ist konfiguriert, die erste Bohrschablone mittels Drucks, welchen der Patient durch ein Schließen des Kiefers und eines Gegenkiefers auf das erste Positionierungselement aufbringt, in der ersten Position zu fixieren zum Einbringen von ersten Implantatbohrungen in den Kiefer für erste dentale Implantate. Die erste Bohrschablone umfasst eine erste Schablonenkontaktfläche zum Herstellen eines Kontakts mit einer Oberfläche der Gingiva des Kiefers und erste Durchgangsöffnungen. Die ersten Durchgangsöffnungen definieren jeweils eine erste Implantatposition zum Einbringen einer der ersten Implantatbohrungen.

Das Verfahren umfasst:
- Erfassen von ersten Formdaten eines ersten Abdrucks der Oberfläche der Gingiva des Kiefers unter Verwendung eines zwischen den beiden Kiefern des Patienten angeordneten Bissregistrats, wobei sich die beiden Kiefer in einer Erfassungsposition relativ zueinander befinden, wobei sich der erste Abdruck in der ersten Position relativ zu dem Kieferknochen befindet und wobei der erste Abdruck die erste Schablonenkontaktfläche definiert, · Erfassen eines auf das zwischen den beiden Kiefern des Patienten angeordnete Bissregistrat (130) aufgebrachten Erfassungsdrucks,
- Erfassen von ersten Strukturdaten des Kieferknochens, während das Bissregistrat zwischen den beiden Kiefern in der Erfassungsposition angeordnet ist,
- Erfassen von Positionsdaten des Bissregistrats relativ zu dem Kieferknochen, während das Bissregistrat zwischen den beiden Kiefern in der Erfassungsposition angeordnet ist,
- Erstellen eines ersten dreidimensionalen digitalen Modells unter Verwendung der erfassten ersten Formdaten, ersten Strukturdaten und Positionsdaten, wobei das erste Modell die Kieferknochenstruktur und die Oberfläche der Gingiva des Kiefers umfasst,
- Festlegen der ersten Implantatpositionen zum Einbringen der ersten Implantatbohrungen in den Kiefer unter Verwendung des ersten Modells,
- Erstellen eines zweiten dreidimensionalen digitalen Modells der ersten Bohrschablone und des ersten Positionierungselements unter Verwendung des ersten Modells und der festgelegten ersten Implantatpositionen, wobei Form und Abmessungen des ersten Positionierungselements so bestimmt werden, dass die beiden Kiefer beim Aufbringen des Erfassungsdrucks auf das erste Positionierungselement in der Erfassungsposition und die erste Bohrschablone in der ersten Position relativ zu dem Kieferknochen angeordnet werden,
- Herstellen des ersten Positionierungselements unter Verwendung des zweiten dreidimensionalen digitalen Modells, wobei das erste Positionierungselement ein Druckerfassungselement umfasst zum Prüfen, ob ein in der Erfassungsposition der beiden Kiefer auf das erste Positionierungselement aufgebrachter Druck mit dem Erfassungsdruck übereinstimmt.

Dies könnte den Vorteil haben, dass ein Positionierungselement für eine Bohrschablone bereitgestellt werden kann, welches dazu konfiguriert ist, eine Bohrschablone, insbesondere eine Schablonenkontaktfläche, in einer vorbestimmten Position relativ zu einem Kieferknochen eines Patienten anzuordnen und zu fixieren. Die dabei resultierende Position der Bohrschablone bzw. Schablonenkontaktfläche stellt beispielsweise eine Position eines Bissregistrat, insbesondere eines Abdrucks in dem Bissregistrat, relativ zu dem Kieferknochen nach. Bei der nachgestellten Position des Bissregistrats bzw. des Abdrucks handelt es sich beispielsweise um eine Position des Bissregistrats bzw. des Abdrucks während eines Erfassens von Strukturdaten des Kiefers bzw. Kieferknochens.

Die Schablonenkontaktfläche ist eine Fläche der Bohrschablone, welche mit der Oberfläche der Gingiva des Kiefers des Patienten in Kontakt tritt. Die Form der Schablonenkontaktfläche ist beispielsweisen identisch mit einem Abdruck der Gingivaoberfläche des Kiefers, welcher mit dem Bissregistrat erfasst wird. Die Formdaten des entsprechenden Abdrucks werden beispielsweise als Vorlage für die Form der Schablonenkontaktfläche verwendet. Sinn und Zweck des Erfassens des Abdrucks kann es beispielsweise sein, eine Formvorlage für die Schablonenkontaktfläche bereitzustellen. Somit kann beispielsweise sichergestellt werden, dass die Schablonenkontaktfläche einem Negativ der Gingivaoberfläche entspricht und beispielweise passgenau an der Gingivaoberfläche ansitzt.

Aufgrund der Nachgiebigkeit der Gingiva besteht die Gefahr, dass es zu ungewollten Abweichungen zwischen der tatsächlichen Position der Schablonenkontaktfläche relativ zu dem Kieferknochen und der Referenzposition des Abdrucks des Bissregistrats während des Erfassens der Kieferknochenstrukturdaten kommen kann. Das Positionierungselement kann den Vorteil haben, dass dieses ein Positionieren des Bohrschablone bzw. ein Ausrichten der Bohrschablone in dem Mundraum des Patienten ermöglicht, womit ungewollte Abweichungen verhindert werden können.

Neben dem Positionieren des Bohrschablone kann das Positionierungselement ferner den Vorteil haben, dass die beiden Kiefer beim Aufbringen von Druck auf das Positionierungselement in einer Position relativ zueinander angeordnet werden, welche einer Erfassungsposition entspricht. Bei dieser Erfassungsposition handeltes sich um Position, welche die beiden Kiefer relativ zueinander während des Erfassens der Kieferknochenstrukturdaten eingenommen haben. Diese Erfassungsposition der Kiefer resultiert beispielsweise aus dem Anordnen des Bissregistrats zwischen den Kiefern während des Erfassens der Kieferknochenstrukturdaten. Beispielsweise kann die Erfassungsposition von der Form und den Abmessungen des Bissregistrats abhängen. Ferner kann die Erfassungsposition beispielsweise von dem Druck abhängen, welchen die beiden Kiefer während des Erfassens der Kieferknochenstrukturdaten auf das Bissregistrat aufbringen.

Beispielsweise können die Form und Abmessungen des Positionierungselements so gewählt sein, dass die Position der Kieferknochen relativ zueinander der Erfassungsposition entspricht. Insbesondere können die Form und Abmessungen des Positionierungselements so gewählt sein, dass ein Abstand zwischen den Kieferknochen einem Erfassungsabstand zwischen den Kieferknochen während des Erfassens der Kieferknochenstrukturdaten entspricht. Sollen zwischen den Kiefern des Patienten genau eine Bohrschablone und genau ein Positionierungselement angeordnet werden, können Form und Abmessungen des Positionierungselements beispielsweise so gewählt sein, dass Form und Abmessungen des Positionierungselements in Kombination mit der Bohrschablone der Form und den Abmessungen des Bissregistrats entsprechen.

Sollen zwischen den Kiefern des Patienten zwei Bohrschablonen und genau ein Positionierungselement angeordnet werden, können Form und Abmessungen des Positionierungselements beispielsweise so gewählt sein, dass Form und Abmessungen des Positionierungselements in Kombination mit den beiden Bohrschablonen der Form und den Abmessungen des Bissregistrats entsprechen.

Sollen zwischen den Kiefern des Patienten zwei Bohrschablonen und zwei Positionierungselemente angeordnet werden, können Form und Abmessungen der beiden Positionierungselemente beispielsweise jeweils so gewählt sein, dass Form und Abmessungen der beiden Positionierungselemente in Kombination mit beiden Bohrschablonen der Form und den Abmessungen des Bissregistrats entsprechen.

Ferner kann beispielsweise der Druck erfasst werden, welchen die beiden Kiefer während des Erfassens der Kieferknochenstrukturdaten auf das Bissregistrat aufbringen. Dies kann beispielsweise unter Verwendung eines Druckerfassungselements des Bissregistrats erfolgen, welches den auf das Bissregistrat aufgebrachten Erfassungsdruck erfasst. Das Positionierungselement kann beispielsweise ebenfalls ein Druckerfassungselement umfassen, mit welchem der mit den Kiefern auf das Positionierungselement aufgebrachte Druck erfasst und geprüft werden kann, ob dieser dem auf das Bissregistrat aufgebrachten Erfassungsdruck entspricht.

Beispielsweise kann es unter Verwendung des Positionierungselements ermöglicht werden, eine Bohrschablone für einen Kiefer mittels Drucks, welchen der Patient durch ein Schlie-βen des Kiefers und eines Gegenkiefers auf das erste Positionierungselement aufbringt, in einer Position relativ zu dem Knochen des Kiefers zu positionieren und zu fixieren, welche der Position des Bissregistrat relativ zu dem Knochen des Kiefers während des Erfassens der Kieferknochenstrukturdaten entspricht. Ferner kann sichergestellt werden, dass zugleich die beiden Kieferknochen relativ zueinander in einer Position angeordnet sind, welche der Erfassungsposition der Kiefer relativ zueinander während des Erfassens der Kieferknochenstrukturdaten entspricht.

Beispielsweise werden die Positionen von Implantatbohrungen in den Kiefer bzw. Kieferknochen unter Verwendung der Kieferknochenstrukturdaten geplant bzw. festgelegt. Das Positionierungselement kann den Vorteil haben, dass mit diesem die Erfassungssituation nachgestellt werden kann, d.h. die relative Kieferknochenposition und die Position der weichen Gingiva relative zu dem zugehörigen Kieferknochen während des Erfassens der Kieferknochenstrukturdaten. Indem unter Verwendung des Positionierungselements die Erfassungssituation nachgestellt bzw. simuliert wird, kann beispielsweise sichergestellt werden, dass die Situation, in welcher die Implantatbohrungen unter Verwendung der Bohrschablone in den Kieferknochen eingebracht werden, der Situation entspricht, für welche die Positionen der Implantatbohrungen geplant bzw. festgelegt wurden. Somit können beispielsweise Abweichungen zwischen den geplanten Positionen und den tatsächlichen Positionen der Implantatbohrungen minimiert und/oder ausgeschlossen werden.

Die Positionen der Implantatbohrungen werden beispielsweise durch die Bohrschablone festgelegt. Indem die Bohrschablone in derselben Position relativ zu dem Kiefer bzw. Kieferknochen angeordnet und fixiert werden kann, kann beispielsweise sichergestellt werden, dass die durch die Bohrschablone vorgegebenen Positionen der Implantatbohrungen genau den geplanten Positionen der Implantatbohrungen entsprechen.

Ist die Bohrschablone unter Verwendung des Positionierungselements in der vorgesehenen Position relativ zu einem Kieferknochen des Kiefers positioniert und kieferdruckbasiert fixiert, können beispielsweise die Implantatbohrungen an den Positionen, welche die entsprechenden Durchgangsöffnungen des Bohrschablone definieren, in den Kieferknochen eingebracht werden. Beispielsweise können in dafür vorgesehenen Positionen zunächst Fixierungsbohrungen in den Kieferknochen eingebracht werden, in welchen Fixierungselemente eingebracht werden. Diese Fixierungselemente sind beispielsweise dazu konfiguriert, die Bohrschablone in der vorgehenden Position zum Einbringen der Implantatbohrungen kieferdruckunabhängig zu fixieren. Beispielsweise ermöglicht dies ein Einbringen der ersten Implantatbohrungen in den Kiefer, ohne dass der Patient Druck auf das Positionierungselement oder die Bohrschablone aufbringt.

Formdaten einer Oberfläche der Gingiva des Kiefers können beispielsweise unter Verwendung eines mittels eines Bissregistrats erfassten Abdrucks der entsprechenden Oberfläche erfasst werden. Hierzu wird das Bissregistrat beispielsweise zwischen den beiden Kiefern des Patienten angeordnet, welcher mit den Kiefern Druck auf das Bissregistrat ausübt zum Erzeugen eines oder zweier Abdrücke in dem Bissregistrat. Dabei befinden sich die Kiefer beispielsweise in der Erfassungsposition relativ zueinander, d.h. in einer Position in welcher auch die Kieferknochenstrukturdaten erfasst werden. Der Abdruck der Kieferoberfläche, z.B. Gingivaoberfläche, definiert beispielsweise die Schablonenkontaktfläche der Bohrschablone bzw. dient als deren geometrische Vorlage. Ferner befindet sich der Abdruck bei dessen Erzeugung in einer Position relativ zu dem Kieferknochen, welche mit der Schablonenkontaktfläche unter Verwendung des Positionierungselements nachgestellt bzw. simuliert wird.

Die Formdaten können beispielsweise durch einen separaten Scan des Bissregistrats erfasst werden. Dieser Scan kann beispielsweise unter Verwendung von elektromagnetischer Strahlung, etwa im sichtbaren Wellenlängenbereich erfolgen. Beispielsweise kann dieser Scan beispielsweise unter Verwendung eines Computertomographie-Verfahrens erfolgen. Alternativ kann dieser Scan unter Verwendung eines haptischen bzw. taktilen Scan-Verfahrens erfolgen, bei welchem die Oberflächen des Bissregistrats mit einem Taster abgetastet werden.

Die Formdaten können beispielsweise zusammen mit den Kieferknochenstrukturdaten erfasst werden. Beispielsweise ist das für das Erzeugen des Abdrucks verwendete Material des Bissregistrats opak, d.h. sichtbar, für das zum Erfassen der Kieferknochenstrukturdaten verwendete Scan-Verfahren, sodass der Abdruck auf dem Scan der Kieferknochenstruktur sichtbar ist. Ist der Abdruck nicht sichtbar, so kann beispielsweise das oben erwähnte separate Scan-Verfahren zum Erfassen der Formdaten verwendet werden.

Beispielsweise können Strukturdaten des Kieferknochens erfasst werden, während das Bissregistrat zwischen den beiden Kiefern in der Erfassungsposition angeordnet ist. Diese Kieferknochenstrukturdaten können beispielsweise verwendet werden zum Erstellen eines digitalen Modells des Kiefers, welches zum Festlegen der Implantatbohrungen in dem Kiefer verwendet wird. Beispielsweise ist die Gingiva transparent, d.h. unsichtbar, für das zum Erfassen der Kieferknochenstrukturdaten verwendeten Scan-Verfahren. Da sich die Implantatbohrungen beispielsweise durch die Gingiva in den Kieferknochen hineinerstrecken, kann für das Festlegen der Positionen der Implantatbohrungen neben den Kieferknochenstrukturdaten Oberflächenstrukturdaten der Gingiva notwendig sein. Diese Oberflächenstrukturdaten der Gingiva können beispielsweise unter Verwendung der mittels des Bissregistrats erfassten Formdaten erfasst werden. Die Oberflächenstrukturdaten der Gingiva können beispielsweise verwendet werden zum Erstellen eines digitalen Modells der Gingiva. Insbesondere können unter Verwendung der Oberflächenstrukturdaten der Gingiva bzw. des Modells der Gingiva die Positionen der Mündungen der geplanten Implantatbohrungen in der Gingivaoberfläche festgelegt werden. Diese Mündungen definieren beispielsweise die Positionen der Durchgangsöffnungen der auf der Gingivaoberfläche aufliegenden Bohrschablone. Durch diese Durchgangsöffnungen werden die Implantatbohrungen durch die Gingiva hindurch in den Kieferknochen eingebracht.

Beispielsweise werden zum Bestimmen der relativen Position des von dem Bissregistrat umfassten Abdrucks und damit der Gingivaoberfläche zu dem Kieferknochen, welche als Referenzposition zum Positionieren der Bohrschablone dient, Positionsdaten des Bissregistrats relativ zu dem Kieferknochen erfasst, während das Bissregistrat zwischen den beiden Kiefern in der Erfassungsposition angeordnet ist.

Diese Positionsdaten können beispielsweise zusammen mit den Kieferknochenstrukturdaten erfasst werden. Beispielsweise ist das für das Erzeugen des Abdrucks verwendete Material des Bissregistrats opak, d.h. sichtbar, für das zum Erfassen der Kieferknochenstrukturdaten verwendete Scan-Verfahren. Beispielsweise wird zusammen mit den Kieferknochenstrukturdaten das Bissregistrat mit dem Abdruck erfasst. Aus diesen Scan-Daten können beispielsweise die Positionsdaten des Bissregistrats relativ zu dem Kieferknochen bestimmt werden. Beispielsweise kann das für das Erzeugen des Abdrucks verwendete Material des Bissregistrats transparent, d.h. unsichtbar, für das zum Erfassen der Kieferknochenstrukturdaten verwendete Scan-Verfahren sein. Damit die Position des Bissregistrats relativ zu dem Kieferknochen dennoch zusammen mit den Kieferknochenstrukturdaten erfasst werden kann, umfasst das Bissregistrat beispielsweise ein oder mehrere Marker, welche opak für das zum Erfassen der Kieferknochenstrukturdaten verwendete Scan-Verfahren sind.

Beispielsweise werden die erfassten Positionsdaten des Bissregistrats relativ zu dem Kieferknochen verwendet, um das digitale Modelle der Gingiva und/oder der Gingivaoberfläche relativ zu dem digitalen Modell der Kieferknochenstruktur zu positionieren. Das Modell der Gingiva bzw. der Gingivaoberfläche basiert beispielsweise auf den Formdaten des Abdrucks der Gingivaoberfläche in dem Bissregistrat. Die so relativ zueinander angeordneten und ausgerichteten Modelle werden beispielsweise zum Festgelegen der Positionen der Implantatbohrungen durch die Gingiva in den Kieferknochen hinein verwendet.

Beispielsweise werden zum Bestimmen der relativen Position des Bissregistrats zum Kieferknochen, Strukturdaten eines dentalen Objekts des Kiefers verwendet, welches opak, d.h. sichtbar, für das zum Erfassen der Kieferknochenstrukturdaten verwendete Scan-Verfahren ist und zudem einen Abdruck in dem Bissregistrat hinterlässt. Das dentale Objekt kann als Bezugsobjekt zum Bestimmen der relativen Position verwendet werden, da seine relative Position zu dem Kieferknochen aus den Kieferknochenstrukturdaten und seine relative Position zu dem Bissregistrat aus den Formdaten des Abdrucks bekannt sind.

Unter Verwendung der erfassten Formdaten des Abdrucks in dem Bissregistrat, der Kieferknochenstrukturdaten und der Positionsdaten des Bissregistrats relativ zu dem Kieferknochen kann ein gemeinsames dreidimensionales digitales Modell der Kieferknochenstruktur und der Gingivaoberfläche erstellt werden. Dieses gemeinsame Modell umfasst beispielsweise ein unter Verwendung der Kieferknochenstrukturdaten erstelltes dreidimensionales digitales Modell der Kieferknochenstruktur sowie ein unter Verwendung der Formdaten erstelltes dreidimensionales digitales Modell der Gingivaoberfläche. Diese beiden Modelle werden in dem gemeinsamen Modell beispielsweise in einer relativen Position zueinander angeordnet, welche unter Verwendung der Positionsdaten des Bissregistrats relativ zu dem Kieferknochen bestimmt wird.

Das gemeinsame Modell wird beispielsweise zum Festlegen von Implantatpositionen zum Einbringen von Implantatbohrungen in den Kiefer, d.h. durch die Gingiva hindurch in den Kieferknochen hinein, verwendet. Im Zuge des Festlegens von Implantatpositionen werden beispielsweise dreidimensionale digitale Modelle der Implantatbohrungen in dem gemeinsamen Modell ergänzt und positioniert. Beispielsweise wird das gemeinsame Modell um Modelle der einzubringenden Implantate in den Implantatbohrungen ergänzt.

Beispielsweise wird ein dreidimensionales digitales Modell der Bohrschablone und des Positionierungselements unter Verwendung des gemeinsamen Modells von Kieferknochenstruktur und Gingivaoberfläche erstellt. Die Modelle der Implantatbohrungen, welche die Implantatpositionen definieren, können beispielsweise dazu verwendet werden, Durchgangsbohrungen in dem Modell der Bohrschablone zu erstellen. Diese Durchgangsbohrungen können zum Einbringen der Implantatbohrungen verwendet werden. Beispielsweise definieren die Mündungen der Implantatbohrungen in der Gingivaoberfläche die Positionen der Durchgangsöffnungen in der Bohrschablone. Die Positionen der Durchgangsöffnungen der Bohrschablone werden beispielsweise so gewählt, dass die Mündungen der Durchgangsöffnungen in der Schablonenkontaktfläche mit den Mündungen der geplanten Implantatbohrungen in der Gingivaoberfläche zusammenfallen. Beispielsweise umfasst das Modell der Bohrschablone und des Positionierungselements Modelle der einzubringenden Implantate.

Form und Abmessungen des Modells des Positionierungselements können so bestimmt werden, dass die beiden Kiefer beim Aufbringen von Druck auf das erste Positionierungselement in der Erfassungsposition und die Bohrschablone in einer Position relativ zu dem Kieferknochen angeordnet werden, welche der Position des Bissregistrats relativ zu dem Kieferknochen während des Erfassens der Kieferknochenstrukturdaten entspricht. Beispielsweise können Form und Abmessungen des Positionierungselements in Abhängigkeit von ein oder zwei Bohrschablonen und/oder einem weiter Positionierungselement so bestimmt werden, dass die beiden Kiefer in der Erfassungsposition und die Bohrschablone in der vorgesehenen Position angeordnet werden, wenn das Positionierungselement zusammen mit den ein oder zwei Bohrschablonen und/oder dem weiteren Positionierungselement zwischen den beiden Kiefern angeordnet wird und die beiden Kiefer Druck auf die entsprechenden zwischen ihnen angeordneten Schablonen und Elemente aufbringen.

Das Modell des Positionierungselements kann, etwa als Vorlage, dazu verwendet werden, das entsprechende Positionierungselement herzustellen. Dies kann beispielsweise den Vorteil haben, dass zusätzlich zu einem CAD-Verfahren zum Erstellen des Modells des Positionierungselements ein CAM-Verfahren zum Herstellen des entsprechenden Positionierungselements bereitgestellt werden kann. Das Positionierungselement kann beispielsweise unter Verwendung eines 3D-Druckers gedruckt werden oder mit einer Bearbeitungsvorrichtung, etwa einer spanabhebenden Bearbeitungsvorrichtung, wie beispielsweise einem Fräser, hergestellt werden.

Das Positionierungselement könnte den Vorteil haben, dass es ein präzises Positionieren und/oder Fixieren der Bohrschablone auf bzw. an dem Kiefer ermöglicht. Eine präzise Positionierung der Bohrschablone ermöglicht ein präzises Festlegen von Positionen, an welchen geplante Bohrungen, wie etwa Implantatbohrungen, vorzunehmen sind. Somit kann sichergestellt werden, dass Bohrungen, welche unter Verwendung der Bohrschablone ausgeführt jeweils präzise an der dafür vorgesehenen Stelle erfolgen können.

Das Positionierungselement kann eine erfassungsgetreue Fixierung der Bohrschablone ermöglichen, d.h. eine Fixierung, bei welcher sich die beiden Kiefer in der Erfassungsposition relativ zueinander befinden, in welcher die Formdaten und Strukturdaten erfasst wurden. Dabei kann sichergestellt werden, dass die Position und Ausrichtung der Schablonenkontaktfläche mit der Position und Ausrichtung des Abdrucks in der ersten Position relativ zu dem Kieferknochen, während des Erfassens der Form- und Strukturdaten, übereinstimmt.

Dies könnte den Vorteil haben, dass die Konfiguration des Positionierungselements darauf ausgerichtet werden kann, die relative Kieferposition und/oder -ausrichtung nachzustellen, in der die Strukturdaten erfasst wurden, die als Grundlage für die Planung der Bohrungen dienen. Somit kann sichergestellt werden, dass die Ausführungssituation, in welcher die geplanten Bohrungen ausgeführt werden, exakt der Erfassungssituation bzw. der Planungssituation entspricht und Abweichungen zwischen Planung und Ausführung vermieden werden können. Es kann also vorrangig Rücksicht genommen auf die Bedingungen, unter welchen Daten erfasst wurden, auf denen die Planung der Bohrschablone und des Positionierungselements beruhen. Beispielsweise können Fragen bezüglich der zukünftigen Position des Zahnersatzes und/oder einer daraus resultierenden Okklusion, im Zuge der Planung der Implantatbohrungen berücksichtigt werden, fließen aber nicht notwendiger Weise in die Planung des Positionierungselements ein. Mit anderen Worten kann das Positionierungselement zum Nachstellen der Erfassungssituation, nicht zum Nachstellen einer Verwendungssituation mit einem durch die Implantate verankerten Zahnersatz konfiguriert sein. Dies könnte den Vorteil haben, dass das Positionierungselement weder ein Modell des Zahnersatzes umfasst noch ein solches berücksichtigen muss. Somit könnte die Planung, Herstellung und/oder Verwendung des Positionierungselements vereinfacht werden.

Ferner könnte der Vorteil bestehen, dass die Erfassungsposition der Kiefer relativ zueinander direkt für eine Planung der Bohrschablone verwendet werden kann, wobei das Positionierungselement sicherstellt, dass die relative Kieferposition bei der Verwendung der Bohrschablone mit der Erfassungsposition übereinstimmt. Beispielsweise kann ein einziges Positionierungselement, beispielsweise ein einstückig ausgebildetes Positionierungselement, dazu verwendet werden, die Bohrschablone genau in der Position relative zu dem Kieferknochen anzuordnen und/oder zu fixieren, welche der Position beim Erfassen der Formdaten und der Strukturdaten entspricht.

Beispielsweise wird eine Simulation der Erfassungsposition herangezogen, etwa in Form des ersten digitalen Modells, unter dessen Verwendung eine Relation von Bohrschablone und Positionierungselement bestimmt wird, welche genau dem Bissregistrat und seiner Positionierung während des Erfassens der Form- und Strukturdaten entspricht. Damit kann sichergestellt werden, dass Form und Abmessungen des Positionierungselements so konfiguriert werden, dass die beiden Kiefer beim Aufbringen von Druck auf das Positionierungselement in der Erfassungsposition und die erste Bohrschablone in der ersten Position relativ zu dem Kieferknochen angeordnet werden.

Dies könnte den Vorteil haben, dass es nicht notwendig ist, bei der Digitalisierung der Knochenstruktur des Kiefers und/oder der Digitalisierung der Auflageflächen der Gingiva, d.h. dem Erstellen des ersten digitalen Modells, Rücksicht auf die Position des zukünftigen Zahnersatzes zu nehmen.

Beim Festlegen der Implantatpositionen können ferner die strukturellen Verhältnisse im Kieferknochen und um diesen herum berücksichtigt werden. Somit kann sichergestellt werden, dass diese Verhältnisse auch beim Ausführen der Implantatbohrungen unter Verwendung der festlegten Implantatpositionen berücksichtigt werden. Es kann beim Festlegen der Implantatpositionen beispielsweise darauf geachtet werden, dass bei Implantatbohrungen an der entsprechenden Implantatpositionen der Kiefernerv, d.h. der Nervus mandibularis, nicht beschädigt wird, die Knochenwand bzw. Knochenwände um die Implantatbohrungen herum auf allen Seiten eine ausreichende Mindestdicke aufweisen, um für die Implantate einen ausreichenden Halt bereitstellen zu können, von den Implantatbohrungen keine Hauptadern getroffen werden, und/oder dass die Implantatbohrungen jeweils einen die einsetzenden Implantate korrekten Bohrwinkel aufweisen. Dabei können die Implantatpositionen für die geplanten Implantate präzise festgelegt werden. Beispielsweise ist eine Implantatpositionen im Submillimeterbereich genau festzulegen, beispielsweise auf 0,1 mm genau.

Für eine Planung von Implantatbohrungen und ein Festlegen von Implantatpositionen können beispielsweise 3D-Scan- und Visualisierungstechnologien verwendet werden. Für die geplanten Implantatbohrungen kann eine Bohrschablone hergestellt werden, welche es ermöglicht bzw. sicherstellt, dass die Implantatbohrungen exakt an den für die einzubringenden Implantate geplanten Implantatpositionen und/oder mit den exakten für diese geplanten Tiefen in den Kiefer bzw. den Kieferknochen des Patienten eingebracht werden.

Ein Bissregistrat dient beispielsweise einer Aufzeichnung und Bestimmung einer Kieferrelation. Ein solches Registrat umfasst beispielsweise eine Platte, mit einem Wachs, Kunststoff und/oder einem weichen Metall, welche zwischen die vollbezahnten, teilbezahnten oder zahnlosen Kiefer eines Patienten eingebracht wird, um die Lage des Unterkiefers relative zum Oberkiefer aufzuzeichnen bzw. zu bestimmen. Ein Registrat dient, beispielsweise als intraorales Hilfsmittel, zur Erkennung, Vermessung und Fixierung von Kieferstellungen und Kieferkontakten aller Art. Ein Bissregistrat dient dabei beispielsweise der Bestimmung relativer Positionen von Antagonisten im Gebiss. Unter Verwendung eines Bissregistrats kann beispielsweise bei der Herstellung einer Prothese geprüft werden, ob ein anatomisch optimaler Biss gegeben ist. Beispielsweise kann ein Bissregistrat zum Erfassen einer Oberfläche, beispielsweise einer Gingivaoberfläche, und/oder von dentalen Objekten eines oder beider Kiefer verwendet werden.

Eine zahnmedizinische Versorgung eines teilweise oder vollständig zahnlosen Kiefers umfasst beispielsweise ein Einbringen einer Verankerung für einen Zahnersatz. Ein Zahnersatz wird beispielsweise unter Verwendung von ein oder mehreren Implantaten im Kiefer des Patienten verankert. Zum Einsetzten der Implantate wird beispielsweise für jedes der einzusetzenden Implantate jeweils ein Bohrloch in den Kiefer gebohrt, d.h. eine Implantatbohrung eingebracht, welches zur Aufnahme des entsprechenden Implantats konfiguriert ist. Zum Bohren solcher Bohrlöcher können Bohrschablone zum Einsatz kommen, welche die Positionen der zu bohrenden Bohrlöcher vorgeben. Die Bohrschablone wird zum Einbringen der Implantatbohrungen auf der Gingiva des Kiefers angeordnet.

Beispielsweise handelt es sich bei dem Kiefer des Patienten um einen zahnlosen Kiefer. Beispielsweise kann das gemeinsame Modell von Kieferknochenstruktur und Gingivaoberfläche erstellt werden, ohne dass Zähne oder andere dentale Objekte als Bezugsobjekte notwendig sind. Beispielsweise kann das Positionierungselement eine zahnmedizinische Versorgung eines oder zweier zahnloser Kiefer eines Patienten ermöglichen. Beispielsweise kann eine Bohrschablonen beim Aufbringen von Druck auf das Positionierungselement mit den beiden Kiefern in einer vorgesehenen Position relativ zu dem Kieferknochen angeordnet werden. Dieses Positionieren kann beispielsweise unabhängig von einem Vorhandensein von Zähnen oder anderen dentalen Objekten in den Kiefern erfolgen, d.h. ein oder beide Kiefer können zahnlos sein. Ein Fixieren und Abstützen der Bohrschablone an dem zahnlosen Kiefer kann beispielsweise unter Verwendung des Positionierungselements erfolgen, über welches ein kieferdruckbasiertes Fixieren und Abstützen der Bohrschablone implementiert werden kann.

Beispielsweise kann das Positionierungselement ferner dazu verwendet werden, wenn eine korrekte Position der Bohrschablone erreicht ist, die Bohrschablone während des Einbringens der Implantatbohrungen kieferdruckbasiert konstant in der korrekten Position zu halten, ohne dass es zu einem Verrutschen kommt. Beispielsweise kann das Positionierungselement dazu verwendet werden, wenn eine korrekte Position der Bohrschablone erreicht ist, die Bohrschablone während eines Einbringens von Fixierungsbohrungen sowie einem Einbringen von Fixierungselementen in die Fixierungsbohrungen kieferdruckbasiert konstant in der korrekten Position zu halten, ohne dass es zu einem Verrutschen kommt. Die Fixierungselementen können dann beispielsweise dazu verwendet werden, die Bohrschablone während des Einbringens der Implantatbohrungen kieferdruckunabhängig konstant in der korrekten Position zu halten, ohne dass es zu einem Verrutschen kommt. Nach dem Einbringen der Fixierungselemente, welche die Bohrschablone an dem Kiefer bzw. dem Kieferknochen fixieren, kann der Patient beispielsweise aufhören mit seinen Kiefern Druck auf das Positionierungselement aufzubringen. Beispielsweise kann der Patient die Kiefer öffnen, um das Einbringen der Implantatbohrungen zu erleichterten. Durch ein Öffnen der Kiefer kann beispielsweise ein Zugang zu den Durchgangsöffnungen der Bohrschablone erleichtert werden. Ferner kann beispielsweise das Positionierungselement aus dem Mundraum des Pateienten entfernt werden, um das Einbringen der Implantatbohrungen weiter zu erleichterten. Durch das Entfernen des Positionierungselements kann der Zugang zu den Durchgangsöffnungen der Bohrschablone beispielsweise weiter erleichtert werden.

Bei einem zahnlosen, d.h. adentalen, Kiefer ergeben sich mehrere Herausforderungen für eine Verwendung einer Bohrschablone. Im Falle eines zahnlosen Kiefers existieren neben dem Kieferknochen, welcher als interne Referenzstruktur dient, keine externen Referenzstrukturen bzw. Bezugsstrukturen auf dem Kiefer, welche dazu verwendet werden könnten, die Bohrschablone relativ zum Kieferknochen, exakt auf dem Kiefer zu positionieren. Somit kann sich das Positionieren einer Bohrschablone als Herausforderung erweisen. Neben dem Positionieren kann sich bei einem zahnlosen Kiefer zudem als schwierig erweisen einen ausreichenden Halt für die Bohrschablone bereitzustellen. Bei einem zahnlosen Kiefer fehlt es an Strukturen, wie etwa Zähnen, an denen eine solche Bohrschablone fixiert und/oder abgestützt werden kann. Vielmehr sitzt eine Bohrschablone in diesem Fall auf der weichen, nachgiebigen Gingiva, d.h. dem Zahnfleisch, des Kiefers auf. Die Bohrschablone fehlte es mithin an Halt, da sie sich auf und/oder mit der Gingiva bewegen kann. Unter Verwendung des hier beschriebenen Verfahrens kann beispielsweise ein Positionierungselement bereitgestellt werden, welches sowohl ein Positionieren als auch ein Fixieren einer Bohrschablone an einem zahnlosen Kiefer ermöglicht. Das Positionieren und Fixieren unter Verwendung des Positionierungselements erfolgt beispielsweise kieferdruckbasiert bzw. kieferdruckabhängig. Es kann eine Positionierung zwischen den Kiefern erfolgen, welche durch den Druck fixiert wird. Die einander gegenüberliegenden Kiefer mit ihren komplexen Oberflächenstrukturen, können dabei die Position der zwischen ihnen angeordneten Kombination aus Bohrschablone(n) und Positionierungselement(en) eindeutig festlegen. Dabei kann beispielsweise für einen gegebenen Kiefer jeweils der gegenüberliegende Kiefer als Referenzstruktur dienen.

Selbst wenn eine korrekte Position einmal erreicht ist, kann es sich im Allgemeinen als schwierig gestalten die Bohrschablone während des Einbringens von Implantatbohrungen konstant in der korrekten Position zu halten, ohne dass es zu einem Verrutschen kommt. Das Positionierungselement kann es beispielsweise ermöglichen die Bohrschablone während des Einbringens der Implantatbohrungen kieferdruckbasiert konstant in der korrekten Position zu halten, ohne dass es zu einem Verrutschen kommt.

Beispielsweise ist das erste Positionierungselement konfiguriert, die erste Bohrschablone in der ersten Position zum Einbringen von ersten Fixierungsbohrungen in den Kiefer für erste Fixierungselemente zu fixieren. Die erste Bohrschablone umfasst beispielsweise zweite Durchgangsöffnungen. Die zweiten Durchgangsöffnungen definieren jeweils eine erste Fixierungsposition zum Einbringen einer der ersten Fixierungsbohrungen. Die ersten Fixierungselemente sind beispielsweise konfiguriert zum kieferdruckunabhängigen Fixieren der ersten Bohrschablone in der ersten Position zum Einbringen der ersten Implantatbohrungen in den Kiefer für die ersten dentalen Implantate. Das Verfahren umfasst beispielsweise ferner ein Festlegen der ersten Fixierungspositionen zum Einbringen der ersten Fixierungsbohrungen in den Kiefer unter Verwendung des ersten Modells. Das zweite dreidimensionale digitale Modell der ersten Bohrschablone und des ersten Positionierungselements wird beispielsweise ferner unter Verwendung festgelegten ersten Fixierungspositionen erstellt.

Dies könnte den Vorteil haben, dass ein kieferdruckunabhängiges Fixieren der Bohrschablone in der vorgesehenen Position zum Einbringen der Implantatbohrungen in den Kiefer ermöglicht werden kann. Das kieferdruckunabhängige Fixieren kann insbesondere ohne Verwendung des Positionierungselements erfolgen. Beispielsweise kann das Positionierungselement nach dem Einbringen der Fixierungselemente aus dem Mundraum des Patienten entfernt werden. Beispielsweise wird die Bohrschablone zum Einbringen der Fixierungsbohrungen in den Kiefer kieferdruckabhängig bzw. kieferdruckbasiert unter Verwendung des Positionierungselements in der vorgesehenen Position fixiert.

Die Planung der Fixierungsbohrungen kann beispielsweise analog zu der Planung der Implantatbohrungen erfolgen. Beispielsweise werden unter Verwendung des gemeinsamen Modells der Kieferknochen und des Gingivaoberfläche Positionen der Fixierungsbohrungen festgelegt. Beispielsweise wird das gemeinsame Modell mit dreidimensionalen digitalen Modellen der Fixierungsbohrungen ergänzt. Beispielsweise wird das gemeinsame Modell mit dreidimensionalen digitalen Modellen der in die Fixierungsbohrungen einzubringenden Fixierungselementen ergänzt.

Beispielsweise wird das Modell der Bohrschablone in dem Modell der Bohrschablone und des Positionierungselements so konfiguriert, dass sie zusätzliche Durchgangsöffnungen zum Einbringen der Fixierungsbohrungen an den in dem gemeinsamen Modell vorgesehenen Positionen durch die Gingiva hindurch in den Kieferknochen hinein umfasst. Die Positionen der zusätzlichen Durchgangsöffnungen der Bohrschablone werden beispielsweise so gewählt, dass die Mündungen der zusätzlichen Durchgangsöffnungen in der Schablonenkontaktfläche mit den Mündungen der geplanten Fixierungsbohrungen in der Gingivaoberfläche zusammenfallen. Beispielsweise wird das Modell der Bohrschablone und des Positionierungselements um dreidimensionale digitale Modelle der in die Fixierungsbohrungen einzubringenden Fixierungselemente ergänzt.

Um die Bohrschablone für die Implantatbohrungen konstant an einer festen Position relative zum Kiefer zu halten, könnten beispielsweise Fixierungselemente verwendet werden. Die Bohrschablone könnte neben Implantatbohrungen zusätzliche Fixierungsbohrungen zum Einbringen von Fixierungselementen in den Kiefer festlegen. Unter Verwendung der Fixierungselemente kann die Bohrschablone am Kiefer in einer stabilen Position fixiert werden, wobei die Fixierungselemente der Bohrschablone auf der weichen Gingiva halt geben. Im Falle eines zahnlosen Kiefers, können sich beim Einbringen der Fixierungsbohrungen im Allgemeinen dieselben Schwierigkeiten ergeben, welche sich auch beim Einbringen von Implantatbohrungen ergeben: Schwierigkeiten können sowohl beim Positionieren der Bohrschablone als auch beim konstanten Halten der Bohrschablone in einer entsprechenden Position für das Einbringen der Fixierungsbohrungen ergeben. Der Bohrschablone kann es auf der weichen Gingiva an Positionsreferenzen und Halt fehlen, weshalb die Gefahr eines Verrutschens der Bohrschablone beim Positionieren und/oder Einbringen der Fixierungsbohrungen besteht. Weichen jedoch die Fixierungsbohrungen von ihren geplanten Positionen im Kiefer ab, weichen im Ergebnis die Position der Bohrschablone und mithin auch die von der Bohrschablone vorgegebenen Positionen für die Implantatbohrungen von den geplanten Positionen ab. Unter Verwendung eines Positionierungselements für die Bohrschablone könnten diese Schwierigkeiten beispielsweise vermieden werden. Das Positionierungselement kann ein Positionieren und Fixieren der Bohrschablone an einer vorgesehenen Position relativ zu dem Kieferknochen ermöglichen. Somit können die Durchgangsöffnungen zum Einbringen der Fixierungsbohrungen in den Kiefer an den vorgesehenen Positionen relativ zu der Gingiva und dem Kieferknochen positioniert werden. Während des Einbringens der Fixierungsbohrungen in den Kiefer und der Fixierungselemente in die Fixierungsbohrungen kann die Bohrschablone kieferdruckbasiert unter Verwendung des Positionierungselements in der vorgesehenen Position relativ zum Kieferknochen gehalten werden. Mithin können auch die eingebrachten Fixierungselemente die Bohrschablone in der vorgesehenen Position fixieren. Diese Fixierung unter Verwendung der Fixierungselement ist kieferdruckunabhängig, d.h. erlaubt ein Öffnen der Kiefer und/oder ein Entfernen des Positionierungselements, ohne dass die Position der Bohrschablone relativ zu dem Kieferknochen verändert wird.

Dies könnte den Vorteil haben, dass das Positionierungselement ein präzises Positionieren und Fixieren der Bohrschablone auf bzw. an dem Kiefer ermöglicht, noch bevor die Fixierungsbohrungen ausgeführt werden. Somit kann sichergestellt werden, dass auch die Fixierungsbohrungen und damit die anschließend auszuführenden Implantatbohrungen jeweils präzise an den dafür vorgesehenen Positionen erfolgen.

Fehlt es einer Bohrschablone an Halt, beispielsweise im Falle eines zahnlosen Kiefers, kann sich die Bohrschablone auf der weichen Gingiva bewegen, womit die von der Bohrschablone bereitgestellten Positionsangabe ungenau und damit fehlerhaft werden. Um die Bohrschablone für die Implantatbohrungen fest in der ersten Position zu halten, werden beispielsweise Fixierungselemente verwendet, welche seitlich, etwa labial und/oder bukkal, in den Kiefer eingebracht werden. Hierfür legt die Bohrschablone beispielsweise Fixierungspositionen fest, welche Positionen sind, an denen Fixierungsbohrungen zur Aufnahme entsprechender Fixierungselemente eingebracht werden. Diese Fixierungsbohrungen werden beispielsweise zusätzlich zu den Implantatbohrungen in der Bohrschablone mit eingeplant, sodass die Bohrschablone diese mitumfasst.

Für Fixierungsbohrungen ergibt sich im Falle eines zahnlosen Kiefers dieselbe Problematik, wie bereits für die Implantatbohrungen. Da die Gingiva weich ist und es sowohl an Referenzstrukturen zum Positionieren als auch an Fixierungsstrukturen zur Fixierung der einmal positionierten Bohrschablone auf bzw. an dem Kiefer fehlt, besteht die Gefahr eines Verrutschens der Bohrschablone auch beim Einbringen der Fixierungsbohrungen. Diese Gefahr kann mit einem Positionierungselement vermieden werden.

Ferner könnte der Vorteil bestehen, dass es nicht notwendig ist beim Bohren und Einsetzten der Fixierungselemente Rücksicht auf die Position des zukünftigen Zahnersatzes zu nehmen. Dadurch, dass die präzisen Verhältnisse unter denen die Form- und Strukturdaten erfasst wurden, bei einem druckbasierten Fixieren der Bohrschablone unter Verwendung des Positionierungselements exakt nachgestellt werden, kann eine hochpräzise Positionierung der Fixierungsbohrungen und damit der Implantatbohrungen erreicht werden, welche exakt mit den Planungsvorgaben übereinstimmt. Das kieferdruckbasierte Fixieren des Bohrschablone bezieht sich auf ein Aufbringen von Druck auf das Positionierungselement durch die Kiefer, wobei die Kiefer in der Erfassungsposition und die Bohrschablone in der vorbestimmten Position relativ zu dem Kieferknochen angeordnet werden. Hierfür werden beispielsweise eine passende Form und passende Abmessungen des Positionierungselements unter Verwendung des zweiten digitalen Modells bestimmt.

Beispielsweise umfasst das Verfahren ferner ein Herstellen der ersten Bohrschablone unter Verwendung des zweiten dreidimensionalen digitalen Modells. Dies könnte den Vorteil haben, dass zusätzlich zu dem Positionierungselement die Bohrschablone bereitgestellt werden kann, für welche das Positionierungselement konfiguriert ist.

Das Modell der Bohrschablone kann, etwa als Vorlage, dazu verwendet werden, die entsprechende Bohrschablone herzustellen. Dies kann beispielsweise den Vorteil haben, dass zusätzlich zu einem CAD-Verfahren zum Erstellen des Modells der Bohrschablone ein CAM-Verfahren zum Herstellen der entsprechenden Bohrschablone bereitgestellt werden kann. Die Bohrschablone kann beispielsweise unter Verwendung eines 3D-Druckers gedruckt werden oder mit einer Bearbeitungsvorrichtung, etwa einer spanabhebenden Bearbeitungsvorrichtung, wie beispielsweise einem Fräser, hergestellt werden.

Beispielsweise umfasst das erste Positionierungselement ein oder mehrere erste Verbindungselemente, welche konfiguriert sind zum Herstellen und Lösen einer zerstörungsfrei lösbaren ersten Verbindung zwischen dem ersten Positionierungselement und der ersten Bohrschablone im Mundraum des Patienten.

Dies könnte den Vorteil haben, dass das Positionierungselement aus dem Mundraum des Patienten entfernt werden kann, während die Bohrschablone im Mundraum verbleibt. Beispielsweise kann die in dem Mundraum verbleibende Bohrschablone an dem Kiefer unter Verwendung von Fixierungselementen fixiert sein. Beim Einbringen der Bohrschablone und des Positionierungselements in den Mundraum des Patienten können die Bohrschablone und das Positionierungselement beispielsweise über die zerstörungsfrei lösbare Verbindung verbunden sein und gemeinsam eingebracht werden. Beispielsweise können die Bohrschablone und das Positionierungselement nacheinander in den Mundraum des Patienten eingebracht und dort unter Verwendung der zerstörungsfrei lösbaren Verbindung miteinander verbunden werden.

Beispielsweise umfasst die zerstörungsfrei lösbare erste Verbindung eine Steckverbindung. Dies könnte den Vorteil haben, dass eine effiziente zerstörungsfrei lösbare Verbindung bereitgestellt werden kann. Beispielsweise umfassen das Positionierungselement und/oder die Bohrschablone ein oder mehrere weibliche Verbindungselemente, welche zur Aufnahme von ein oder mehreren männlichen Verbindungselementen der Bohrschablone bzw. des Positionierungselements konfiguriert sind.

Beispielsweise umfasst die erste Bohrschablone das erste Positionierungselement. Beispielsweise umfasst das erste Positionierungselement erste Zugangsöffnungen zu den ersten Durchgangsöffnungen der ersten Bohrschablone. Dies könnte den Vorteil haben, dass das Positionierungselement zum Einbringen der Implantatbohrungen im Mundraum des Patienten verbleiben kann. Zugang zu den Durchgangsöffnungen der Bohrschablone zum Einbringen der Implantatbohrungen wird beispielsweise über die Zugangsöffnungen des Positionierungselements bereitgestellt.

Beispielsweise sind die ersten Zugangsöffnungen zugänglich zum Einbringen der Implantatbohrungen, während mit den Kiefern Druck auf das Positionierungselement ausgeübt wird. Beispielsweise sind die ersten Zugangsöffnungen zugänglich zum Einbringen der Implantatbohrungen, wenn mit den Kiefern kein Druck auf das Positionierungselement ausgeübt wird und die Kiefer weiter voneinander entfernt werden, als es in der Erfassungsposition der Fall ist. Beispielsweise sind die Kiefer dazu so weit voneinander zu entfernen, dass der Gegenkiefer oder eine zweite Bohrschablone den Kontakt mit dem Positionierungselement verliert. Beispielsweise sind die ersten Zugangsöffnungen als Kanäle ausgestaltet, welche jeweils zu einer der Durchgangsöffnungen führen. Beispielsweise sind die ersten Zugangsöffnungen als Ausnehmung aus dem Positionierungselement ausgestaltet. Beispielsweise weist das Positionierungselement zumindest abschnittsweise eine Gitterstruktur auf, deren Zwischenräume die Ausnehmungen bereitstellen. Beispielsweise ist das Positionierungselement in vestibularer Richtung, z.B. labial und/oder bukkal, offen. Beispielsweise ist das Positionierungselement palatinal bzw. lingual geschlossen. Beispielsweise ist das Positionierungselement ausgehöhlt. Beispielsweise umfasst das Positionierungselement zum Stützen des Hohlraums ein oder mehrere Stützstreben, welche sich beispielsweise senkrecht zwischen den beiden Kiefern erstecken.

Beispielsweise werden unter Verwendung des zwischen den beiden Kiefern in der Erfassungsposition angeordneten Bissregistrats zweite Formdaten eines zweiten Abdrucks einer Oberfläche des Gegenkiefers erfasst, welche zum Erstellen des ersten dreidimensionalen digitalen Modells verwendet werden, welches ferner die zweite Oberfläche des Gegenkiefers umfasst. Die zweite Oberfläche des Gegenkiefers kann beispielsweise als Vorlage für die geometrische Form einer Kieferkontaktfläche des Positionierungselements oder einer Schablonenkontaktfläche einer zweiten Bohrschablone verwendet werden.

Beispielsweise umfasst das erste Positionierungselement eine Kieferkontaktfläche zum Herstellen eines Kontakts mit der Oberfläche des Gegenkiefers. Der zweite Abdruck definiert die Kieferkontaktfläche. Über die Kieferkontaktfläche kann der Gegenkiefer beispielsweise direkt Druck auf das Positionierungselement ausüben.

Beispielsweise umfasst das Verfahren ferner ein Erfassen von zweiten Strukturdaten eines Gegenkieferknochens des Gegenkiefers, während das Bissregistrat zwischen den beiden Kiefern in der Erfassungsposition angeordnet ist. Beispielsweise werden die Gegenkieferknochenstrukturdaten zusammen mit den Kieferknochenstrukturdaten erfasst. Die zweiten Strukturdaten werden zum Erstellen des ersten dreidimensionalen digitalen Modells verwendet, welches ferner die Gegenkieferknochenstruktur umfasst. Das Verfahren umfasst ferner ein Herstellen einer zweiten Bohrschablone, welche eine zweite Schablonenkontaktfläche zum Herstellen eines Kontakts mit der Oberfläche des Gegenkiefers umfasst. Der zweite Abdruck definiert die zweite Schablonenkontaktfläche. Die Oberfläche des Gegenkiefers ist eine Oberfläche der Gingiva des Gegenkiefers. Die zweite Bohrschablone umfasst ferner dritte Durchgangsöffnungen. Die dritten Durchgangsöffnungen definieren jeweils eine zweite Implantatposition zum Einbringen einer zweiten Implantatbohrung in den Gegenkiefer für zweite dentale Implantate. Die erfassten Positionsdaten umfassen Positionsdaten des Bissregistrats relativ zu dem Gegenkieferknochen. Das Verfahren umfasst ferner ein Festlegen der zweiten Implantatpositionen zum Einbringen der zweiten Implantate in den Gegenkiefer unter Verwendung des ersten Modells. Das zweite Modell umfasst die zweite Bohrschablone. Zum Erstellen des zweiten Modells werden ferner die festgelegten zweiten Implantatpositionen verwendet.

Dies könnte den Vorteil haben, dass neben einer Bohrschablone zum Einbringen von Implantatbohrungen in den Kiefer zusätzlich eine Bohrschablone zum Einbringen von Implantatbohrungen in den Gegenkiefer erstellt werden kann. Beide Bohrschablonen können beispielsweise unter Verwendung des Positionierungselements jeweils in einer Position relativ zu dem Kieferknochen bzw. dem Gegenkieferknochen druckbasiert positioniert und fixiert werden, welche einer Position des Bissregisters relativ zu dem Kiefer bzw. dem Gegenkiefer während des Erfassens des Kieferknochenstrukturdaten bzw. der Gegenkieferknochenstrukturdaten entspricht.

Beispielsweise handelt es sich bei dem Gegenkiefer des Patienten um einen zahnlosen Gegenkiefer.

Beispielsweise ist das erste Positionierungselement ferner konfiguriert, die zweite Bohrschablone in der zweiten Position mittels Drucks, welchen der Patient durch ein Schließen der beiden Kiefer auf das erste Positionierungselement aufbringt, zu fixieren. Form und Abmessungen des ersten Positionierungselements sind ferner so bestimmt, dass die zweite Bohrschablone in der zweiten Position relativ zum Gegenkiefer angeordnet wird.

Dies könnte den Vorteil haben, dass die zweite Bohrschablone bzw. die zweite Schablonenkontaktfläche in einer zweiten Position relativ zum Gegenkiefer positioniert und fixiert werden kann, welche der Position des Bissregistrats bzw. des zweiten Abdrucks relativ zu dem Gegenkieferknochen während des Erfassens der Strukturdaten des Gegenkieferknochens entspricht bzw. die entsprechende Referenzposition simuliert.

Beispielsweise ist das erste Positionierungselement konfiguriert, die zweite Bohrschablone in der zweiten Position zum Einbringen von zweiten Fixierungsbohrungen in den Gegenkiefer für zweite Fixierungselemente zu fixieren. Die zweite Bohrschablone umfasst beispielsweise ferner vierte Durchgangsöffnungen, welche jeweils eine zweite Fixierungsposition zum Einbringen einer der zweiten Fixierungsbohrungen definieren. Die zweiten Fixierungselemente sind beispielsweise konfiguriert zum kieferdruckunabhängigen Fixieren der zweiten Bohrschablone in einer zweiten Position relativ zum Gegenkieferknochen zum Einbringen von zweiten Implantatbohrungen in den Gegenkiefer für zweite dentale Implantate. Das Verfahren umfasst beispielsweise ferner ein Festlegen der zweiten Fixierungspositionen zum Einbringen der zweiten Fixierungselemente in den Gegenkiefer unter Verwendung des ersten Modells. Zum Erstellen des zweiten Modells werden beispielsweise ferner die festgelegten zweiten Fixierungspositionen verwendet.

Dies könnte den Vorteil haben, dass die zweite Bohrschablone kieferdruckabhängig in der zweiten Position positioniert und konfiguriert werden kann. Dieses kieferdruckabhängige Positionieren und Fixieren kann einem Einbringen von Fixierungsbohrungen und Fixierungselementen in den Gegenkiefer dienen.

Dies könnte den Vorteil haben, dass mittels Fixierungselemente ein kieferdruckunabhängiges Fixieren der Bohrschablone in der zweiten Position zum Einbringen der Implantatbohrungen in den Gegenkiefer ermöglicht werden kann. Das kieferdruckunabhängige Fixieren kann insbesondere ohne Verwendung des Positionierungselements erfolgen. Beispielsweise kann das Positionierungselement nach dem Einbringen der Fixierungselemente aus dem Mundraum des Patienten entfernt werden.

Die Planung der zweiten Fixierungsbohrungen kann beispielsweise analog zu der Planung der ersten Fixierungsbohrungen erfolgen. Beispielsweise werden unter Verwendung des gemeinsamen Modells der Kieferknochen und der Gingivaoberflächen Positionen der Fixierungsbohrungen in dem Gegenkiefer festgelegt. Beispielsweise wird das gemeinsame Modell mit dreidimensionalen digitalen Modellen der Fixierungsbohrungen im Gegenkiefer ergänzt. Beispielsweise wird das gemeinsame Modell mit dreidimensionalen digitalen Modellen der in die Fixierungsbohrungen im Gegenkiefer einzubringenden Fixierungselementen ergänzt.

Beispielsweise wird das Modell der zweiten Bohrschablone in dem Modell der Bohrschablonen und des Positionierungselements so konfiguriert, dass die zweite Bohrschablone zusätzliche Durchgangsöffnungen zum Einbringen der Fixierungsbohrungen an den in dem gemeinsamen Modell vorgesehenen Positionen durch die Gingiva hindurch in den Gegenkieferknochen umfasst. Die Positionen der zusätzlichen Durchgangsöffnungen der zweiten Bohrschablone werden beispielsweise so gewählt, dass die Mündungen der zusätzlichen Durchgangsöffnungen in der Schablonenkontaktfläche mit den Mündungen der geplanten Fixierungsbohrungen in der Gingivaoberfläche des Gegenkiefers zusammenfallen. Beispielsweise wird das Modell der Bohrschablonen und des Positionierungselements um dreidimensionale digitale Modelle der in die Fixierungsbohrungen im Gegenkiefer einzubringenden Fixierungselemente ergänzt.

Beispielsweise ist das erste Positionierungselement ferner konfiguriert, die zweite Bohrschablone in der zweiten Position zum Einbringen der zweiten Fixierungsbohrungen in den Gegenkiefer für die zweiten Fixierungselemente zu fixieren.

Beispielsweise umfasst das erste Positionierungselement ferner ein oder mehrere zweite Verbindungselemente, welche konfiguriert sind zum Herstellen und Lösen einer zerstörungsfrei lösbaren zweiten Verbindung zwischen dem ersten Positionierungselement und der zweiten Bohrschablone im Mundraum des Patienten.

Dies könnte den Vorteil haben, dass das Positionierungselement aus dem Mundraum des Patienten entfernt werden kann, während die zweite Bohrschablone zusammen mit der ersten Bohrschablone im Mundraum verbleibt. Beispielsweise kann die in dem Mundraum verbleibende zweite Bohrschablone an dem Gegenkiefer unter Verwendung von Fixierungselementen fixiert sein. Beim Einbringen der beiden Bohrschablonen und dem Positionierungselement in den Mundraum des Patienten können ein oder zwei der beiden Bohrschablonen und das Positionierungselement beispielsweise über die entsprechenden zerstörungsfrei lösbaren Verbindungen verbunden sein und gemeinsam eingebracht werden. Beispielsweise können die Bohrschablonen und das Positionierungselement nacheinander in den Mundraum des Patienten eingebracht und dort unter Verwendung der entsprechenden zerstörungsfrei lösbaren Verbindungen miteinander verbunden werden.

Beispielsweise umfasst die zerstörungsfrei lösbare zweite Verbindung eine Steckverbindung. Dies könnte den Vorteil haben, dass eine effiziente zerstörungsfrei lösbare Verbindung bereitgestellt werden kann. Beispielsweise umfassen das Positionierungselement und/oder die zweite Bohrschablone ein oder mehrere weibliche Verbindungselemente, welche zur Aufnahme von ein oder mehreren männlichen Verbindungselementen der zweiten Bohrschablone bzw. des Positionierungselements konfiguriert sind.

Beispielsweise umfasst die zweite Bohrschablone das erste Positionierungselement.

Beispielsweise umfasst das erste Positionierungselement zweite Zugangsöffnungen zu den dritten Durchgangsöffnungen der zweiten Bohrschablone. Dies könnte den Vorteil haben, dass das Positionierungselement zum Einbringen der Implantatbohrungen in den Gegenkiefer im Mundraum des Patienten verbleiben kann. Zugang zu den Durchgangsöffnungen der zweiten Bohrschablone zum Einbringen der Implantatbohrungen in den Gegenkiefer wird beispielsweise über die Zugangsöffnungen des Positionierungselements bereitgestellt.

Beispielsweise sind die zweiten Zugangsöffnungen zugänglich zum Einbringen der Implantatbohrungen in den Gegenkiefer, während mit den Kiefern Druck auf das Positionierungselement ausgeübt wird. Beispielsweise sind die zweiten Zugangsöffnungen zugänglich zum Einbringen der Implantatbohrungen in den Gegenkiefer, wenn mit den Kiefern kein Druck auf das Positionierungselement ausgeübt wird und die Kiefer weiter voneinander entfernt werden, als es in der Erfassungsposition der Fall ist. Beispielsweise sind die Kiefer dazu so weit voneinander zu entfernen, dass die erste Bohrschablone den Kontakt mit dem Positionierungselement verliert. Beispielsweise sind die zweiten Zugangsöffnungen als Kanäle ausgestaltet, welche jeweils zu einer der Durchgangsöffnungen führen. Beispielsweise sind die zweiten Zugangsöffnungen als Ausnehmung aus dem Positionierungselement ausgestaltet. Beispielsweise weist das Positionierungselement zumindest abschnittsweise eine Gitterstruktur auf, deren Zwischenräume die Ausnehmungen bereitstellen. Beispielsweise ist das Positionierungselement in vestibularer Richtung, z.B. labial und/oder bukkal, offen. Beispielsweise ist das Positionierungselement palatinal bzw. lingual geschlossen. Beispielsweise ist das Positionierungselement ausgehöhlt. Beispielsweise umfasst das Positionierungselement zum Stützen des Hohlraums ein oder mehrere Stützstreben, welche sich beispielsweise senkrecht zwischen den beiden Kiefern erstecken.

Beispielsweise umfassen die erste und zweite Bohrschablone das erste Positionierungselement gemeinsam. Dies könnte den Vorteil haben, dass ein gemeinsames Element bereitgestellt werden kann, welches beide Bohrschablonen und das erste Positionierungselement umfasst. Dieses gemeinsame Element kann in den Mundraum des Patienten eingebracht werden. Wenn der Patient mit den Kiefer Druck auf dieses gemeinsame Element aufbringt, genauer gesagt auf die beiden Bohrschablonen und damit auf das Positionierungselement, können die beiden Bohrschablonen beispielsweise jeweils in einer vorgesehenen Position relativ zu dem Kiefer bzw. zu dem Gegenkiefer positioniert und fixiert werden. Ferner können die beiden Kiefer so beispielsweise relativ zueinander in der Erfassungsposition angeordnet werden.

Beispielsweise umfasst das Verfahren ferner ein Herstellen eines zweiten Positionierungselements. Beispielsweise umfasst das Herstellen der zweite Bohrschablone ferner das Herstellen des zweiten Positionierungselements. Das zweite Positionierungselement in Kombination mit dem ersten Positionierungselement ist beispielsweise konfiguriert, die zweite Bohrschablone in der zweiten Position mittels Drucks, welchen der Patient durch ein Schließen der Kiefer auf das erste und zweite Positionierungselement aufbringt, zu fixieren. Form und Abmessungen des zweiten Positionierungselements werden so bestimmt, dass die beiden Kiefer beim Aufbringen des Drucks auf das erste und zweite Positionierungselement in der Erfassungsposition und die zweite Bohrschablone in der zweiten Position relativ zum Gegenkiefer angeordnet werden.

Dies könnte den Vorteil haben, dass für jedes der Bohrschablonen jeweils ein Positionierungselement bereitgestellt werden kann. Die beiden Positionierungselemente in Kombination miteinander können die beiden Bohrschablonen jeweils in der vorgesehenen Position relativ zu dem Kieferknochen bzw. Gegenkieferknochen positionieren und fixieren.

Beispielsweise ist das zweite Positionierungselement in Kombination mit dem ersten Positionierungselement konfiguriert, die zweite Bohrschablone in der zweiten Position zum Einbringen der zweiten Fixierungsbohrungen in den Gegenkiefer für die zweiten Fixierungselemente zu fixieren.

Beispielsweise umfasst das zweite Positionierungselement ein oder mehrere dritte Verbindungselemente, welche konfiguriert sind zum Herstellen und Lösen einer zerstörungsfrei lösbaren dritten Verbindung zwischen dem zweiten Positionierungselement und der zweiten Bohrschablone im Mundraum des Patienten.

Dies könnte den Vorteil haben, dass das zweite Positionierungselement aus dem Mundraum des Patienten entfernt werden kann, während die zweite Bohrschablone zusammen mit der ersten Bohrschablone im Mundraum verbleibt. Beispielsweise kann die in dem Mundraum verbleibende zweite Bohrschablone an dem Gegenkiefer unter Verwendung von Fixierungselementen fixiert sein. Beim Einbringen der beiden Bohrschablonen und der beiden Positionierungselemente in den Mundraum des Patienten können die Bohrschablonen und die Positionierungselemente beispielsweise jeweils paarweise über entsprechende zerstörungsfrei lösbare Verbindungen verbunden sein und gemeinsam eingebracht werden. Beispielsweise können die Bohrschablonen und Positionierungselemente nacheinander in den Mundraum des Patienten eingebracht und dort unter Verwendung der entsprechenden zerstörungsfrei lösbaren Verbindungen miteinander verbunden werden.

Beispielsweise umfasst die zerstörungsfrei lösbare dritte Verbindung eine Steckverbindung. Dies könnte den Vorteil haben, dass eine effiziente zerstörungsfrei lösbare Verbindung bereitgestellt werden kann. Beispielsweise umfassen das zweite Positionierungselement und/oder die zweite Bohrschablone ein oder mehrere weibliche Verbindungselemente, welche zur Aufnahme von ein oder mehreren männlichen Verbindungselementen der zweiten Bohrschablone bzw. des zweiten Positionierungselements konfiguriert sind.

Beispielsweise ist das zweite Positionierungselement von der zweiten Bohrschablone umfasst. Beispielsweise umfasst das zweite Positionierungselement zweite Zugangsöffnungen zu den dritten Durchgangsöffnungen der zweiten Bohrschablone. Dies könnte den Vorteil haben, dass das zweite Positionierungselement zum Einbringen der Implantatbohrungen in den Gegenkiefer im Mundraum des Patienten verbleiben kann. Zugang zu den Durchgangsöffnungen der zweiten Bohrschablone zum Einbringen der Implantatbohrungen in den Gegenkiefer wird beispielsweise über die Zugangsöffnungen des zweiten Positionierungselements bereitgestellt.

Beispielsweise sind die zweiten Zugangsöffnungen zugänglich zum Einbringen der Implantatbohrungen in den Gegenkiefer, während mit den Kiefern Druck auf die Positionierungselemente ausgeübt wird. Beispielsweise sind die zweiten Zugangsöffnungen zugänglich zum Einbringen der Implantatbohrungen in den Gegenkiefer, wenn mit den Kiefern kein Druck auf die Positionierungselemente ausgeübt wird und die Kiefer weiter voneinander entfernt werden, als es in der Erfassungsposition der Fall ist. Beispielsweise sind die Kiefer dazu so weit voneinander zu entfernen, dass das erste Positionierungselement den Kontakt mit dem zweiten Positionierungselement verliert. Beispielsweise sind die zweiten Zugangsöffnungen als Kanäle ausgestaltet, welche jeweils zu einer der Durchgangsöffnungen führen. Beispielsweise sind die zweiten Zugangsöffnungen als Ausnehmung aus dem zweiten Positionierungselement ausgestaltet. Beispielsweise weist das zweite Positionierungselement zumindest abschnittsweise eine Gitterstruktur auf, deren Zwischenräume die Ausnehmungen bereitstellen. Beispielsweise ist das zweite Positionierungselement in vestibularer Richtung, z.B. labial und/oder bukkal, offen. Beispielsweise ist das zweite Positionierungselement palatinal bzw. lingual geschlossen. Beispielsweise ist das zweite Positionierungselement ausgehöhlt. Beispielsweise umfasst das zweite Positionierungselement zum Stützen des Hohlraums ein oder mehrere Stützstreben, welche sich beispielsweise senkrecht zwischen den beiden Kiefern erstecken.

Beispielsweise umfasst das erste Positionierungselement ein oder mehrere vierte Verbindungselemente und das zweite Positionierungselement ein oder mehrere fünfte Verbindungselemente. Die vierten und fünften Verbindungselemente sind konfiguriert zum Herstellen und Lösen einer zerstörungsfrei lösbaren vierten Verbindung zwischen den beiden Positionierungselementen im Mundraum des Patienten.

Dies könnte den Vorteil haben, dass die Verbindung zwischen den beiden Positionierungselementen in dem Mundraum des Patienten gelöst werden kann. Dies kann beispielsweise zum Erleichterten des Einbringens der Implantatbohrungen in die Kiefer erfolgen, während die Positionierungselemente im Mundraum des Patienten verbleiben. Dies könnte den Vorteil haben, dass die beiden Positionierungselemente unabhängig voneinander, beispielsweise nacheinander, aus dem Mundraum des Patienten entfernt werden können, während die Bohrschablonen in dem Mundraum verbleiben. Beispielsweise wird nur eines der beiden Positionierungselemente aus dem Mundraum entfernt. Beispielsweise werden beide Positionierungselemente aus dem Mundraum entfernt. Beispielsweise können die in dem Mundraum verbleibenden Bohrschablonen an den Kiefern unter Verwendung von Fixierungselementen fixiert sein. Beim Einbringen der Bohrschablonen und Positionierungselemente in den Mundraum des Patienten können die Bohrschablonen und Positionierungselemente jeweils paarweise beispielsweise über die entsprechenden zerstörungsfrei lösbaren Verbindungen verbunden sein und gemeinsam eingebracht werden. Beispielsweise können beide Bohrschablonen und beide Positionierungselemente lösbar miteinander verbunden zusammen in dem Mundraum eingebracht werden. Die Bohrschablonen und Positionierungselemente können beispielsweise nacheinander in den Mundraum des Patienten eingebracht und dort unter Verwendung der entsprechenden zerstörungsfrei lösbaren Verbindungen nach und nach miteinander verbunden werden.

Beispielsweise umfasst die zerstörungsfrei lösbare vierte Verbindung eine Steckverbindung. Dies könnte den Vorteil haben, dass eine effiziente zerstörungsfrei lösbare Verbindung bereitgestellt werden kann. Beispielsweise umfassen das erste Positionierungselement und/oder das zweite Positionierungselement ein oder mehrere weibliche Verbindungselemente, welche zur Aufnahme von ein oder mehreren männlichen Verbindungselementen des zweiten Positionierungselements bzw. des ersten Positionierungselements konfiguriert sind.

Beispielsweise werden die zweiten Implantatpositionen der zweiten Implantatbohrungen und/oder zweiten Fixierungspositionen der zweiten Fixierungsbohrungen jeweils unter Erfüllung eines oder mehrerer der folgenden ersten Positionierungskriterien festgelegt: Erstrecken der zweiten Implantatbohrungen und/oder zweiten Fixierungsbohrungen jeweils durch eine Knochenwand der Gegenkieferknochenstruktur mit einer vordefinierten ersten Mindeststärke, Einhalten jeweils eines vordefinierten ersten Mindestabstands der zweiten Implantatbohrungen und/oder zweiten Fixierungsbohrungen von Hauptnerven im Gegenkiefer, Einhalten jeweils eines vordefinierten zweiten Mindestabstands der zweiten Implantatbohrungen und/oder zweiten Fixierungsbohrungen von Adern im Gegenkiefer.

Dies könnte den Vorteil haben, dass durch Einhalten der Mindeststärke der Knochenwand beispielsweise ein ausreichender Halt für die Implantate und/oder Fixierungselemente sichergestellt werden kann. Durch das Einhalten des Mindestabstands zu Hauptnerven ein Verletzen der entsprechenden Hauptnerven verhindert werden kann. Durch das Einhalten des Mindestabstands zu Adern ein Verletzen der entsprechenden verhindert werden kann.

Beispielsweise sind die zweite Bohrschablone für die dritten Durchgangsöffnungen und/oder vierten Durchgangsöffnungen jeweils mit einem Bohrführungselement versehen, welches dazu konfiguriert ist, ein Bohrwerkzeug beim Einbringen der zweiten Implantatbohrungen und/oder zweiten Fixierungsbohrungen zu führen und/oder eine erste Bohrtiefe der zweiten Implantatbohrungen und/oder zweiten Fixierungsbohrungen zu begrenzen.

Dies könnte den Vorteil haben, dass das Bohrwerkzeug durch die Bohrführungselemente jeweils geführt werden kann. Die Bohrführungselemente können beispielsweise durch die dritten Durchgangsöffnungen und/oder vierten Durchgangsöffnungen bereitgestellt werden, welche zumindest abschnittsweise als Kanäle konfiguriert sind, welche das Bohrwerkzeug führen. Beispielsweise sind die Bohrführungselemente jeweils an den dritten Durchgangsöffnungen und/oder vierten Durchgangsöffnungen angeordnet, beispielsweise in Form von Fortsätzen, welche jeweils einen Durchgangskanal zum Führen des Bohrwerkzeugs aufweisen. Beispielsweise umfassen die Bohrführungselemente jeweils einen Anschlag durch welchen ein tieferes Einbringen des Bohrwerkzeugs in das Bohrführungselement und damit der Bohrtiefe begrenzt werden kann. Der Anschlag kann beispielsweise durch eine Mündung eines Kanals des Bohrführungselements oder einen Übergang zwischen zwei Durchmessern des Kanals des Bohrführungselements bereitgestellt werden. Beispielsweise ist ein innerer Durchmesser kleiner als ein äußerer Durchmesser. Beispielsweise schlägt an den Anschlag ein Abschnitt eines Haltelements des Bohrwerkzeugs bzw. eines Bohreinsatzes an.

Beispielsweise sind das erste und/oder das zweite Positionierungselement für die dritten Durchgangsöffnungen und/oder vierten Durchgangsöffnungen jeweils mit einem dritten Bohrführungselement versehen, welches dazu konfiguriert ist, das Bohrwerkzeug beim Einbringen der zweiten Implantatbohrungen und/oder zweiten Fixierungsbohrungen zu führen und/oder die erste Bohrtiefe der zweiten Implantatbohrungen und/oder zweiten Fixierungsbohrungen zu begrenzen.

Dies könnte den Vorteil haben, dass das Bohrwerkzeug durch die Bohrführungselemente jeweils geführt werden kann. Die Bohrführungselemente für die dritten Durchgangsöffnungen können beispielsweise durch die zweiten Zugangsöffnungen bereitgestellt werden, welche zumindest abschnittsweise als Kanäle konfiguriert sind, welche das Bohrwerkzeug führen. Beispielsweise sind die Bohrführungselemente jeweils an den dritten Durchgangsöffnungen und/oder vierten Durchgangsöffnungen angeordnet, beispielsweise in Form von Fortsätzen, welche jeweils einen Durchgangskanal zum Führen des Bohrwerkzeugs aufweisen. Beispielsweise umfassen die Bohrführungselemente jeweils einen Anschlag durch welchen ein tieferes Einbringen des Bohrwerkzeugs in das Bohrführungselement und damit der Bohrtiefe begrenzt werden kann. Der Anschlag kann beispielsweise durch eine Mündung eines Kanals des Bohrführungselements oder einen Übergang zwischen zwei Durchmessern des Kanals des Bohrführungselements bereitgestellt werden. Beispielsweise ist ein innerer Durchmesser kleiner als ein äußerer Durchmesser. Beispielsweise schlägt an den Anschlag ein Abschnitt eines Haltelements des Bohrwerkzeugs bzw. eines Bohreinsatzes an.

Beispielsweise werden die ersten Implantatpositionen der ersten Implantatbohrungen und/oder ersten Fixierungspositionen der ersten Fixierungsbohrungen jeweils unter Erfüllung eines oder mehrerer der folgenden zweiten Positionierungskriterien festgelegt: Erstrecken der ersten Implantatbohrungen und/oder ersten Fixierungsbohrungen jeweils durch eine Knochenwand der Kieferknochenstruktur mit einer vordefinierten zweiten Mindeststärke, Einhalten jeweils eines vordefinierten dritten Mindestabstands der ersten Implantatbohrungen und/oder ersten Fixierungsbohrungen von Hauptnerven im Kiefer, Einhalten jeweils eines vordefinierten vierten Mindestabstands der ersten Implantatbohrungen und/oder ersten Fixierungsbohrungen von Adern im Kiefer.

Dies könnte den Vorteil haben, dass durch Einhalten der Mindeststärke der Knochenwand beispielsweise ein ausreichender Halt für die Implantate und/oder Fixierungselemente sichergestellt werden kann. Durch das Einhalten des Mindestabstands zu Hauptnerven ein Verletzen der entsprechenden Hauptnerven verhindert werden kann. Durch das Einhalten des Mindestabstands zu Adern ein Verletzen der entsprechenden verhindert werden kann. Beispielsweise ist die erste Bohrschablone für die ersten Durchgangsöffnungen und/oder zweiten Durchgangsöffnungen jeweils mit einem dritten Bohrführungselement versehen, welches dazu konfiguriert ist, das Bohrwerkzeug beim Einbringen der ersten Implantatbohrungen und/oder ersten Fixierungsbohrungen zu führen und/oder eine zweite Bohrtiefe der ersten Implantatbohrungen und/oder ersten Fixierungsbohrungen zu begrenzen.

Dies könnte den Vorteil haben, dass das Bohrwerkzeug durch die Bohrführungselemente jeweils geführt werden kann. Die Bohrführungselemente können beispielsweise durch die ersten Durchgangsöffnungen und/oder zweiten Durchgangsöffnungen bereitgestellt werden, welche zumindest abschnittsweise als Kanäle konfiguriert sind, welche das Bohrwerkzeug führen. Beispielsweise sind die Bohrführungselemente jeweils an den ersten Durchgangsöffnungen und/oder zweiten Durchgangsöffnungen angeordnet, beispielsweise in Form von Fortsätzen, welche jeweils einen Durchgangskanal zum Führen des Bohrwerkzeugs aufweisen. Beispielsweise umfassen die Bohrführungselemente jeweils einen Anschlag durch welchen ein tieferes Einbringen des Bohrwerkzeugs in das Bohrführungselement und damit der Bohrtiefe begrenzt werden kann. Der Anschlag kann beispielsweise durch eine Mündung eines Kanals des Bohrführungselements oder einen Übergang zwischen zwei Durchmessern des Kanals des Bohrführungselements bereitgestellt werden. Beispielsweise ist ein innerer Durchmesser kleiner als ein äußerer Durchmesser. Beispielsweise schlägt an den Anschlag ein Abschnitt eines Haltelements des Bohrwerkzeugs bzw. eines Bohreinsatzes an.

Beispielsweise ist das erste Positionierungselement für die ersten Durchgangsöffnungen und/oder zweiten Durchgangsöffnungen jeweils mit einem vierten Bohrführungselement versehen, welches dazu konfiguriert ist, das Bohrwerkzeug beim Einbringen der ersten Implantatbohrungen und/oder ersten Fixierungsbohrungen zu führen und/oder die zweite Bohrtiefe der ersten Implantatbohrungen und/oder ersten Fixierungsbohrungen zu begrenzen.

Dies könnte den Vorteil haben, dass das Bohrwerkzeug durch die Bohrführungselemente jeweils geführt werden kann. Die Bohrführungselemente für die ersten Durchgangsöffnungen können beispielsweise durch die ersten Zugangsöffnungen bereitgestellt werden, welche zumindest abschnittsweise als Kanäle konfiguriert sind, welche das Bohrwerkzeug führen. Beispielsweise sind die Bohrführungselemente jeweils an den ersten Durchgangsöffnungen und/oder zweiten Durchgangsöffnungen angeordnet, beispielsweise in Form von Fortsätzen, welche jeweils einen Durchgangskanal zum Führen des Bohrwerkzeugs aufweisen. Beispielsweise umfassen die Bohrführungselemente jeweils einen Anschlag durch welchen ein tieferes Einbringen des Bohrwerkzeugs in das Bohrführungselement und damit der Bohrtiefe begrenzt werden kann. Der Anschlag kann beispielsweise durch eine Mündung eines Kanals des Bohrführungselements oder einen Übergang zwischen zwei Durchmessern des Kanals des Bohrführungselements bereitgestellt werden. Beispielsweise ist ein innerer Durchmesser kleiner als ein äußerer Durchmesser. Beispielsweise schlägt an den Anschlag ein Abschnitt eines Haltelements des Bohrwerkzeugs bzw. eines Bohreinsatzes an.

Beispielsweise werden die ersten und/oder zweiten Strukturdaten unter Verwendung eines ersten Scan-Verfahren erfasst, bei welchem es sich um eines der folgenden Verfahren handelt: ein Computertomographie-Verfahren, ein Volumentomographie-Verfahren oder ein Magnetresonanztomographie-Verfahren. Dies könnte den Vorteil haben, dass für das Erfassen der ersten und/oder zweiten Strukturdaten beispielsweise ein Scan-Verfahren verwendet werden kann, welches zum Erfassen der entsprechenden Strukturdaten optimiert ist.

Beispielsweise werden die Positionsdaten des Bissregistrats unter Verwendung von Material des Bissregistrats erfasst, welches opak für das erste Scan-Verfahren ist. Dies könnte den Vorteil haben, dass das Bissregistrat für das erste Scan-Verfahren zumindest teilweise sichtbar ist.

Beispielsweise werden die Positionsdaten des Bissregistrats unter Verwendung von Markern erfasst, welche das Bissregistrat umfasst und welche opak für das erste Scan-Verfahren sind. Dies könnte den Vorteil haben, dass zumindest die Marker für das erste Scan-Verfahren sichtbar sind und anhand der Positionsdaten der Marker relativ zu dem Kiefer und/oder Gegenkiefer die Positionsdaten des Bissregistrats relativ zu dem Kiefer und/oder Gegenkiefer bestimmt werden können. Beispielsweise umfasst das Bissregistrat zumindest drei, nicht auf einer gemeinsamen Geraden angeordnete Marker. Beispielsweise umfasst das Bissregistrat mehr als drei Marker.

Beispielsweise werden die Positionsdaten des Bissregistrats unter Verwendung eines Kontakts und/oder von Kontaktpunkten zwischen dem zweiten Abdruck und einem dentalen Objekt des Gegenkiefers erfasst, welches opak für das erste Scan-Verfahren ist. Dies könnte den Vorteil haben, dass anhand der Position des dentalen Objekts bzw. eines mit dem zweiten Abdruck in Kontakt tretenden Abschnitts des Kontakts des dentalen Objekts relativ zu dem Kieferknochen die Positionsdaten des Bissregistrats relativ zu dem Kieferknochen und/oder Gegenkieferknochen bestimmt werden können

Beispielsweise werden die ersten und/oder zweiten Formdaten unter Verwendung eines zweiten Scan-Verfahren erfasst, bei welchem es sich um eines der folgenden Verfahren handelt: ein optisches Verfahren, ein Computertomographie-Verfahren, ein haptisches bzw. taktiles Verfahren. Dies könnte den Vorteil haben, dass für das Erfassen der ersten und/oder zweiten Formdaten beispielsweise ein Scan-Verfahren verwendet werden kann, welches zum Erfassen der entsprechenden Formdaten optimiert ist.

Beispielsweise sind die Marker des Bissregisters, welche opak, d.h. sichtbar, für das erste Scan-Verfahren sind ebenfalls opak, d.h. sichtbar, für das zweite Scan-Verfahren.

Beispielsweise umfasst das Verfahren ferner ein Erfassen eines auf das zwischen den beiden Kiefern des Patienten angeordnete Bissregistrat aufgebrachten Erfassungsdrucks. Beispielsweise umfasst das erste Positionierungselement und/oder das zweite Positionierungselement ein Druckerfassungselement zum Prüfen, ob der in der Erfassungsposition der beiden Kiefer auf das erste Positionierungselement und/oder das zweite Positionierungselement aufgebrachte Druck mit dem Erfassungsdruck übereinstimmt.

Dies könnte den Vorteil haben, dass sichergestellt werden kann, dass beim Positionieren und/oder Fixieren der Bohrschablone bzw. der Bohrschablonen mit den Kiefern derselbe Druck auf das Positionierungselement bzw. die Positionierungselemente aufgebracht wird, wie beim Erfassen der Strukturdaten, d.h. der Erfassungsdruck. Der mit den Kiefern aufgebrachte Druck kann zu Verformungen der weichen Gingiva führen. Ein verwenden desselben Drucks kann den Vorteil haben, dass es zu denselben Verformungen der Gingiva kommt. Beispielsweise umfasst auch das Bissregistrat ein Druckerfassungselement.

Das Druckerfassungselement kann beispielsweise einen piezoelektrischen Sensor, ein druckabhängiges Formelement und/oder ein druckabhängiges Farbelement umfassen. Beispielsweise ändert sich die Form der druckabhängige Formelemente in Abhängigkeit von dem aufgebrachten Druck oder die Form der druckabhängige Formelemente tritt mit zunehmendem Druck beispielsweise immer stärker hervor. Beispielsweise ändert sich der Farbe der druckabhängige Farbelemente in Abhängigkeit von dem aufgebrachten Druck. Beispielsweise wird eine drucksensitive Farbe verwendet.

Beispielsweise wird ferner ein Positionierungselement zum Fixieren einer ersten Bohrschablone auf einer Gingiva eines Kiefers eines Patienten in einer ersten Position relativ zu einem Kieferknochen des Kiefers bereitgestellt. Das Positionierungselement ist konfiguriert, die erste Bohrschablone mittels Drucks, welchen der Patient durch ein Schließen des Kiefers und eines Gegenkiefers auf das Positionierungselement aufbringt, in der ersten Position zu fixieren zum Einbringen von ersten Implantatbohrungen in den Kiefer für erste dentale Implantate. Das Positionierungselement ist hergestellt unter Verwendung eines Verfahrens, welches umfasst:
- Erfassen von ersten Formdaten eines ersten Abdrucks der Oberfläche der Gingiva des Kiefers unter Verwendung eines zwischen den beiden Kiefern des Patienten angeordneten Bissregistrats, wobei sich die beiden Kiefer in einer Erfassungsposition relativ zueinander befinden, wobei sich der erste Abdruck in der ersten Position relativ zu dem Kieferknochen befindet und wobei der erste Abdruck eine erste Schablonenkontaktfläche einer ersten Bohrschablone zum Herstellen eines Kontakts mit einer Oberfläche der Gingiva des Kiefers definiert,
- Erfassen von ersten Strukturdaten des Kieferknochens, während das Bissregistrat zwischen den beiden Kiefern in der Erfassungsposition angeordnet ist,
- Erfassen von Positionsdaten des Bissregistrats relativ zu dem Kieferknochen, während das Bissregistrat zwischen den beiden Kiefern in der Erfassungsposition angeordnet ist,
- Erstellen eines ersten dreidimensionalen digitalen Modells unter Verwendung der erfassten ersten Formdaten, ersten Strukturdaten und Positionsdaten, wobei das erste Modell die Kieferknochenstruktur und die Oberfläche der Gingiva des Kiefers umfasst,
- Festlegen erster Implantatpositionen zum Einbringen der ersten Implantatbohrungen in den Kiefer unter Verwendung des ersten Modells,
- Erstellen eines zweiten dreidimensionalen digitalen Modells der ersten Bohrschablone und des Positionierungselements unter Verwendung des ersten Modells und der festgelegten ersten Implantatpositionen, wobei Form und Abmessungen des Positionierungselements so bestimmt werden, dass die beiden Kiefer beim Aufbringen von Druck auf das Positionierungselement in der Erfassungsposition und die erste Bohrschablone in der ersten Position relativ zu dem Kieferknochen angeordnet werden, wobei die erste Bohrschablone die erste Schablonenkontaktfläche und erste Durchgangsöffnungen umfasst, wobei die ersten Durchgangsöffnungen jeweils eine der ersten Implantatpositionen definieren,
- wobei das zweite dreidimensionale digitale Modell zum Herstellen des Positionierungselements verwendet wird.

Beispielsweise handelt es sich bei dem Positionierungselement um ein Positionierungselement, welches mit einem der zuvor beschriebenen Verfahren zum Herstellen eines Positionierungselements hergestellt wurde. Beispielsweise handelt es sich bei dem Kiefer des Patienten um einen zahnlosen Kiefer.

Beispielsweise ist das Positionierungselement konfiguriert, die erste Bohrschablone in der ersten Position zum Einbringen von ersten Fixierungsbohrungen in den Kiefer für erste Fixierungselemente zu fixieren. Die erste Bohrschablone umfasst beispielsweise zweite Durchgangsöffnungen, welche jeweils eine der ersten Fixierungspositionen definieren. Die ersten Fixierungselemente sind beispielsweise konfiguriert zum kieferdruckunabhängigen Fixieren der ersten Bohrschablone in der ersten Position zum Einbringen der ersten Implantatbohrungen in den Kiefer für die ersten dentale Implantate. Das Verfahren umfasst beispielsweise ferner ein Festlegen erster Fixierungspositionen zum Einbringen der ersten Fixierungsbohrungen und erster Implantatpositionen zum Einbringen der ersten Implantatbohrungen in den Kiefer unter Verwendung des ersten Modells. Das zweite dreidimensionale digitale Modell der ersten Bohrschablone und des Positionierungselements wird beispielsweise ferner unter Verwendung der festgelegten ersten Fixierungspositionen erstellt.

Beispielsweise wird ferner eine Kombination aus dem Positionierungselement nach einem der vorangehenden Beispiele für Positionierungselements und einer unter Verwendung des zweiten dreidimensionalen digitalen Modells hergestellten ersten Bohrschablone bereitgestellt.

Beispielsweise handelt es sich bei der ersten Bohrschablone um eine Bohrschablone, welche mit einem der zuvor beschriebenen Verfahren zum Herstellen einer Bohrschablone hergestellt wurde.

Beispielsweise umfasst das Positionierungselement ein oder mehrere erste Verbindungselemente, welche konfiguriert sind zum Herstellen und Lösen einer zerstörungsfrei lösbaren ersten Verbindung zwischen dem Positionierungselement und der ersten Bohrschablone im Mundraum des Patienten.

Beispielsweise umfasst die erste Bohrschablone das Positionierungselement. Beispielsweise umfasst das Positionierungselement erste Zugangsöffnungen zu den ersten Durchgangsöffnungen der ersten Bohrschablone.

Beispielsweise umfasst das Positionierungselement eine Kieferkontaktfläche zum Herstellen eines Kontakts mit einer Oberfläche des Gegenkiefers. Die Kieferkontaktfläche ist von einem zweiten unter Verwendung des zwischen den beiden Kiefern in der Erfassungsposition angeordneten Bissregistrats erfassten Abdruck der Oberfläche des Gegenkiefers definiert.

Beispielsweise umfasst die Kombination ferner eine zweite Bohrschablone, welche eine zweite Schablonenkontaktfläche zum Herstellen eines Kontakts mit der Oberfläche des Gegenkiefers umfasst. Der zweite Abdruck die zweite Schablonenkontaktfläche definiert. Die Oberfläche des Gegenkiefers ist eine Oberfläche der Gingiva des Gegenkiefers. Die zweite Bohrschablone umfasst ferner dritte Durchgangsöffnungen. Die dritten Durchgangsöffnungen definieren jeweils eine zweite Implantatposition zum Einbringen einer der zweiten Implantatbohrungen.

Beispielsweise handelt es sich bei der zweiten Bohrschablone um eine Bohrschablone, welche mit einem der zuvor beschriebenen Verfahren zum Herstellen einer Bohrschablone hergestellt wurde.

Beispielsweise ist das Positionierungselement ferner konfiguriert, die zweite Bohrschablone in der zweiten Position mittels Drucks, welchen der Patient durch ein Schließen der beiden Kiefer auf das Positionierungselement aufbringt, zu fixieren. Form und Abmessungen des Positionierungselements sind ferner so bestimmt, dass die zweite Bohrschablone in der zweiten Position relativ zum Gegenkiefer angeordnet wird.

Beispielsweise ist das Positionierungselement konfiguriert, die zweite Bohrschablone in der zweiten Position zum Einbringen von zweiten Fixierungsbohrungen in den Gegenkiefer für zweite Fixierungselemente zu fixieren. Die zweite Bohrschablone umfasst beispielsweise ferner vierte Durchgangsöffnungen, welche jeweils eine zweite Fixierungsposition zum Einbringen einer der zweiten Fixierungsbohrungen definieren. Die zweiten Fixierungselemente sind beispielsweise konfiguriert zum kieferdruckunabhängigen Fixieren der zweiten Bohrschablone in einer zweiten Position relativ zum Gegenkieferknochen zum Einbringen von zweiten Implantatbohrungen in den Gegenkiefer für zweite dentale Implantate.

Beispielsweise ist das Positionierungselement ferner konfiguriert, die zweite Bohrschablone in der zweiten Position zum Einbringen der zweiten Fixierungsbohrungen in den Gegenkiefer für die zweiten Fixierungselemente zu fixieren.

Beispielsweise umfasst das Positionierungselement ferner ein oder mehrere zweite Verbindungselemente, welche konfiguriert sind zum Herstellen und Lösen einer zerstörungsfrei lösbaren zweiten Verbindung zwischen dem Positionierungselement und der zweiten Bohrschablone im Mundraum des Patienten.

Beispielsweise umfasst die zweite Bohrschablone das Positionierungselement. Beispielsweise umfasst das Positionierungselement zweite Zugangsöffnungen zu den dritten Durchgangsöffnungen der zweiten Bohrschablone. Beispielsweise umfassen die erste und zweite Bohrschablone gemeinsam das Positionierungselement.

Beispielsweise umfasst die Kombination ferner ein zweites Positionierungselement. Das zweite Positionierungselement in Kombination mit dem Positionierungselement ist konfiguriert, die zweite Bohrschablone in der zweiten Position mittels Drucks, welchen der Patient durch ein Schließen der Kiefer auf die beiden Positionierungselemente aufbringt, zu fixieren. Form und Abmessungen des zweiten Positionierungselements werden so bestimmt, dass die beiden Kiefer beim Aufbringen des Drucks auf die beiden Positionierungselemente in der Erfassungsposition und die zweite Bohrschablone in der zweiten Position relativ zum Gegenkiefer angeordnet werden.

Beispielsweise handelt es sich bei dem zweiten Positionierungselement um ein Positionierungselement, welches mit einem der zuvor beschriebenen Verfahren zum Herstellen eines Positionierungselements hergestellt wurde.

Beispielsweise ist das zweite Positionierungselement in Kombination mit dem Positionierungselement konfiguriert, die zweite Bohrschablone in der zweiten Position zum Einbringen der zweiten Fixierungsbohrungen in den Gegenkiefer für die zweiten Fixierungselemente zu fixieren.

Beispielsweise umfasst das zweite Positionierungselement ein oder mehrere dritte Verbindungselemente, welche konfiguriert sind zum Herstellen und Lösen einer zerstörungsfrei lösbaren dritten Verbindung zwischen dem zweiten Positionierungselement und der zweiten Bohrschablone im Mundraum des Patienten.

Beispielsweise ist das zweite Positionierungselement von der zweiten Bohrschablone umfasst. Beispielsweise umfasst das zweite Positionierungselement zweite Zugangsöffnungen zu den dritten Durchgangsöffnungen der zweiten Bohrschablone.

Beispielsweise umfasst das Positionierungselement ein oder mehrere vierte Verbindungselemente und das zweite Positionierungselement umfasst ein oder mehrere fünfte Verbindungselemente. Die vierten und fünften Verbindungselemente sind konfiguriert zum Herstellen und Lösen einer zerstörungsfrei lösbaren vierten Verbindung zwischen dem ersten und dem zweiten Positionierungselement im Mundraum des Patienten.

Im Weiteren werden Beispiele der Erfindung mit Bezugnahme auf die Zeichnungen näher erläutert. Es zeigen:
- Figuren 1: ein exemplarisches Bissregistrat zwischen Kiefern sowie Daten der Kieferknochen und des Bissregistrats,
- Figuren 2: exemplarische Positionierungselemente und Bohrschablonen,
- Figuren 3: ein exemplarisches Bissregistrat und ein exemplarisches Positionierungselemente mit Druckerfassungselementen,
- Figuren 4: exemplarische Positionierungselemente und Bohrschablonen,
- Figuren 5: ein exemplarisches Bissregistrat zwischen Kiefern sowie Daten der Kieferknochen und des Bissregistrats,
- Figuren 6: exemplarische Positionierungselemente und Bohrschablonen,
- Figuren 7: exemplarische Positionierungselemente und Bohrschablonen,
- Figuren 8: ein exemplarisches Bissregistrat und ein exemplarisches Positionierungselemente mit Druckerfassungselementen,
- Figuren 9: exemplarische Kombinationen schematischer Positionierungselemente und Bohrschablonen,
- Figur 10: ein exemplarisches Computersystem zum Erstellen digitaler Modelle von Positionierungselementen und Bohrschablonen,
- Figur 11: ein exemplarisches Computersystem zum Erstellen digitaler Modelle von Positionierungselementen und Bohrschablonen,
- Figur 12: eine exemplarische Herstellungsvorrichtung zum Herstellen von Positionierungselementen und Bohrschablonen,
- Figur 13: eine exemplarische Herstellungsvorrichtung zum Herstellen von Positionierungselementen und Bohrschablonen,
- Figur 14: ein schematisches Flussdiagramm eines exemplarischen Verfahrens zum Herstellen eines Positionierungselements und
- Figur 15: ein schematisches Flussdiagramm eines exemplarischen Verfahrens zum Herstellen und Verwenden eines Positionierungselements.

Elemente der nachfolgenden Beispiele, die einander entsprechen, werden mit denselben Bezugszeichen gekennzeichnet.

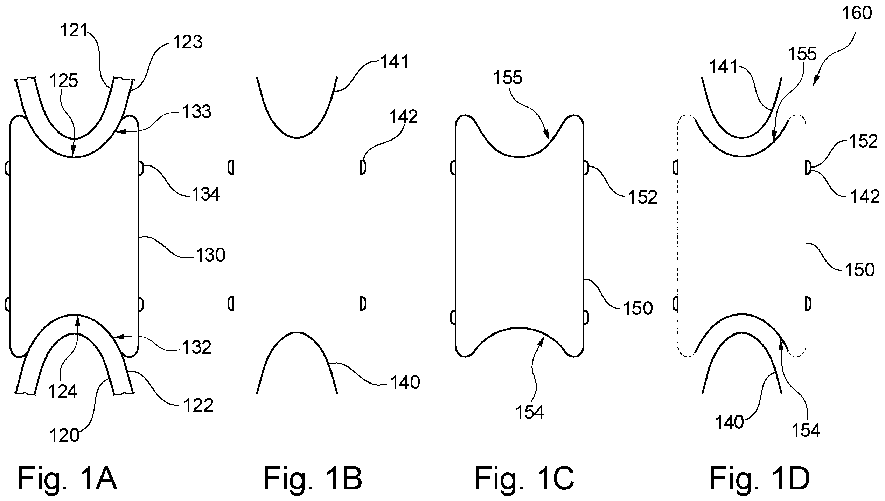

Figuren 1A bis 1D zeigen Querschnitte eines exemplarischen Bissregistrats 130 zwischen Kiefern sowie Daten der Kieferknochen 140, 141 und des Bissregistrats 150. In Figur 1A ist ein Querschnitt durch einen Kieferknochen 120 mit einer darauf angeordneten Gingiva 122 sowie einem Gegenkieferknochen 121 und einer drauf angeordneten Gingiva 123 gezeigt. Zwischen den beiden Kiefern ist ein Bissregistrat 130 angeordnet, welches beispielsweise Marker 134 umfasst. Die Marker 134 sind beispielsweise opak, d.h. sichtbar, für ein Scan-Verfahren zum Erfassen der Strukturen der beiden Kieferknochen 120, 121. Der Patient beißt auf das Bissregistrat 123, wodurch Abdrücke 132, 133 in dem Bissregistrat erzeugt werden. Die geometrische Form des Abdruck 132 entspricht beispielsweise der geometrischen Form einer Oberfläche 124 der Gingiva 122 des Kiefers, d.h. ist deren Negativ, während die geometrische Form des Abdruck 133 beispielsweise der geometrischen Form einer Oberfläche 125 der Gingiva 132 des Gegenkiefers entspricht, d.h. deren Negativ ist. Beispielsweise sind beide Kiefer zahnlos.

In Figur 1B sind die Scan-Daten eines Scans der Strukturen des Kieferknochens 120 und Gegenkieferknochens 121 gezeigt. Die Scan-Daten umfassen neben Scan-Daten 140 des Kieferknochens 120 und Scan-Daten 141 des Gegenkieferknochens 121 Scan-Daten 142 der Marker 134. Beispielsweise könnten auch andere Teile des Bissregistrats 130 oder das gesamte Bissregistrat 130 opak, d.h. sichtbar, für das Scan-Verfahren zum Erfassen der Strukturen der beiden Kieferknochen 120, 121 sein. Falls andere Teile des Bissregistrats 130 oder das gesamte Bissregistrat 130 opak sind, würde die Figur 1B ferner Scan-Daten der entsprechenden Teile des Bissregistrats 130 oder des gesamten Bissregistrats 130 umfassen.

In Figur 1C sind Scan-Daten eines zweiten Scan-Verfahrens zum Erfassen von Formdaten des Bissregistrats 130, insbesondere der Abdrücke 132, 133, gezeigt. Die Scan-Daten 150 des Bissregistrats umfassen beispielsweise zumindest Scan-Daten 154, 155 der Abdrücke 132, 133 sowie Scan-Daten 152 der Marker 134. Die Scan-Daten 150 können beispielsweise auch die gesamte Oberfläche des Bissregistrats 130 umfassen. Falls das gesamte Bissregistrat 130 opak für das Scan-Verfahren zum Erfassen der Strukturen der beiden Kieferknochen 120, 121 ist, könnte das zweite Scan-Verfahren beispielsweise auch entfallen, da dann bereits das erste Scan-Verfahren alle Oberflächendaten des Bissregistrats 130 umfassen könnte.

In Figur 1D ist ein Querschnitt durch ein digitales Modell 160 der Kieferknochen 120, 121 und des Bissregistrats 130 gezeigt. Das digitale Modell 160 umfasst die Scan-Daten 140 des Kieferknochens 120, die Scan-Daten 141 des Gegenkieferknochens 121 sowie die Scan-Daten 154, 155 der Abdrücke 132, 133. Die relative Position des Bissregistrats 130 zu den Kiefernknochen 120, 121 wird dadurch bestimmt, dass die mit den beiden Scan-Verfahren erfassten Scan-Daten 142, 152 der Marker 134 zur Deckung gebracht werden. Die Scan-Daten 142, 152 der Marker 134 stellen also Positionsdaten des Bissregistrats 130 relativ zu den Kieferknochen 120, 121 dar. Durch die Scan-Daten 154, 155 des Kieferknochens 140 und Gegenkieferknochens 141 sowie den Scan-Daten 154, 155 der Abdrücke 132, 133 werden ferner beispielsweise die Konturen der Gingivae 122, 123 indirekt erfasst, obwohl die Gingivae 122, 123 durch die Scan-Verfahren beispielsweise nicht direkt erfasst werden. Die Scan-Daten 154, 155 der Abdrücke 132, 133 dienen beispielsweise als Vorlagen für die geometrische Form von Schablonenkontaktflächen zu erstellender Bohrschablonen. Dabei definiert die Position der Scan-Daten 154, 155 der Abdrücke 132, 133 relative zu den die Scan-Daten 154, 155 der Kieferknochen 140, 141 beispielsweise eine Referenzposition für die Schablonenkontaktflächen relativ zu den Kieferknochen 140, 141.

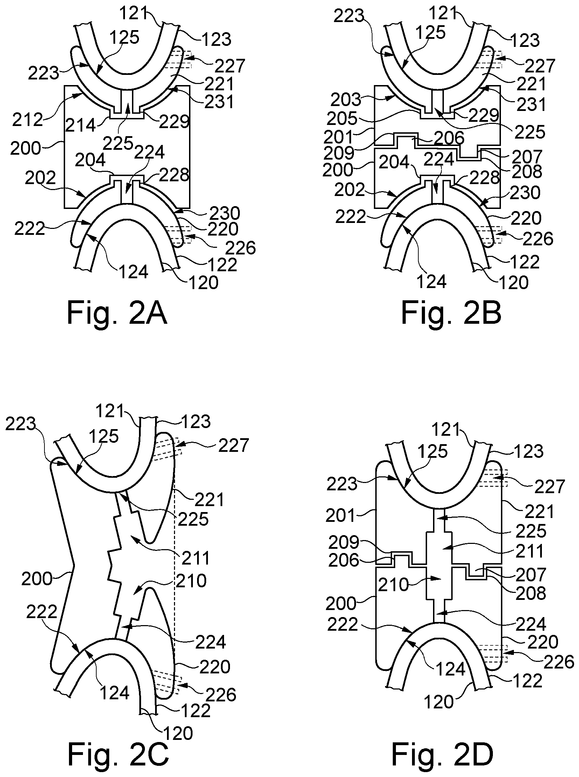

Figuren 2A bis 2D zeigen Querschnitte exemplarischer Positionierungselemente und Bohrschablonen. Die entsprechenden Positionierungselemente und Bohrschablonen entsprechen digitalen Modellen der Positionierungselemente und Bohrschablonen, welche an einem Computer unter Verwendung des digitalen Modells 160 erstellt werden. Dabei werden die digitalen Modelle der Positionierungselemente und Bohrschablonen beispielsweise an die von dem digitalen Modell 160 bereitgestellten geometrischen Bedingungen im Mundraum des Patienten angepasst. Figur 2A zeigt zwei Bohrschablonen 220, 221, welche auf der Gingiva 122 des Kiefers bzw. Kieferknochens 120 und der Gingiva 123 des Gegenkiefers bzw. Gegenkieferknochens 121 angeordnet sind. Die Bohrschablonen 220, 221 umfassen jeweils eine Schablonenkontaktfläche 222, 223, deren geometrische Form durch die mit dem Bissregistrat 130 erfassten Abdrücke 132, 133 der Gingivaoberflächen 124 bzw. 125 vorgegeben sind. Die Bohrschablonen 220, 221 umfassen beispielsweise jeweils Durchgangsöffnungen 224, 225 zum Einbringen von Implantatbohrungen durch die Gingivae 122, 123 hindurch in die Kieferknochen 120, 121. Ferner umfassen die Bohrschablonen 220, 221 beispielsweise jeweils weitere Durchgangsöffnungen 226, 227 zum Einbringen von Fixierungsbohrungen durch die Gingivae 122, 123 hindurch in die Kieferknochen 120, 121. In den Fixierungsbohrungen können Fixierungselemente zum Fixieren der Bohrschablonen 220, 221 in den durch das Positionierungselement 200 vorgegebenen Positionen relativ zu den Kieferknochen 120, 121 angeordnet werden. Beispielsweise sind beide Kiefer zahnlos. Die Bohrschablonen 220, 221 werden unter Verwendung des zwischen diesen angeordneten Positionierungselements 200 in Positionen relativ zu den Kieferknochen 120, 121 positioniert und fixiert. Dabei sind Form und Abmessungen des Positionierungselements 200 so bestimmt, dass die Positionen der Bohrschablonen 220, 221 bzw. Schablonenkontaktflächen 222, 223 relativ zu den Kieferknochen 120, 121 den in Figur 1D durch die Scan-Daten der Abdrücke definierten Referenzpositionen entsprechen. Form und Abmessungen des Positionierungselements 200 sind beispielsweise so bestimmt, dass das Positionierungselement 200 zusammen mit den Bohrschablonen 220, 221 hinsichtlich der Abmessungen dem Bissregistrat 130 mit den Abdrücken 132, 133 entspricht. Das Positionieren und Fixieren der Bohrschablonen 220, 221 durch das Positionierungselement 200 erfolgt druckbasiert durch Druck, welchen der Patient mit seien Kiefern über die Bohrschablonen 220, 221 auf das Positionierungselement 200 ausübt.

Das Positionierungselement 200 umfasst beispielsweise Kontaktflächen 202, 212 um mit Oberflächen 230, 231 der Bohrschablonen 220, 221 in schlüssigen Kontakt zu treten. Beispielsweise umfassen das Positionierungselement 200 und die Bohrschablonen 220, 221 Verbindungselemente, um eine zerstörungsfrei lösbare Verbindung zwischen dem Positionierungselement 200 und der jeweiligen Bohrschablonen 220, 221 herzustellen. Ferner können entsprechende Verbindungelemente auch zur Ausrichtung des Positionierungselements 200 relativ zu den Bohrschablonen 220, 221 dienen. Beispielsweise umfasst das Positionierungselement 200 weibliche Verbindungelemente 204, 214, welche zur Aufnahme männlicher Verbindungelemente 228, 229 der Bohrschablonen 220, 221 vorgesehen sind. Ebenso könnten beispielsweise auch die Bohrschablonen 220, 221 zusätzlich oder alternativ weibliche Verbindungelemente umfassen, welche zur Aufnahme männlicher Verbindungelemente des Positionierungselements 200 vorgesehen sind.

Unter Verwendung des Positionierungselements 200 können die Bohrschablonen 220, 221 in den für sie vorgesehenen Positionen druckbasiert positioniert und fixiert werden zum Einbringen von Fixierungsbohrungen und Fixierungselemente. Mittels der Fixierungselemente können die Bohrschablonen 220, 221 an den vorgesehenen Positionen druckunabhängig fixiert werden. Somit kann der Patient beispielsweise den Mund öffnen und das Positionierungselement 200 kann aus dem Mundraum entnommen werden. Durch die Entnahme des Positionierungselements 200 wird beispielsweise der Zugang zu den Durchgangsöffnungen 224, 225 der Bohrschablonen 220, 221 zum Einbringen der Implantatbohrungen freigegeben.

Figur 2B zeigt zwei Bohrschablonen 220, 221, welche den Bohrschablonen 220, 221 aus Figur 2A entsprechen. In Figur 2B sind zwei Positionierungselemente 200, 201 gezeigt, welche über eine zerstörungsfrei lösbare Verbindung miteinander verbindbar sind. Die Kombination aus den beiden Positionierungselementen 200, 201 entspricht beispielsweise dem in Figur 2A gezeigten einzelnen Positionierungselement 200. Das in Figur 2B gezeigte Positionierungselement 200 umfasst neben einer Kontaktfläche 202 zum in Kontakttreten mit der Bohrschablone 220 beispielsweise ein weibliches Verbindungselement 204 zum Aufnehmen eines männlichen Verbindungselements 228 der Bohrschablone 220. Ferner umfasst das Positionierungselement 200 beispielsweise Verbindungselemente zum Herstellen der zerstörungsfrei lösbaren Verbindung mit dem Positionierungselement 201. Beispielsweise umfasst das Positionierungselement 200 weibliche Verbindungselemente 208 zur Aufnahme männlicher Verbindungselemente 207 des Positionierungselements 201 und/oder männliche Verbindungselemente 206, welche für eine Aufnahme in weiblichen Verbindungselementen 209 des Positionierungselements 201 konfiguriert sind. Das Positionierungselement 201 umfasst neben einer Kontaktfläche 203 zum in Kontakttreten mit der Bohrschablone 221 beispielsweise ein weibliches Verbindungselement 205 zum Aufnehmen eines männlichen Verbindungselements 229 der Bohrschablone 221. Ferner umfasst das Positionierungselement 201 beispielsweise Verbindungselemente zum Herstellen der zerstörungsfrei lösbaren Verbindung mit dem Positionierungselement 200. Beispielsweise umfasst das Positionierungselement 201 weibliche Verbindungselemente 209 zur Aufnahme männlicher Verbindungselemente 206 des Positionierungselements 200 und/oder männliche Verbindungselemente 207, welche für eine Aufnahme in weiblichen Verbindungselementen 208 des Positionierungselements 200 konfiguriert sind. Die Verbindungselemente können ferner auch zur Ausrichtung der beiden Positionierungselemente 200, 201 relativ zueinander dienen.

Unter Verwendung der Positionierungselemente 200, 201 können die Bohrschablonen 220, 221 in den für sie vorgesehenen Positionen druckbasiert positioniert und fixiert werden zum Einbringen von Fixierungsbohrungen und Fixierungselemente. Mittels der Fixierungselemente können die Bohrschablonen 220, 221 an den vorgesehenen Positionen druckunabhängig fixiert werden. Somit kann der Patient beispielsweise den Mund öffnen und die Positionierungselemente 200, 201 können aus dem Mundraum entnommen werden. Durch die Entnahme der Positionierungselemente 200, 201 wird beispielsweise der Zugang zu den Durchgangsöffnungen 224, 225 der Bohrschablonen 220, 221 zum Einbringen der Implantatbohrungen freigegeben.

Figur 2C zeigt eine exemplarische Kombination aus zwei Bohrschablonen 220, 221 und einem Positionierungselement 200. Das Positionierungselement 200 ist von den beiden Bohrschablonen 220, 221 umfasst. Die Kombination aus dem Positionierungselement 200 und den beiden Bohrschablonen 220, 221 ist beispielsweise einstückig ausgestaltet. Die beiden Bohrschablonen 220, 221 umfassen jeweils eine Schablonenkontaktfläche 222, 223. Ferner umfassen die Bohrschablonen 220, 221 beispielsweise jeweils Durchgangsöffnungen 224, 225 zum Einbringen von Implantatbohrungen durch die Gingivae 122, 123 hindurch in die Kieferknochen 120, 121. Ferner können die Bohrschablonen 220, 221 beispielsweise jeweils weitere Durchgangsöffnungen 226, 227 zum Einbringen von Fixierungsbohrungen durch die Gingivae 122, 123 hindurch in die Kieferknochen 120, 121 umfassen. In den Fixierungsbohrungen können Fixierungselemente zum Fixieren der Bohrschablonen 220, 221 in den durch das Positionierungselement 200 vorgegebenen Positionen relativ zu den Kieferknochen 120, 121 angeordnet werden. Das Positionierungselement 200 umfasst beispielsweise Zugangsöffnungen 210, 211, welche einen Zugang zu den Durchgangsöffnungen 224, 225 der Bohrschablonen 220, 221 freigeben, während das Positionierungselement 200 in dem Mundraum des Patienten angeordnet ist. Somit muss das Positionierungselement 200 zum Einbringen der Implantatbohrungen beispielsweise nicht aus dem Mundraum des Patienten entfernet werden. Während des Einbringens Implantatbohrungen können die Bohrschablonen 220, 221 unter Verwendung des Positionierungselements 200 druckabhängig in der vorgesehenen Position fixiert werden und/oder unter Verwendung von Fixierungselementen druckunabhängig in der vorgesehenen Position fixiert werden. Zur Stabilisierung des Positionierungselements 200, kann dieses zusätzliche Stützelemente umfassen, welche sich zwischen den Bohrschablonen 220, 221 erstrecken. Beispielsweise sind die Zugangsöffnungen 210, 211 zwischen den Stützelementen angeordnet.

Figur 2D zeigt zwei Bohrschablonen 220, 221, welche jeweils ein Positionierungselement 200, 201 umfassen. Beispielsweise sind die Bohrschablonen 220, 221 jeweils einstückig mit den entsprechenden Positionierungselement 200, 201 ausgestaltet. Die beiden Bohrschablonen 220, 221 umfassen jeweils eine Schablonenkontaktfläche 222, 223. Ferner umfassen die Bohrschablonen 220, 221 beispielsweise jeweils Durchgangsöffnungen 224, 225 zum Einbringen von Implantatbohrungen durch die Gingivae 122, 123 hindurch in die Kieferknochen 120, 121. Ferner können die Bohrschablonen 220, 221 beispielsweise jeweils weitere Durchgangsöffnungen 226, 227 zum Einbringen von Fixierungsbohrungen durch die Gingivae 122, 123 hindurch in die Kieferknochen 120, 121 umfassen. In den Fixierungsbohrungen können Fixierungselemente zum Fixieren der Bohrschablonen 220, 221 in den durch das Positionierungselement 200 vorgegebenen Positionen relativ zu den Kieferknochen 120, 121 angeordnet werden. Die Positionierungselemente 200, 201 umfassen beispielsweise jeweils eine Zugangsöffnung 210, 211, welche einen Zugang zu der Durchgangsöffnung 224, 225 der entsprechenden Bohrschablonen 220, 221 freigibt, während die Positionierungselemente 200, 201 in dem Mundraum des Patienten angeordnet sind. Somit müssen die Positionierungselemente 200, 201 zum Einbringen der Implantatbohrungen beispielsweise nicht aus dem Mundraum des Patienten entfernet werden.

Beispielsweise sind die Positionierungselemente 200, 201 über eine zerstörungsfrei lösbare Verbindung miteinander verbindbar. Das Positionierungselement 200 umfasst beispielsweise Verbindungselemente zum Herstellen der zerstörungsfrei lösbaren Verbindung mit dem Positionierungselement 201. Beispielsweise umfasst das Positionierungselement 200 weibliche Verbindungselemente 208 zur Aufnahme männlicher Verbindungselemente 207 des Positionierungselements 201 und/oder männliche Verbindungselemente 206, welche für eine Aufnahme in weiblichen Verbindungselementen 209 des Positionierungselements 201 konfiguriert sind. Das Positionierungselement 201 umfasst beispielsweise Verbindungselemente zum Herstellen der zerstörungsfrei lösbaren Verbindung mit dem Positionierungselement 200. Beispielsweise umfasst das Positionierungselement 201 weibliche Verbindungselemente 209 zur Aufnahme männlicher Verbindungselemente 206 des Positionierungselements 200 und/oder männliche Verbindungselemente 207, welche für eine Aufnahme in weiblichen Verbindungselementen 208 des Positionierungselements 200 konfiguriert sind. Die Verbindungselemente können ferner auch zur Ausrichtung der beiden Positionierungselemente 200, 201 relativ zueinander dienen.

Zum Einbringen der Implantatbohrungen wird beispielsweise die zerstörungsfrei lösbare Verbindung zwischen den beiden Positionierungselemente 200, 201 durch ein Öffnen der Kiefer des Patienten gelöst und die beiden Positionierungselemente 200, 201 werden voneinander beabstandet, sodass ein Zugang über die Zugangsöffnungen 210, 211 zu den Durchgangsöffnungen 224, 225 freigegebene wird. Somit können die Durchgangsöffnungen 224, 225 zum Einbringen der Implantatbohrungen genutzt werden. Während des Einbringens Implantatbohrungen können die Bohrschablonen 220, 221 unter Verwendung von Fixierungselementen druckunabhängig in der vorgesehenen Position fixiert werden.

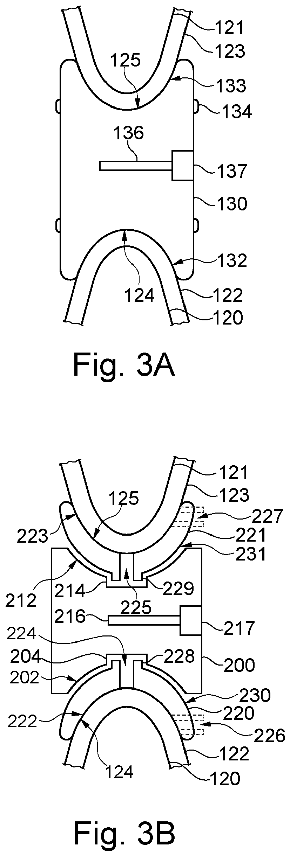

Figuren 3A und 3B zeigen Querschnitte eines exemplarischen Bissregistrats 130 und eines exemplarischen Positionierungselements 200 mit Druckerfassungselementen 136, 216. Figur 3A zeigt ein Bissregistrat 130, welches dem in Figur 1A gezeigten Bissregistrat 130 entspricht. Das Bissregistrat 130 in Figur 3A weist zusätzlich ein Druckerfassungselement 136 zum Erfassen eines während des Scannens der Kieferknochenstrukturdaten auf das Bissregistrat 130 mit den Kiefern aufgebrachten Drucks auf. Bei dem Druckerfassungselement 136 kann es sich beispielsweise um einen piezoelektrischen Sensor handeln. Ferner kann das Bissregistrat 130 ein Druckanzeigeelement 137 umfassen, welches den mit dem Druckerfassungselement 136 erfassten Druck anzeigt. Das Druckanzeigeelement 137 kann beispielsweise von dem Bissregistrat 130 umfasst sein oder als externes Druckanzeigeelement 137 bereitgestellt werden. Beispielsweise stellt das Druckanzeigeelement 137 eine visuelle Anzeige des mit dem Druckerfassungselement 136 erfassten Drucks bereit. Die visuelle Anzeige kann beispielsweise quantitativ in Form von Zahlen oder qualitativ in Form von Farben erfolgen. Beispielsweise kann das Druckanzeigeelement 137 auch durch das Druckerfassungselement 136 bereitgestellt werden, etwa im Fall eines Druckerfassungselement 136 in Form eines druckabhängiges Verformungselements, dessen Form druckabhängig ist, oder eines druckabhängiges Farbelement, dessen Farbe druckabhängig ist. Der während des Scannens der Kieferknochenstrukturdaten auf das Bissregistrat 130 mit den Kiefern aufgebrachte und erfasste Druck kann als Referenzdruck verwendet werden.

Figur 3B zeigt ein Positionierungselement 200, welches dem in Figur 2A gezeigten Positionierungselement 200 entspricht. Das Positionierungselement 200 in Figur 3B weist zusätzlich ein Druckerfassungselement 216 zum Erfassen eines auf das Positionierungselement 200 mit den Kiefern aufgebrachten Drucks auf. Bei dem Druckerfassungselement 216 kann es sich beispielsweise um einen piezoelektrischen Sensor handeln. Ferner kann das Positionierungselement 200 ein Druckanzeigeelement 217 umfassen, welches den mit dem Druckerfassungselement 216 erfassten Druck anzeigt. Das Druckanzeigeelement 217 kann beispielsweise von dem Positionierungselement 200 umfasst sein oder als externes Druckanzeigeelement 217 bereitgestellt werden. Beispielsweise stellt das Druckanzeigeelement 217 eine visuelle Anzeige des mit dem Druckerfassungselement 216 erfassten Drucks bereit. Die visuelle Anzeige kann beispielsweise quantitativ in Form von Zahlen oder qualitativ in Form von Farben erfolgen. Beispielsweise kann das Druckanzeigeelement 217 auch durch das Druckerfassungselement 216 bereitgestellt werden, etwa im Fall eines Druckerfassungselement 216 in Form eines druckabhängiges Verformungselements, dessen Form druckabhängig ist, oder eines druckabhängiges Farbelement, dessen Farbe druckabhängig ist. Mit dem Druckerfassungselement 216 kann sichergestellt werden, dass auf das Positionierungselement 200 ein Druck aufgebracht wird, welcher identisch mit dem Referenzdruck ist. Auch die in den Figuren 2B bis 2D gezeigten weiteren exemplarischen Positionierungselemente 200, 201 können beispielsweise ein Druckerfassungselement 216 und/oder ein Druckanzeigeelement 217 umfassen.

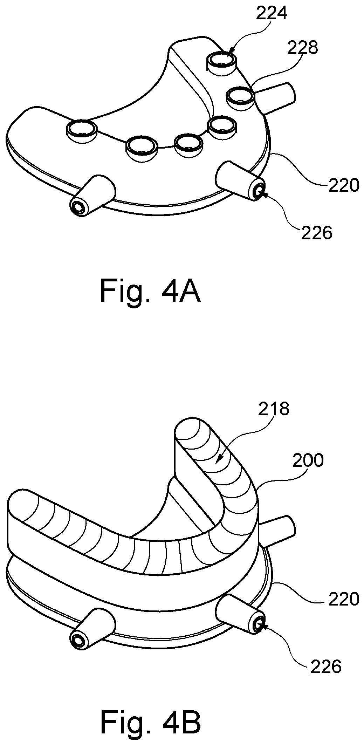

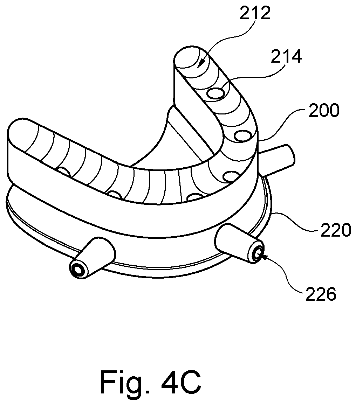

Figuren 4A bis 4C zeigen exemplarische Positionierungselemente 200 und Bohrschablonen 220. Figur 4A zeigt eine perspektivische Ansicht einer Bohrschablone 220, welche dazu konfiguriert ist auf einem Kiefern eines Patienten, beispielsweise einem zahnlosen Kiefer, angeordnet zu werden. Die Bohrschablone 220 umfasst Durchgangsöffnungen 224 zum Einbringen von Implantatbohrungen an zuvor festgelegten Implantatpositionen. Ferner umfasst die Bohrschablone 220 Verbindungelemente 228 zum Herstellen einer zerstörungsfrei lösbaren Verbindung mit dem Positionierungselement 200. Die Verbindungelemente 228 sind in Figur 4A exemplarisch als Erhöhungen um die Mündungen der Durchgangsöffnungen 224 gezeigt. Sie könnten aber auch unabhängig von den Durchgangsöffnungen 224 bereitgestellt werden. Die Verbindungelemente 228 sind beispielsweise dazu vorgesehen in Aufnahmen, wie etwa die in Figur 4C gezeigten Verbindungselemente 214, des Positionierungselements 200 aufgenommen zu werden. Die Erhöhungen 228 können alternativ oder zusätzlich Bohrführungselemente bereitstellen. Ferner können die Mündungen der Durchgangsöffnungen 224 in den Bohrführungselementen beispielsweise jeweils einen Anschlag zur Begrenzung der Bohrtiefe bereitstellen. Ferner umfasst die Bohrschablone 220 Durchgangsöffnungen 226 zum Einbringen von Fixierungsbohrungen an zuvor festgelegten Fixierungspositionen. Diese Fixierungsbohrungen sind zum Einbringen von Fixierungselementen konfiguriert, um die Bohrschablone 220 druckunabhängig an dem Kiefer in einer vorgesehenen Position fixieren zu können. Die Fortsätze an der Bohrschablone 220, durch welche sich die Durchgangsöffnungen 226 erstrecken, stellen beispielsweise Bohrführungselemente bereit. Ferner können die Mündungen der Durchgangsöffnungen 226 in den Bohrführungselementen beispielsweise jeweils einen Anschlag zur Begrenzung der Bohrtiefe bereitstellen.

Figur 4B zeigt die Bohrschablone 220 aus Figur 4A mit einem darauf angeordneten exemplarischen Positionierungselement 200. Das Positionierungselement 200 umfasst eine Kieferkontaktfläche 218 zum Herstellen eines Kontakts mit der Oberfläche eines Gegenkiefers. Zwischen dem Positionierungselement 200 und der Bohrschablone 220 wird beispielsweise eine zerstörungsfrei lösbare Verbindung hergestellt. Hierzu weist das Positionierungselement 200 auf einer der Bohrschablone 220 zugewandten Seite eine Kontaktfläche zum Herstellen eines Kontakts mit der Bohrschablone auf, welche der in der Figur 4C gezeigten Kontaktfläche 212 entspricht. Die entsprechende Kontaktfläche umfasst beispielsweise Verbindungselemente in Form von Aufnahmen zum Aufnehmen der Verbindungselemente 228 der Bohrschablone 220.

Figur 4C zeigt die Bohrschablone 220 aus Figur 4A mit einem darauf angeordneten exemplarischen Positionierungselement 200. Das Positionierungselement 200 umfasst eine Kontaktfläche 212 zum Herstellen eines Kontakts mit einer weiteren Bohrschablone, welche beispielsweise wie die in Figur 4A gezeigte Bohrschablone 220 ausgestaltet sein kann. Die Kontaktfläche 212 weist Verbindungselemente 214 zum Herstellen einer zerstörungsfrei lösbaren Verbindung mit der weiteren Bohrschablone. Hierzu sind die Verbindungselemente 214 beispielsweise als Aufnahmen zum Aufnehmen von Verbindungselementen der weiteren Bohrschablone ausgebildet, wie den in Figur 4A gezeigten Verbindungselemente 228 der Bohrschablone 220.

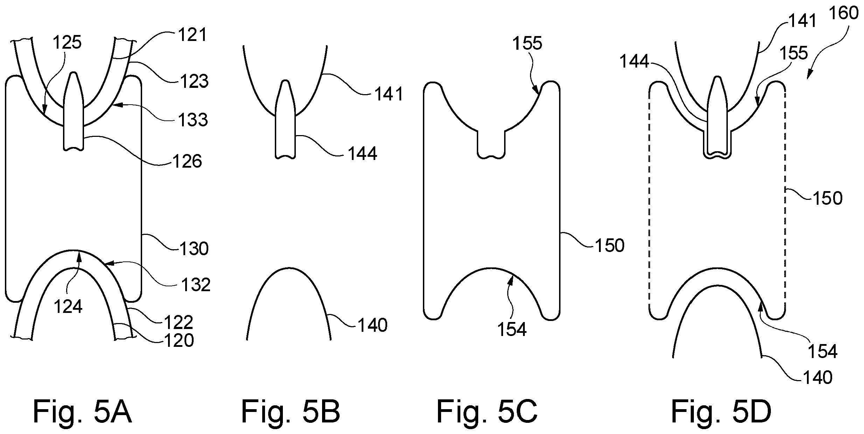

Figuren 5A bis 5D zeigen Querschnitte eines exemplarischen Bissregistrats 130 zwischen Kiefern sowie Daten der Kieferknochen 140, 141 und des Bissregistrats 150. In Figur 5A ist ein Querschnitt durch einen Kieferknochen 120 mit einer darauf angeordneten Gingiva 122 sowie einem Gegenkieferknochen 121 und einer drauf angeordneten Gingiva 123 gezeigt. Zwischen den beiden Kiefern ist ein Bissregistrat 130 angeordnet, welches dem in Figur 1A gezeigten Bissregistrat entspricht, aber keine Marker umfasst. Im Falle der Figur 5A weist der Gegenkiefer beispielsweise ein dentales Objekt 126 auf, wie etwa einen Zahn oder ein bereits bestehendes Implantat. Somit umfasst der Abdruck 133 neben einem Abdruck der Gingivaoberfläche 125 einen Abdruck des dentalen Objekts 126. Bei dem dentalen Objekt 126 handelt es sich beispielsweise um ein fixes Objekt, welches als Referenzstruktur verwendet werden kann. Das dentale Objekt 126 ist beispielsweise opak für das Scan-Verfahren zum Erfassen der Kieferknochenstrukturdaten. Zugleich ist der passende Abdruck des dentalen Objekts 126 mit dem zweiten Scan-Verfahren zum Erfassen der Formdaten des Abdrucks erfassbar. Dabei Bilden die Formdaten des Abdrucks das passende Negativ zu dem dentale Objekt 126. Indem die Formdaten des Abdrucks des dentalen Objekts 126 mit den Strukturdaten des dentalen Objekts 126 zu Deckung gebracht werden, kann die relative Position von Daten des Bissregisters 130 zu Daten des dentalen Objekts 126 bestimmt werden. Mithin können zusätzliche Marker überflüssig sein.

In Figur 5B sind Scan-Daten eines Scans der Strukturen der Kieferknochen 120 und Gegenkieferknochen 121 gezeigt. Die Scan-Daten umfassen neben Scan-Daten 140 des Kieferknochens 120 und Scan-Daten 141 des Gegenkieferknochens 121 Scan-Daten 144 des dentalen Objekts 126. In Figur 5C sind Scan-Daten eines zweiten Scan-Verfahrens zum Erfassen von Formdaten des Bissregistrats 130, insbesondere der Abdrücke 132, 133 gezeigt. Die Scan-Daten 150 des Bissregistrats umfassen beispielsweise zumindest Scan-Daten 154, 155 der Abdrücke 132, 133, wobei der Abdruck 133 einen Abdruck des dentalen Objekts 126 umfasst. Die Scan-Daten 150 können beispielsweise auch die gesamte Oberfläche des Bissregistrats 130 umfassen.

In Figur 5D ist ein Querschnitt durch ein digitales Modell 160 der Kieferknochen 120, 121 und des Bissregistrats 130 gezeigt. Das digitale Modell 160 umfasst die Scan-Daten 140 des Kieferknochens 120, die Scan-Daten 141 des Gegenkieferknochens 121, die Scan-Daten 144 des dentalen Objekts 126 sowie die Scan-Daten 154, 155 der Abdrücke 132, 133. Die relative Position des Bissregistrats 130 zu den Kiefernknochen 120, 121 wird dadurch bestimmt, dass die mit dem ersten Scan-Verfahren erfassten Scan-Daten 144 des dentalen Objekts 126 mit den mit dem zweiten Scan-Verfahren erfassten Scan-Daten des Abdrucks 155, welcher einen Abdruck des dentalen Objekts 126 umfasst zur Deckung gebracht werden. Die Scan-Daten 144 des dentalen Objekts 126 stellen zusammen mit den Scan-Daten 155 des Abdrucks des dentalen Objekts 126 also Positionsdaten des Bissregistrats 130 relativ zu den Kieferknochen 120, 121 dar. Durch die Scan-Daten 154, 155 des Kieferknochens 140 und Gegenkieferknochens 141 sowie den Scan-Daten 154, 155 der Abdrücke 132, 133 werden ferner beispielsweise die Konturen der Gingivae 122, 123 indirekt erfasst, obwohl die Gingivae 122, 123 durch die Scan-Verfahren beispielsweise nicht direkt erfasst werden. Die Scan-Daten 154 des Abdrucks 132 dient beispielsweise als Vorlagen für eine geometrische Form einer Schablonenkontaktfläche einer zu erstellenden Bohrschablone. Dabei definiert die Position der Scan-Daten 154, 155 der Abdrücke 132, 133 relative zu den die Scan-Daten 154, 155 der Kieferknochen 140, 141 beispielsweise eine Referenzposition für die Schablonenkontaktfläche der Bohrschablone relativ zu den Kieferknochen 140, 141.

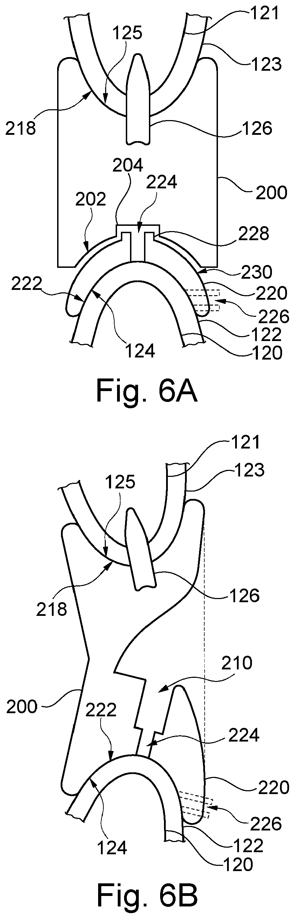

Figuren 6A und 6B zeigen Querschnitte exemplarischer Positionierungselemente 200 und Bohrschablonen 220. Figur 6A zeigt eine Bohrschablone 220, welche auf der Gingiva 122 des Kiefers bzw. Kieferknochens 120 angeordnet ist. Die Bohrschablone 220 umfasst eine Schablonenkontaktfläche 222, deren geometrische Form durch den mit dem Bissregistrat 130 erfassten Abdruck 132 der Gingivaoberflächen 124 vorgegeben ist. Die Bohrschablone 220 umfasst beispielsweise Durchgangsöffnungen 224 zum Einbringen von Implantatbohrungen durch die Gingiva 122 hindurch in den Kieferknochen 120. Ferner umfassen die Bohrschablonen 220 beispielsweise jeweils weitere Durchgangsöffnungen 226 zum Einbringen von Fixierungsbohrungen durch die Gingiva 122 hindurch in den Kieferknochen 120. In den Fixierungsbohrungen können Fixierungselemente zum Fixieren der Bohrschablonen 220 in der durch das Positionierungselement 200 vorgegebenen Position relativ zu dem Kieferknochen 120 angeordnet werden. Die Bohrschablone 220 wird unter Verwendung des zwischen der Bohrschablone 220 und der Gingiva 123 des Gegenkieferknochens 121 angeordneten Positionierungselements 200 in einer Position relativ zu dem Kieferknochen 120 positioniert und fixiert. Dabei sind Form und Abmessungen des Positionierungselements 200 so bestimmt, dass die Position der Bohrschablonen 220 bzw. Schablonenkontaktfläche 222 relativ zu dem Kieferknochen 120 den in Figur 5D durch die Scan-Daten 154 des Abdrucks 132 definierte Referenzposition entspricht. Form und Abmessungen des Positionierungselements 200 sind beispielsweise so bestimmt, dass das Positionierungselement 200 zusammen mit der Bohrschablone 220 hinsichtlich der Abmessungen dem Bissregistrat 130 mit den Abdrücken 132, 133 entspricht. Das Positionieren und Fixieren der Bohrschablone 220 durch das Positionierungselement 200 erfolgt druckbasiert durch Druck, welchen der Patient mit seien Kiefern auf die Bohrschablone 220 und das Positionierungselement 200 ausübt.

Das Positionierungselement 200 umfasst beispielsweise eine Kontaktfläche 202 um mit der Oberflächen 230 der Bohrschablone 220 in schlüssigen Kontakt zu treten. Beispielsweise umfassen das Positionierungselement 200 und die Bohrschablone 220 Verbindungselemente, um eine zerstörungsfrei lösbare Verbindung zwischen dem Positionierungselement 200 und der Bohrschablonen 220 herzustellen. Ferner können entsprechende Verbindungelemente auch zur Ausrichtung des Positionierungselements 200 relativ zu der Bohrschablonen 220 dienen. Beispielsweise umfasst das Positionierungselement 200 weibliche Verbindungelemente 204, welche zur Aufnahme männlicher Verbindungelemente 228 der Bohrschablone 220 vorgesehen sind. Ebenso könnte beispielsweise auch die Bohrschablone 220 zusätzlich oder alternativ weibliche Verbindungelemente umfassen, welche zur Aufnahme männlicher Verbindungelemente des Positionierungselements 200 vorgesehen sind.

Beispielsweise umfasst das Positionierungselement 200 ferner eine Kieferkontaktfläche 218 um mit der Oberflächen 125 der Gingiva 123 sowie dem dentalen Objekt 126 des Gegenkiefers in Kontakt zu treten. Durch den Kontakt mit dem dentalen Objekt 126 kann das Positionierungselement 200 relativ zum Gegenkiefernknochen 121 ausgerichtet werden. Die geometrische Form der Kieferkontaktfläche 218 wird beispielsweise durch die Scan-Daten 155 des Abdrucks 133 mit dem Abdruck des dentalen Objekts 126 festgelegt.

Unter Verwendung des Positionierungselements 200 kann die Bohrschablone 220 in der vorgesehenen Position druckbasiert positioniert und fixiert werden zum Einbringen von Fixierungsbohrungen und Fixierungselemente. Mittels der Fixierungselemente kann die Bohrschablone 220 an der vorgesehen Position druckunabhängig fixiert werden. Somit kann der Patient beispielsweise den Mund öffnen und das Positionierungselement 200 kann aus dem Mundraum entnommen werden. Durch die Entnahme des Positionierungselements 200 wird beispielsweise der Zugang zu den Durchgangsöffnungen 224 der Bohrschablone 220 zum Einbringen der Implantatbohrungen freigegeben.

Figur 6B zeigt eine exemplarische Kombination aus einer Bohrschablone 220 und einem Positionierungselement 200. Das Positionierungselement 200 ist von der Bohrschablone 220 umfasst. Die Kombination aus dem Positionierungselement 200 und der Bohrschablone 220 ist beispielsweise einstückig ausgestaltet. Die Bohrschablone 220 umfasst eine Schablonenkontaktfläche 222. Ferner umfasset die Bohrschablone 220 beispielsweise Durchgangsöffnungen 224 zum Einbringen von Implantatbohrungen durch die Gingiva 122 hindurch in den Kieferknochen 120. Ferner kann die Bohrschablone 220 beispielsweise weitere Durchgangsöffnungen 226 zum Einbringen von Fixierungsbohrungen durch die Gingiva 122 hindurch in den Kieferknochen 120 umfassen. In den Fixierungsbohrungen können Fixierungselemente zum Fixieren der Bohrschablone 220 in der durch das Positionierungselement 200 vorgegebenen Position relativ zu dem Kieferknochen 120 angeordnet werden. Das Positionierungselement 200 umfasst beispielsweise Zugangsöffnungen 210, welche einen Zugang zu den Durchgangsöffnungen 224 der Bohrschablone 220 freigeben, während das Positionierungselement 200 in dem Mundraum des Patienten angeordnet ist. Somit muss das Positionierungselement 200 zum Einbringen der Implantatbohrungen beispielsweise nicht aus dem Mundraum des Patienten entfernet werden. Während des Einbringens Implantatbohrungen kann die Bohrschablone 220 unter Verwendung des Positionierungselements 200 druckabhängig in der vorgesehenen Position fixiert werden und/oder unter Verwendung von Fixierungselementen druckunabhängig in der vorgesehenen Position fixiert werden. Zur Stabilisierung des Positionierungselements 200, kann dieses zusätzliche Stützelemente umfassen. Beispielsweise sind die Zugangsöffnungen 210 zwischen den Stützelementen angeordnet.

Beispielsweise umfasst das Positionierungselement 200 ferner eine Kieferkontaktfläche 218 um mit der Oberflächen 125 der Gingiva 123 sowie dem dentalen Objekt 126 des Gegenkiefers in Kontakt zu treten. Durch den Kontakt mit dem dentalen Objekt 126 kann das Positionierungselement 200 relativ zum Gegenkiefernknochen 121 ausgerichtet werden. Die geometrische Form der Kieferkontaktfläche 218 wird beispielsweise durch die Scan-Daten 155 des Abdrucks 133 mit dem Abdruck des dentalen Objekts 126 festgelegt.

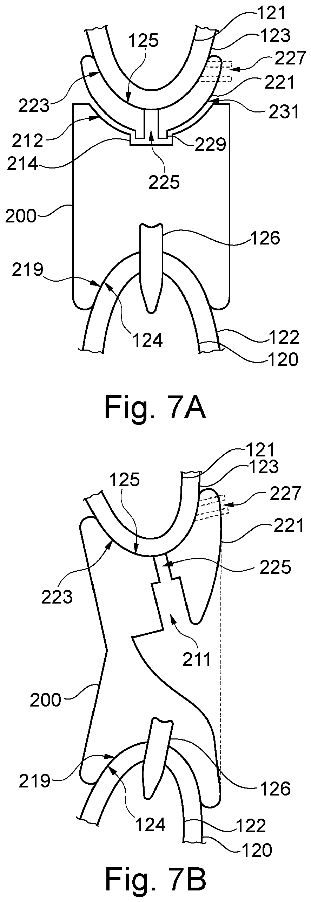

Figuren 7A und 7B zeigen Querschnitte exemplarischer Positionierungselemente 200 und Bohrschablonen 221. Figur 7A zeigt eine Situation, welche der Figur 6A entspricht. Allerdings weist der Kiefer anstelle des Gegenkiefers das dentale Objekt 126 auf und die Bohrschablone 221 ist an der Gingiva 123 des anderen Kiefers bzw. Kieferknochens 121 angeordnet. Die Bohrschablone 221 umfasst eine Schablonenkontaktfläche 223, deren geometrische Form durch den mit dem Bissregistrat 130 erfassten Abdruck 133 der Gingivaoberflächen 125 vorgegeben ist. Die Bohrschablone 221 umfasst beispielsweise Durchgangsöffnungen 225 zum Einbringen von Implantatbohrungen durch die Gingiva 123 hindurch in den Kieferknochen 121. Ferner umfassen die Bohrschablonen 221 beispielsweise jeweils weitere Durchgangsöffnungen 227 zum Einbringen von Fixierungsbohrungen durch die Gingiva 123 hindurch in den Kieferknochen 121. In den Fixierungsbohrungen können Fixierungselemente zum Fixieren der Bohrschablonen 221 in der durch das Positionierungselement 200 vorgegebenen Position relativ zu dem Kieferknochen 121 angeordnet werden. Die Bohrschablone 221 wird unter Verwendung des zwischen der Bohrschablone 221 und der Gingiva 122 des Kieferknochens 120 angeordneten Positionierungselements 200 in einer Position relativ zu dem Kieferknochen 121 positioniert und fixiert. Das Positionieren und Fixieren der Bohrschablone 221 durch das Positionierungselement 200 erfolgt druckbasiert durch Druck, welchen der Patient mit seien Kiefern auf die Bohrschablone 221 und das Positionierungselement 200 ausübt.

Das Positionierungselement 201 umfasst beispielsweise eine Kontaktfläche 212 um mit der Oberflächen 231 der Bohrschablone 221 in schlüssigen Kontakt zu treten. Beispielsweise umfassen das Positionierungselement 200 und die Bohrschablone 221 Verbindungselemente, um eine zerstörungsfrei lösbare Verbindung zwischen dem Positionierungselement 200 und der Bohrschablonen 221 herzustellen. Ferner können entsprechende Verbindungselemente auch zur Ausrichtung des Positionierungselements 200 relativ zu der Bohrschablonen 221 dienen. Beispielsweise umfasst das Positionierungselement 200 weibliche Verbindungelemente 214, welche zur Aufnahme männlicher Verbindungelemente 229 der Bohrschablone 221 vorgesehen sind. Ebenso könnte beispielsweise auch die Bohrschablone 221 zusätzlich oder alternativ weibliche Verbindungelemente umfassen, welche zur Aufnahme männlicher Verbindungelemente des Positionierungselements 200 vorgesehen sind.

Beispielsweise umfasst das Positionierungselement 200 ferner eine Kieferkontaktfläche 219 um mit der Oberflächen 124 der Gingiva 122 sowie dem dentalen Objekt 126 in Kontakt zu treten. Durch den Kontakt mit dem dentalen Objekt 126 kann das Positionierungselement 200 relativ zum Kiefernknochen 120 ausgerichtet werden. Die geometrische Form der Kieferkontaktfläche 219 wird beispielsweise durch Scan-Daten eines Abdrucks des Bissregistrats mit dem Abdruck des dentalen Objekts 126 festgelegt.

Unter Verwendung des Positionierungselements 200 kann die Bohrschablone 221 in der vorgesehenen Position druckbasiert positioniert und fixiert werden zum Einbringen von Fixierungsbohrungen und Fixierungselemente. Mittels der Fixierungselemente kann die Bohrschablone 221 an der vorgesehen Position druckunabhängig fixiert werden. Somit kann der Patient beispielsweise den Mund öffnen und das Positionierungselement 200 kann aus dem Mundraum entnommen werden. Durch die Entnahme des Positionierungselements 200 wird beispielsweise der Zugang zu den Durchgangsöffnungen 225 der Bohrschablone 221 zum Einbringen der Implantatbohrungen freigegeben.

Figur 7B zeigt eine Situation, welche der Figur 6B entspricht. Allerdings weist der Kiefer anstelle des Gegenkiefers das dentale Objekt 126 auf und die Bohrschablone 221 ist an der Gingiva 123 des anderen Kiefers bzw. Kieferknochens 121 angeordnet. Figur 7B zeigt eine exemplarische Kombination aus einer Bohrschablone 221 und einem Positionierungselement 200. Das Positionierungselement 200 ist von der Bohrschablone 221 umfasst. Die Kombination aus dem Positionierungselement 200 und der Bohrschablone 221 ist beispielsweise einstückig ausgestaltet. Die Bohrschablone 221 umfasst eine Schablonenkontaktfläche 223. Ferner umfasst die Bohrschablone 221 beispielsweise Durchgangsöffnungen 225 zum Einbringen von Implantatbohrungen durch die Gingiva 123 hindurch in den Kieferknochen 121. Ferner kann die Bohrschablone 221 beispielsweise weitere Durchgangsöffnungen 227 zum Einbringen von Fixierungsbohrungen durch die Gingiva 123 hindurch in den Kieferknochen 121 umfassen. In den Fixierungsbohrungen können Fixierungselemente zum Fixieren der Bohrschablone 221 in der durch das Positionierungselement 200 vorgegebenen Position relativ zu dem Kieferknochen 121 angeordnet werden. Das Positionierungselement 200 umfasst beispielsweise Zugangsöffnungen 211, welche einen Zugang zu den Durchgangsöffnungen 225 der Bohrschablone 221 freigeben, während das Positionierungselement 200 in dem Mundraum des Patienten angeordnet ist. Somit muss das Positionierungselement 200 zum Einbringen der Implantatbohrungen beispielsweise nicht aus dem Mundraum des Patienten entfernet werden. Während des Einbringens Implantatbohrungen kann die Bohrschablone 221 unter Verwendung des Positionierungselements 200 druckabhängig in der vorgesehenen Position fixiert werden und/oder unter Verwendung von Fixierungselementen druckunabhängig in der vorgesehenen Position fixiert werden. Zur Stabilisierung des Positionierungselements 200, kann dieses zusätzliche Stützelemente umfassen. Beispielsweise sind die Zugangsöffnungen 211 zwischen den Stützelementen angeordnet.

Beispielsweise umfasst das Positionierungselement 200 ferner eine Kieferkontaktfläche 219 um mit der Oberflächen 124 der Gingiva 122 sowie dem dentalen Objekt 126 in Kontakt zu treten. Durch den Kontakt mit dem dentalen Objekt 126 kann das Positionierungselement 200 relativ zum Gegenkiefernknochen 120 ausgerichtet werden. Die geometrische Form der Kieferkontaktfläche 219 wird beispielsweise durch Scan-Daten eines Abdrucks eines Bissregistrats mit dem Abdruck des dentalen Objekts 126 festgelegt.

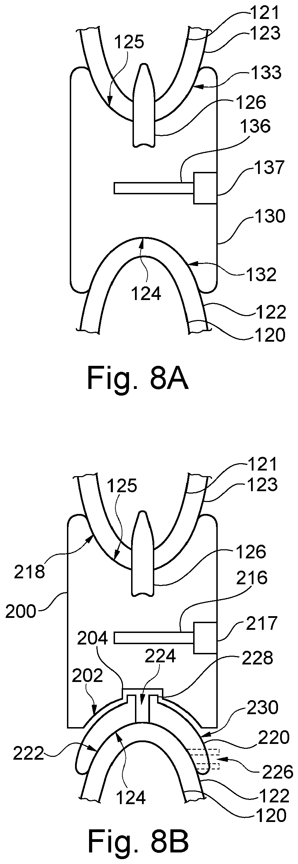

Figuren 8A und 8B zeigen Querschnitte eines exemplarischen Bissregistrats 130 und eines exemplarischen Positionierungselements 200 mit Druckerfassungselementen 136, 216. Figur 8A zeigt ein Bissregistrat 130, welches dem in Figur 5A gezeigten Bissregistrat 130 entspricht. Das Bissregistrat 130 in Figur 8A weist zusätzlich ein Druckerfassungselement 136 zum Erfassen eines während des Scannens der Kieferknochenstrukturdaten auf das Bissregistrat 130 mit den Kiefern aufgebrachten Drucks auf. Bei dem Druckerfassungselement 136 kann es sich beispielsweise um einen piezoelektrischen Sensor handeln. Ferner kann das Bissregistrat 130 ein Druckanzeigeelement 137 umfassen, welches den mit dem Druckerfassungselement 136 erfassten Druck anzeigt. Das Druckanzeigeelement 137 kann beispielsweise von dem Bissregistrat 130 umfasst sein oder als externes Druckanzeigeelement 137 bereitgestellt werden. Beispielsweise stellt das Druckanzeigeelement 137 eine visuelle Anzeige des mit dem Druckerfassungselement 136 erfassten Drucks bereit. Die visuelle Anzeige kann beispielsweise quantitativ in Form von Zahlen oder qualitativ in Form von Farben erfolgen. Beispielsweise kann das Druckanzeigeelement 137 auch durch das Druckerfassungselement 136 bereitgestellt werden, etwa im Fall eines Druckerfassungselement 136 in Form eines druckabhängiges Verformungselements, dessen Form druckabhängig ist, oder eines druckabhängiges Farbelement, dessen Farbe druckabhängig ist. Der während des Scannens der Kieferknochenstrukturdaten auf das Bissregistrat 130 mit den Kiefern aufgebrachte und erfasste Druck kann als Referenzdruck verwendet werden.

Figur 8B zeigt ein Positionierungselement 200, welches dem in Figur 6A gezeigten Positionierungselement 200 entspricht. Das Positionierungselement 200 in Figur 8B weist zusätzlich ein Druckerfassungselement 216 zum Erfassen eines auf das Positionierungselement 200 mit den Kiefern aufgebrachten Drucks auf. Bei dem Druckerfassungselement 216 kann es sich beispielsweise um einen piezoelektrischen Sensor handeln. Ferner kann das Positionierungselement 200 ein Druckanzeigeelement 217 umfassen, welches den mit dem Druckerfassungselement 216 erfassten Druck anzeigt. Das Druckanzeigeelement 217 kann beispielsweise von dem Positionierungselement 200 umfasst sein oder als externes Druckanzeigeelement 217 bereitgestellt werden. Beispielsweise stellt das Druckanzeigeelement 217 eine visuelle Anzeige des mit dem Druckerfassungselement 216 erfassten Drucks bereit. Die visuelle Anzeige kann beispielsweise quantitativ in Form von Zahlen oder qualitativ in Form von Farben erfolgen. Beispielsweise kann das Druckanzeigeelement 217 auch durch das Druckerfassungselement 216 bereitgestellt werden, etwa im Fall eines Druckerfassungselement 216 in Form eines druckabhängiges Verformungselements, dessen Form druckabhängig ist, oder eines druckabhängiges Farbelement, dessen Farbe druckabhängig ist. Mit dem Druckerfassungselement 216 kann sichergestellt werden, dass auf das Positionierungselement 200 ein Druck aufgebracht wird, welcher identisch mit dem Referenzdruck ist. Auch die in den Figuren 6B sowie 7A und 7B gezeigten weiteren exemplarischen Positionierungselemente 200 können beispielsweise ein Druckerfassungselement 216 und/oder ein Druckanzeigeelement 217 umfassen.

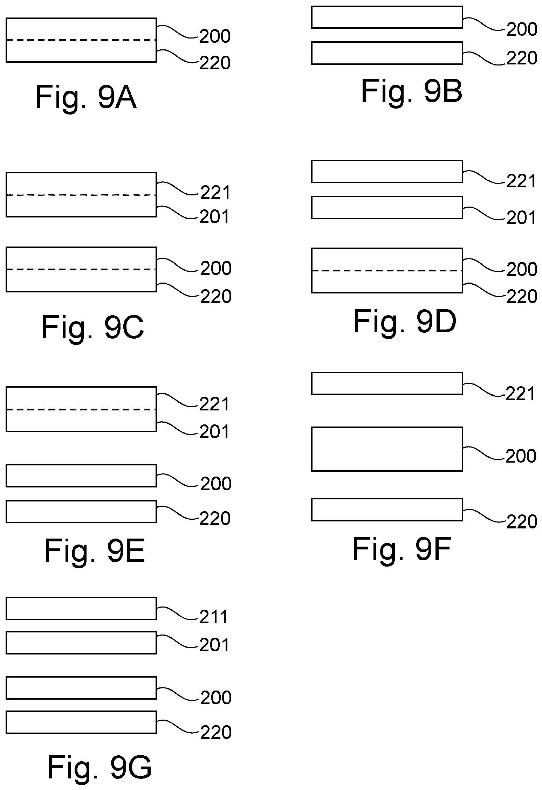

Figuren 9A bis 9F zeigen exemplarische Kombinationen schematischer Positionierungselemente und Bohrschablonen. Figur 9A zeigt eine Bohrschablone 220, welche ein Positionierungselement 200 umfasst. Die Bohrschablone220 ist beispielsweise dazu konfiguriert, unter Verwendung des Positionierungselements 200 auf einem Kiefer positioniert und fixiert zu werden. Das Positionierungselements 200 weist beispielsweise eine Kieferkontaktfläche zum Herstellen eines Kontakts mit der Oberfläche des Gegenkiefers auf.

Figur 9B zeigt eine Bohrschablone 220 und ein Positionierungselement 200, welche mittels einer zerstörungsfrei lösbaren Verbindung miteinander verbindbar sind. Die Bohrschablone 220 ist beispielsweise dazu konfiguriert, unter Verwendung des Positionierungselements 200 auf einem Kiefer positioniert und fixiert zu werden. Das Positionierungselements 200 weist beispielsweise eine Kieferkontaktfläche zum Herstellen eines Kontakts mit der Oberfläche des Gegenkiefers auf.

Figur 9C zeigt eine erste Bohrschablone 220, welche beispielsweise dazu konfiguriert ist, unter Verwendung eines von der ersten Bohrschablone 220 umfassten ersten Positionierungselements 200 auf einem Kiefer positioniert und fixiert zu werden. Ferner wird eine zweite Bohrschablone 221 gezeigt, welche beispielsweise dazu konfiguriert ist, unter Verwendung eines von der ersten Bohrschablone 221 umfassten zweiten Positionierungselements 201 auf einem Gegenkiefer positioniert und fixiert zu werden. Die beiden Positionierungselemente 200 und 201 sind beispielsweise mittels einer zerstörungsfrei lösbaren Verbindung miteinander verbindbar.

Figur 9D zeigt eine erste Bohrschablone 220, welche beispielsweise dazu konfiguriert ist, unter Verwendung eines von der ersten Bohrschablone 220 umfassten ersten Positionierungselements 200 auf einem Kiefer positioniert und fixiert zu werden. Ferner sind eine zweite Bohrschablone 221 und ein zweites Positionierungselement 201 gezeigt, welche mittels einer zerstörungsfrei lösbaren Verbindung miteinander verbindbar sind. Die zweite Bohrschablone 221 ist beispielsweise dazu konfiguriert, unter Verwendung des zweiten Positionierungselements 201 auf einem Gegenkiefer positioniert und fixiert zu werden.

Figur 9E zeigt eine erste Bohrschablone 220 und ein erstes Positionierungselement 200, welche mittels einer zerstörungsfrei lösbaren Verbindung miteinander verbindbar sind. Die erste Bohrschablone 220 ist beispielsweise dazu konfiguriert, unter Verwendung des ersten Positionierungselements 200 auf einem Kiefer positioniert und fixiert zu werden. Ferner wird eine zweite Bohrschablone 221 gezeigt, welche beispielsweise dazu konfiguriert ist, unter Verwendung eines von der ersten Bohrschablone 221 umfassten zweiten Positionierungselements 201 auf einem Gegenkiefer positioniert und fixiert zu werden. Die beiden Positionierungselemente 200 und 201 sind beispielsweise mittels einer zerstörungsfrei lösbaren Verbindung miteinander verbindbar.

Figur 9F zeigt eine erste Bohrschablone 220 und ein erstes Positionierungselement 200, welche mittels einer zerstörungsfrei lösbaren Verbindung miteinander verbindbar sind. Die erste Bohrschablone 220 ist beispielsweise dazu konfiguriert, unter Verwendung des ersten Positionierungselements 200 auf einem Kiefer positioniert und fixiert zu werden. Ferner sind eine zweite Bohrschablone 221 und ein zweites Positionierungselement 201 gezeigt, welche mittels einer zerstörungsfrei lösbaren Verbindung miteinander verbindbar sind. Die zweite Bohrschablone 221 ist beispielsweise dazu konfiguriert, unter Verwendung des zweiten Positionierungselements 201 auf einem Gegenkiefer positioniert und fixiert zu werden.

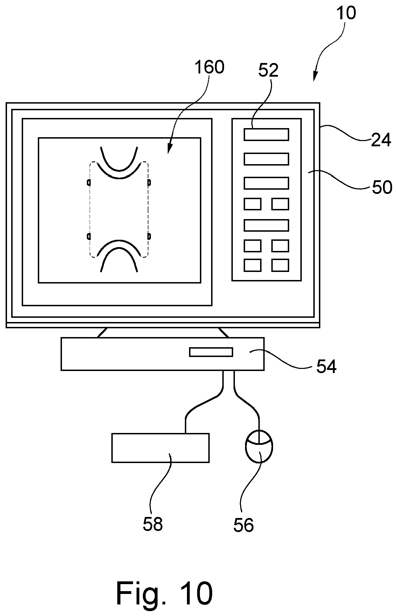

Figur 10 zeigt ein exemplarisches Computersystem 10 zum Erstellen digitaler Modelle von Positionierungselementen und Bohrschablonen. Zum Erstellen der digitalen Modelle von Positionierungselementen und Bohrschablonen wird beispielsweise ein digitales Modell 160 von Kieferknochen und Gingivaoberflächen verwendet. Das digitale Modell 160 basiert beispielsweise auf Scans von Kieferknochenstrukturdaten, Formdaten eines Bissregistrats sowie Positionsdaten der relativen Position von Bissregistrat und Kiefern beruht. Das Computersystem 10 kann beispielsweise ein eine Hardware-Komponente 54 mit einem oder mehreren Prozessoren sowie einem Speicher zur Speicherung von maschinenausführbaren Programmbefehlen enthalten. Ein Ausführen der Programmbefehle durch die Prozessoren kann den einen oder die mehreren Prozessoren veranlassen, das Computersystem 10 zu steuern, um das digitale Modell 160 unter Verwendung von Scan-Daten zu konstruieren. Das Computersystem 10 kann ferner externe Eingabegeräte, wie etwa eine Tastatur 58 und eine Maus 56 umfassen, die es dem Benutzer ermöglichen, mit dem Computersystem 10 zu interagieren. Darüber hinaus kann das Computersystem 10 Ausgabegeräte, wie etwa einen Bildschirm 24 mit einer Nutzerschnittstelle 50 mit Steuerelementen 52 umfassen, die es dem Benutzer ermöglichen, die Konstruktion des digitalen Modells 160 mit Hilfe des Computersystems 10 zu steuern. Das erzeugte digitalen Modells 160 kann auf der Nutzerschnittstelle 50 dargestellt werden.

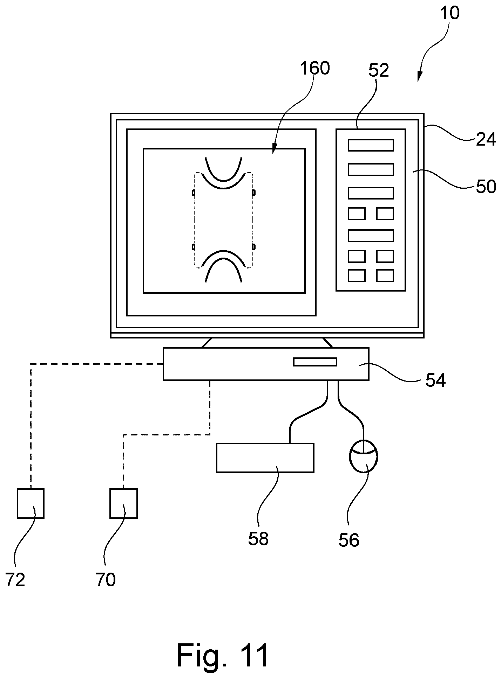

Figur 11 zeigt ein exemplarisches Computersystem 10 zum Erstellen digitaler Modelle von Positionierungselementen und Bohrschablonen. Das Computersystem 10 der Figur 11 entspricht dem in Figur 10 gezeigten Computersystem 10. Gegenüber dem in Figur 10 gezeigten Computersystem 10 umfasst das Computersystem 10 der Figur 11 eine erste und/oder eine zweite Scan-Vorrichtung 70, 72. Die erste Scan-Vorrichtung 70 ist beispielsweise zum Erfassen von Strukturdaten von Kieferknochen zum Erstellen des digitale Modells 160 konfiguriert. Beispielsweise umfasst die erste Scan-Vorrichtung 70 ein Computertomographie-Gerät, ein Volumentomographie-Gerät oder ein Magnetresonanztomographie-Gerät. Die zweite Scan-Vorrichtung 72 ist beispielsweise zum Erfassen von Formdaten eines Bissregistrats zum Erstellen des digitale Modells 160 konfiguriert. Beispielsweise umfasst die zweite Scan-Vorrichtung 72 einen optischen Scanner, ein Computertomographie-Gerät oder einen haptischen bzw. taktilen Scanner.

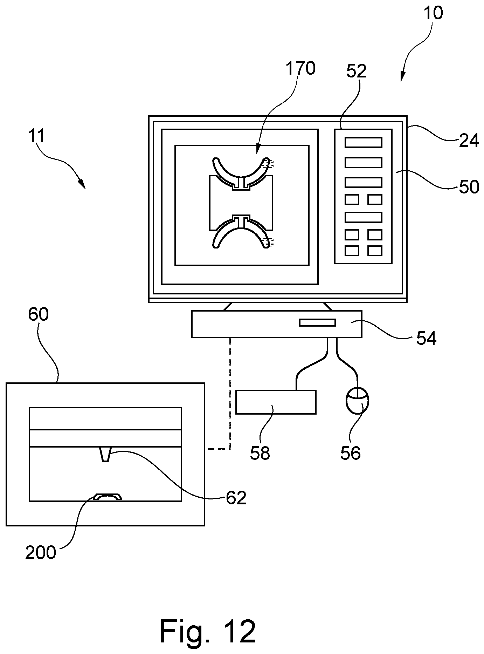

Figur 12 zeigt eine exemplarische Herstellungsvorrichtung 11 zum Herstellen von Positionierungselementen und Bohrschablonen. Die Herstellungsvorrichtung 11 kann beispielsweise das Computersystem 10 aus Figur 10 umfassen. Das Computersystem 10 kann ferner so konfiguriert werden, dass es einen 3D-Drucker 60 zum Herstellen von Positionierungselementen und Bohrschablonen gemäß einem auf dem Computersystem 10 konstruierten digitalen Modell 170 steuert. Beispielsweise kann Positionierungselement 200 gedruckt werden. Der 3D-Drucker 60 kann ein Druckelement 62 umfassen, das so konfiguriert ist, dass es Positionierungselemente und Bohrschablonen, etwa ein Positionierungselement 200, Schicht für Schicht druckt. Je nach Ausführung kann das Computersystem 10 dazu konfiguriert sein, zusätzlich ein oder mehrere weitere 3D-Drucker 60 zu verwenden.

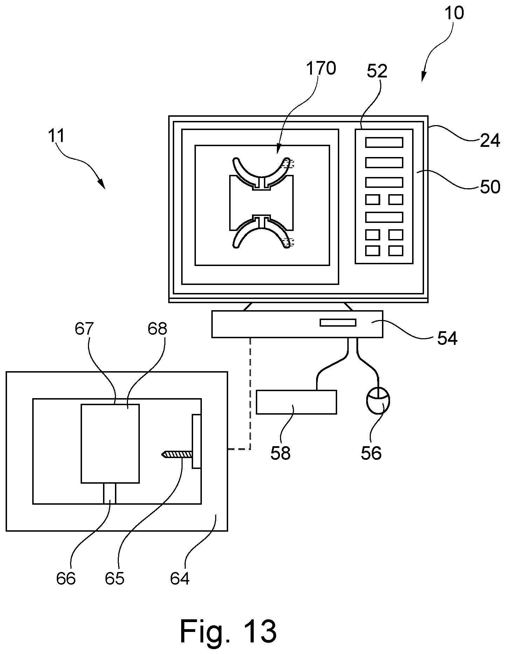

Figur 13 zeigt eine exemplarische Herstellungsvorrichtung 11 zum Herstellen von Positionierungselementen und Bohrschablonen. Die Herstellungsvorrichtung 11 kann beispielsweise das Computersystem 10 aus Figur 10 umfassen. Das Computersystem 10 kann ferner so konfiguriert werden, dass es ein Bearbeitungsgerät 64 steuert, das für die Bearbeitung eines Rohlings 67 unter Verwendung eines oder mehrerer Bearbeitungswerkzeuge 65 konfiguriert ist. Der Rohling 67 aus Material 68 kann unter Verwendung einer Haltevorrichtung 66 bereitgestellt und in eine gewünschte Endform und -größe des herzustellenden Elements geschnitten oder gefräst werden. Dabei werden beispielsweise das eine oder die mehreren Bearbeitungswerkzeuge 72 zum Ausführen eines von dem Computersystem 10 gesteuerten Materialabtragungsprozesses verwendet. Beispielsweise können Positionierungselemente und/oder Bohrschablohnen gemäß einem von dem Computer 10 bereitgestellten digitalen Modell aus dem Rohling hergestellt werden. Das Bearbeitungswerkzeug 65 kann zum Beispiel ein Fräswerkzeug sein. Je nach Ausführung kann das Computersystem 10 dazu konfiguriert sein, zusätzlich ein oder mehrere weitere Bearbeitungsgeräte 64 und/oder einen oder mehrere 3D-Drucker 60 zu verwenden.

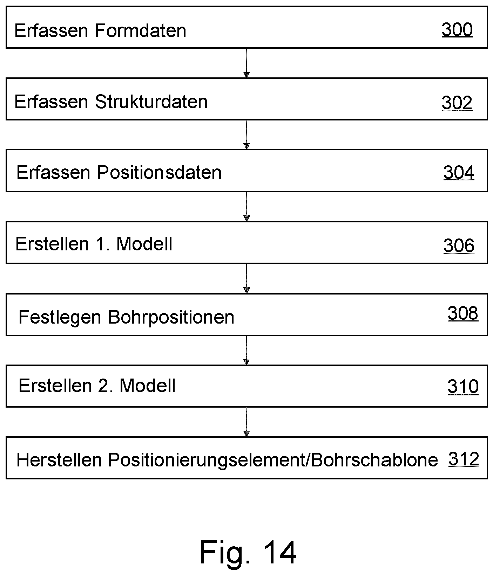

Figur 14 zeigt ein Flussdiagramm eines exemplarischen Verfahrens zum Herstellen eines Positionierungselements und/oder einer Bohrschablone. Das Positionierungselements ist zum Fixieren einer Bohrschablone auf einer Gingiva eines Kiefers eines Patienten konfiguriert. Dabei fixiert das Positionierungselement die Bohrschablone mittels Drucks, welchen der Patient durch ein Schließen des Kiefers und eines Gegenkiefers auf das Positionierungselement aufbringt, in einer Position relativ zu einem Kieferknochen des Kiefers zum Einbringen von Implantatbohrungen in den Kiefer für dentale Implantate. Die Bohrschablone umfasst beispielsweise eine Schablonenkontaktfläche zum Herstellen eines Kontakts mit einer Oberfläche der Gingiva des Kiefers sowie Durchgangsöffnungen, welche jeweils eine Implantatposition zum Einbringen einer der Implantatbohrungen definieren.

In Block 300 werden Formdaten eines Abdrucks der Oberfläche der Gingiva des Kiefers unter Verwendung eines zwischen den beiden Kiefern des Patienten angeordneten Bissregistrats erfasst. Dabei befinden sich die beiden Kiefer in einer Erfassungsposition relativ zueinander, während sich der Abdruck in einer Position relativ zu dem Kieferknochen befindet, die eine Referenzposition für die durch den Abdruck definierte Schablonenkontaktfläche bereitstellt.

In Block 302 werden Strukturdaten des Kieferknochens erfasst, während das Bissregistrat zwischen den beiden Kiefern in der Erfassungsposition angeordnet ist. In Block 304 werden Positionsdaten des Bissregistrats relativ zu dem Kieferknochen erfasst, während das Bissregistrat zwischen den beiden Kiefern in der Erfassungsposition angeordnet ist. Das Erfassen der Kieferknochenstrukturdaten in Block 302 und das Erfassen der Positionsdaten in Block 304 erfolgt beispielsweise simultan im Zuge eines Scan-Verfahrens. Die Formdaten in Block 300 können beispielsweise im Zuge desselben Scan-Verfahrens erfasst werden, falls das Bissregistrat opak für das entsprechende Scan-Verfahren ist. Falls des Bissregistrat für das entsprechende Scan-Verfahren nicht opak ist, erfolgt das Erfassen der Formdaten beispielsweise in einem extra Schritt unter Verwendung eines zweiten Scan-Verfahrens. Beispielsweise wird das Bissregistrat zwischen den Kiefern des Patienten angeordnet und Abdrücke der Kieferoberflächen in dem Bissregistrat erzeugt. Während das Bissregistrat zwischen den beiden Kiefern angeordnet ist, können die Blöcke 302 und 304 ausgeführt werden. Anschließend kann das Bissregistrat mit den Abdrücken aus dem Mundraum des Patienten entfernt werden und die Formdaten des oder der Abdrücke können gemäß Block 300 unter Verwendung des zweiten Scan-Verfahrens erfasst werden.

In Block 306 wird unter Verwendung der in den Blöcken 300, 302 und 304 erfassten Daten, d.h. der Form-, Struktur- und Positionsdaten, ein erstes dreidimensionales digitales Modell der Kieferknochenstruktur und der Gingivaoberfläche erstellt. In Block 308 werden unter Verwendung des in Block 306 erstellten Modells erste Implantatpositionen zum Einbringen der ersten Implantatbohrungen in den Kiefer, d.h. durch die Gingiva hindurch in den Kieferknochen hinein, festlegt. Beispielsweise werden digitale Modelle der Implantatbohrungen zu dem ersten Modell hinzugefügt. In Block 310 wird unter Verwendung des ersten Modells ein zweites dreidimensionales digitales Modell der Bohrschablone(n) und der Positionierungselement(e) erstellt. Ferner werden die in Block 308 festgelegten Implantatpositionen berücksichtigt. Form und Abmessungen der Positionierungselemente werden so bestimmt, dass die beiden Kiefer beim Aufbringen von Druck auf das oder die Positionierungselemente in der Erfassungsposition angeordnet werden. Ferner werden die Form und Abmessungen so bestimmt, dass die erste Bohrschablone(n) in einer Position relativ zu dem Kieferknochen angeordnet werden, welche der Referenzposition entspricht.

In der Bohrschablone werden beispielsweise jeweils an den festgelegten Implantatpositionen Durchgangsöffnungen vorgesehen, durch welche hindurch die Implantatbohrungen in den Kiefer eingebracht werden können. Ferner können in Block 308 zusätzlich zu den Implantatpositionen Fixierungspositionen zum Einbringen von Fixierungsbohrungen in den Kiefer, d.h. durch die Gingiva hindurch in den Kieferknochen hinein, festlegt werden. Beispielsweise werden digitale Modelle der Fixierungsbohrungen zu dem ersten Modell hinzugefügt. Die Fixierungsbohrungen dienen zum Einbringen von Fixierungselementen durch die Bohrschablone(n) hindurch in den Kiefer bzw. Kieferknochen. Die Fixierungselemente sind konfiguriert zum kieferdruckunabhängigen Fixieren der jeweiligen Bohrschablone in der Referenzposition relativ zum Kiefer bzw. Gegenkiefer zum Einbringen der Implantatbohrungen. In Block 310 werden in der Bohrschablone beispielsweise jeweils an den festgelegten Fixierungspositionen zusätzliche Durchgangsöffnungen vorgesehen, durch welche hindurch die Fixierungselemente in den Kiefer eingebracht werden können.

In Block 312 werden die Positionierungselemente und/oder die Bohrschablonen unter Verwendung des zweiten Modells hergestellt. Beispielsweise erfolgt die Herstellung unter Verwendung einer computergesteuerten Herstellungsvorrichtung, wie etwa einer CAM-Vorrichtung. Die Herstellungsvorrichtung umfasst beispielsweise eine 3D-Drucker zum Drucken von Positionierungselementen und/oder Bohrschablonen. Zusätzlich oder alternativ kann die Herstellungsvorrichtung beispielsweise eine Bearbeitungsvorrichtung zum Bearbeiten eines Rohlings bereitstellen, aus welchem Positionierungselemente und/oder die Bohrschablonen etwa durch ein spanabhebendes Bearbeitungsverfahren herausgearbeitet werden.

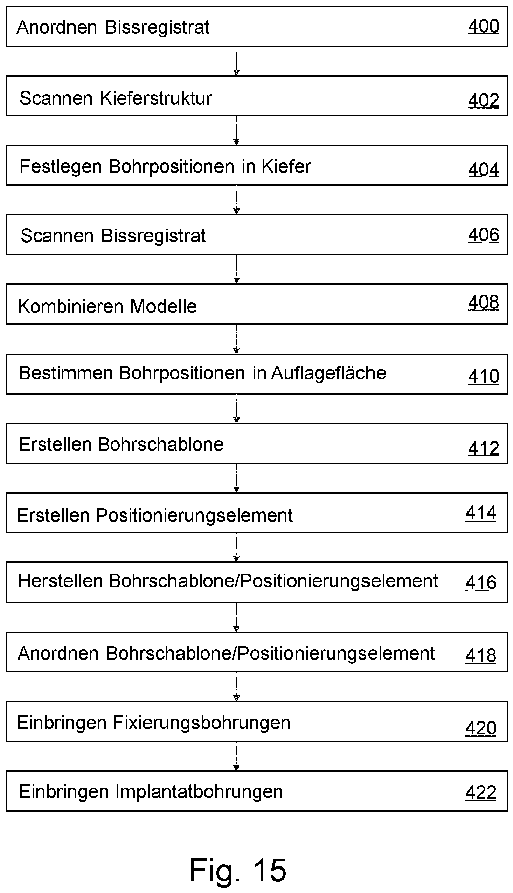

Figur 15 zeigt ein Flussdiagramm eines exemplarischen Verfahrens zum Herstellen und Verwenden eines Positionierungselements sowie einer Bohrschablone. In Block 400 wird beispielsweise ein Bissregistrat im Mund des Patienten angeordnet und ein Abdruck erstellt. Bei diesem Abdruck handelt es sich beispielsweise um einen Abdruck der Gingiva eines Kiefers oder zweier Kiefer des Patienten. Im Falle eines Kiefers handelt es sich beispielsweise um den Ober- oder Unterkiefer des Patienten. Der Kiefer, dessen Abdruck genommen wird, kann teilweise oder vollständig zahnlos sein. Falls der Kiefer vollständig zahnlos ist, umfasst der Abdruck beispielsweise nur die Oberfläche der Gingiva. Falls der Kiefer teilweise zahnlos ist, umfasst der Abdruck beispielsweise neben der Oberfläche der Gingiva den oder die Zähne. Daneben kann der Abdruck gegebenenfalls im bzw. am Kiefer angeordnete Zahnersatzteile umfassen. Bei dem Abdruck kann es sich beispielsweise um einen Abdruck des vollständigen Kiefers oder eines Teils des Kiefers handeln.

Beispielsweise weist das Bissregistrat eine Höhe auf, sodass der Abstand zwischen den Kiefern beim Beißen auf das Bissregistrat in etwa dem Abstand zwischen den Kiefern mit dem zukünftigen Zahnersatz entspricht. Beispielsweise weist das Bissregistrat eine Höhe auf, sodass es beim Beißen mit einem zahnlosen Kiefer die Höhe der fehlenden Zahnreihe des zahnlosen Kiefers kompensiert. Beispielsweise weist das Bissregistrat eine Höhe auf, sodass es beim Beißen mit zwei zahnlosen Kiefern die Höhe der beiden fehlenden Zahnreihe der zahnlosen Kiefer kompensiert. Die Bohrschablone könnte beispielsweise zum Einbringen von Implantaten für einen Zahnersatz zusätzlich zu einem bereits vorhandenen Zahnersatz dienen. Beispielsweise trägt der Patient während der Erstellung des Bissregistrats einen bereits vorhandenen Zahnersatz. Beispielsweise weist das Bissregistrat eine Höhe auf, sodass der Abstand zwischen den Kiefern beim Beißen auf das Bissregistrat größer ist als der Abstand zwischen den Kiefern mit dem zukünftigen Zahnersatz. Beispielsweise ist die Höhe so gewählt, dass der Abstand zwischen Kiefern es einem Zahnarzt erlaubt Implantatbohrungen in einen oder beide Kiefer einzubringen.

Beispielsweise kann sichergestellt werden, dass beim Beißen auf das Bissregistrat eine Kieferstellung erreicht wird, in welcher das Bissregistrat den Bereich der Gingiva, auf welchem bzw. an welchem die zu erstellenden Bohrschablone anzuordnen ist, möglichst vollständig abdeckt bzw. mit diesem in Kontakt tritt. Dies könnte den Vorteil haben, dass die Form und/oder Struktur der Oberfläche des entsprechenden Bereichs der Gingiva präzise erfasst werden kann. Ein präzises erfassen der Form und/oder Struktur der Oberfläche ermöglicht eine Bohrschablone mit einer Schablonenkontaktfläche zu erstellen, welche fest und präzise auf bzw. an der Gingiva sitzt.

Beispielsweise drückt der Patient mit seinen Kiefern mit einer konstanten niedrigen Bisskraft auf das Bissregistrat. Dabei könnte das Bissregistrat einen Druck- bzw. Kraftmesser umfassen, welcher die Bisskraft bzw. den aufgebrachten Druck misst. Dem Patienten könnte eine Rückmeldung, beispielsweise optisch oder akustisch, gegeben werden, welche dem Patienten mitteilt, sobald eine vorbestimmte Bisskraft erreicht ist.

Beispielsweise umfasst das Bissregistrat eine Mehrzahl von Markern, welche opak, etwa radioopak, für ein Scan-Verfahren sind, das zum Erfassen der Strukturdaten des Kieferknochens verwendet wird. Beispielsweise können die Marker an dem Bissregistrat angebracht sein und/oder Teile des Bissregistrat selbst können als Marker konfiguriert, d.h. opak, sein. Beispielsweis ist das Bissregistrat abgesehen von den Markern transparent für das entsprechende Scan-Verfahren. Dies könnte den Vorteil haben, dass sich anhand der Marker, welche in einem Scan der Strukturdaten des Kiefers sichtbar sind, die Position des Bissregistrats relativ zu dem Kiefer, während des Erfassens der Strukturdaten, bestimmen lässt. Dass das Bissregistrat ansonsten transparent ist, könnte ferner den Vorteil haben, dass die Strukturdaten des Kiefers beim Scannen von dem Bissregistrat nicht verdeckt werden.

Beispielsweise wird zum Erfassen der Strukturdaten des Kieferknochens ein Scan-Verfahren verwendet, für welches die Gingiva des entsprechenden Kiefers transparent, d.h. nicht sichtbar, ist. Das Bissregistrat sitzt nicht direkt auf dem Kieferknochen auf, sondern auf der Gingiva und ist dadurch abhängig von der Beschaffenheit der Gingiva beabstandet von dem Kieferknochen angeordnet. Somit lässt sich bei einem für das entsprechende Scan-Verfahren transparenten Bissregistrat aus den Scan-Daten die relative Position des Bissregistrats zu dem Kieferknochen, während des Erfassens der Strukturdaten, nicht bestimmen. Dies gilt insbesondere für zahnlose Kiefer, bei welchen beispielsweise nur die Kieferknochenstruktur in dem entsprechenden Scan sichtbar ist. Anhand entsprechender Marker, etwa drei oder mehr, könnte die Position des Bissregistrats relativ zu dem Kiefer präzise bestimmt werden. Anhand des Bissregistrats selbst kann wiederum die Position des Abdrucks der Oberfläche der Gingiva relativ zu den Markern bestimmt werden, etwa unter Verwendung eines zweiten Scan-Verfahrens. Sind die relativen Positionen von Markern zu Kiefer und Markern zu Oberfläche der Gingiva bekannt, kann daraus beispielsweise die relative Position von Oberfläche der Gingiva zum Kieferknochen, während des Erfassens der Strukturdaten, bestimmt werden, selbst wenn die Gingiva an sich transparent für das entsprechende Scan-Verfahren zum Erfassen der Strukturdaten ist.

Beispielsweise sind beide Kiefer zahnlos, sodass im Zuge eines Scan-Verfahrens der Kieferknochen keine feste Referenzpunkte, wie etwa Zähne, für ein Bestimmen der relativen Position von Bissregistrat bzw. Abdruck des Bissregistrats und Kiefer erfasst werden können. Da die Gingiva selbst nur weiches Gewebe umfasst, kann diese aufgrund ihrer Nachgiebigkeit keine geeigneten Referenzpunkte für präzise Positionsbestimmungen bereitstellen. Mit anderen Worten könnte eine von dem Beißen auf das Bissregistrat unabhängige Bestimmung einer relativen Position der Oberfläche der Gingiva zu dem Kiefer aufgrund der abweichenden Drückverhältnisse keine präzisen Rückschlüsse auf die relative Position der Gingivaoberfläche zu dem Kiefer, während des Erfassens der Strukturdaten, zulassen.

Beispielsweise sind die Marker zusätzlich zum Erfassen mittels eines weiteren Scan-Verfahrens, etwa einem haptischen oder optischen Scan-Verfahren, konfiguriert. Beispielsweise wird dieses weitere Scan-Verfahren zum Erfassen von Abdrücken des Bissregistrats, insbesondere der Oberfläche der Gingiva, verwendet.

Sind dentale Objekte, wie etwa Zähne, in dem Kiefer oder in dem Gegenkiefer vorhanden, welche einen Abdruck in dem Bissregistrat hinterlassen und opak für das verwendete Scan-Verfahren sind, können diese dentalen Objekte als Referenzstrukturen verwendet. Beispielsweise sind anderer Referenzobjekte, wie etwa ein bereits vorhandener Zahnersatz, vorhanden, welche einen Abdruck in dem Bissregistrat hinterlassen und opak für das verwendete Scan-Verfahren sind. Dies könnte den Vorteil haben, dass aus der relativen Position der entsprechenden Referenzpunkte bzw. Referenzobjekte zu dem Kieferknochen und der relativen Position des Abdrucks der entsprechenden Referenzpunkte bzw. Referenzobjekte zu dem Abdruck der Oberfläche der Gingiva die relative Position der Gingivaoberfläche zu dem Kieferknochen bestimmt werden kann. Bei Gegenwart entsprechender Referenzpunkte bzw. Referenzobjekte könnte eine Verwendung von Marken unnötig sein oder die Anzahl der notwendigen Marker könnte reduziert werden, beispielsweise auf zwei oder eins. Beispielsweise könnten entsprechende Referenzpunkte bzw. Referenzobjekte zur Überprüfung einer markerbasierten Positionsbestimmung verwendet werden oder umgekehrt.

In Block 402 wird beispielsweise die Knochenstruktur der Kiefer gescannt, während der Bissregistrat im Mund angeordnet ist. Beispielsweise wird für den Scan der Knochenstruktur eine Scanstärke verwendet, welche darauf eingestellt ist die Densität der Knochen möglichst präzise zu erfassen.

Als Ergebnis des Scans wird beispielsweise eine Darstellung (D1) des oder der Kiefer des Patienten bereitgestellt. Beispielsweise handelt es sich bei der Darstellung D1 um ein dreidimensionales digitales Modell der Knochenstruktur des Kiefers.

Zum Bereitstellen der Darstellung D1 wird beispielsweise der DICOM-Standard verwendet. DICOM (engl.: "Digital Imaging and Communications in Medicine") ist ein offener Standard zur Speicherung und zum Austausch von Informationen im medizinischen Bilddatenmanagement. Diese Informationen können beispielsweise digitale Bilder, Zusatzinformationen wie Segmentierungen, Oberflächendefinitionen, etc. oder Bildregistrierungen sein. DICOM standardisiert sowohl das Format zur Speicherung der Daten als auch das Kommunikationsprotokoll zu deren Austausch.

Abhängig von dem gewählten Scan-Verfahren und/oder den gewählten Scan-Einstellungen, welche beispielsweise optimiert sind zum Erfassen der Knochendensität, kann es vorkommen, dass das Bissregistrat nicht oder nur teilweise miterfasst bzw. aufgenommen wird, d.h. dass das Bissregistrat vollständig oder zumindest teilweise transparent für das entsprechende Scan-Verfahren ist.

Als Scan-Verfahren zum Erfassen der Strukturdaten des Kiefers wird beispielsweise eine Computertomographie (CT), eine digitale Volumentomographie (DVT) oder eine Magnetresonanztomographie (MRT) verwendet. Beispielsweise werden im Zuge einer Computertomographie rechnergestützt eine Vielzahl von aus verschiedenen Richtungen aufgenommener Röntgenbilder ausgewertete, um ein überlagerungsfreies, zwei- oder dreidimensionales Darstellung der Knochenstruktur zu erstellen.

Beispielsweise werden die Ergebnisse des Scan-Verfahrens dazu verwendet, die Knochenstruktur der Kiefer für eine spätere Positionierung der Implantatbohrungen exakt zu erfassen.

Beispielsweise umfassen der Kiefer oder der Gegenkiefer Zähne und/oder Zahnersatz, welche unter Verwendung des Scan-Verfahrens ebenfalls erfasst und beispielsweise ebenfalls in das DICOM übertragen werden.

Beispielsweise umfasst das Bissregistrat eine Mehrzahl von opaken Markern und/oder ein oder mehrere opake Teile, welche unter Verwendung des Scan-Verfahrens ebenfalls erfasst und beispielsweise ebenfalls in das DICOM übertragen werden. Opak bezieht sich auf die Eigenschaft der entsprechende Marker und/oder Teile für die von dem Scan-Verfahren zum Erfassen der Strukturdaten verwendeten Strahlung opak und damit in dem entsprechenden Scan sichtbar zu sein.

Beispielsweise kann auf ein Erfassen des Gegenkiefers und/oder einem Bereitstellen der Strukturdaten des Gegenkiefers verzichtet werden, falls nur ein Kiefer zu versorgen ist und der Gegenkiefer beispielsweise keine Zähne und/oder Zahnersatz aufweist. Beispielsweise wird auf eine Übertragung der Strukturdaten des Gegenkiefers in das DICOM verzichtet. Dies könnte den Vorteil haben, dass der Gegenkiefer, falls dieser nicht zu versorgen ist und zudem keine Referenzpunkte in Form von Zähnen oder Zahnersatz aufweist, vernachlässigt werden kann. Als Referenzpunkte könnten opake Marker und/oder opake Teile des Bissregistrats verwendet werden.

Beispielsweise kann während des Scans zum Erfassen der Strukturdaten ein weiteres Objekt, welcher opak für das Scan-Verfahren ist, als Referenzobjekt im Mundraum angeordnet und erfasst werden. Beispielsweise weist dieses weitere Objekt, wenn es in dem Mundraum angeordnet ist, eine messbare relative Position zu dem Bissregistrat und dem Kieferknochen auf.

In Block 404 werden Positionen für die Implantatbohrungen und/oder Fixierungsbohrungen im Kiefer unter Verwendung des 3D-Modells D1 der Kieferknochenstruktur geplant und digital festgelegt. Die Positionen der Bohrungen, insbesondere der Implantatbohrungen, werden beispielsweise so festgelegt, dass ein oder mehrere Positionierungskriterien erfüllt werden. Hierbei werden beispielsweise Informationen über die Knochenstruktur, insbesondere die Knochenwandstärke, die Positionen der Hauptnerven und/oder die Positionen der Adern im Kiefer verwendet werden. Die Positionierungskriterien können beispielsweise eine oder mehrere der folgenden Kriterien umfassen: Erstrecken der Bohrung durch eine Knochenwand mit einer vordefinierten Mindeststärke, Einhalten eines Mindestabstands der Bohrung von den Hauptnerven im Kiefer und/oder Einhalten eines Mindestabstands der Bohrung zu den Adern im Kiefer.

Beispielsweise wird das Modell D1 zur Planung der Positionen der Bohrungen ferner um visuelle Darstellungen bzw. Modelle der von dem herzustellenden Zahnersatz bereitgestellten Zähnen ergänzt. Zur Visualisierung der Zähne werden beispielsweise Zahnmodelle aus Bibliotheken und/oder Aufnahmen originaler Zähne des Patienten verwendet.

Alternativer Weise kann für die Planung der Positionen der Bohrungen auch keine Visualisierung von Zähnen erfolgen, welche der herzustellende Zahnersatz umfasst.

Beispielsweise wird zusätzlich zu den Implantatbohrungen eine Mehrzahl von Fixierungsbohrungen eingeplant, um die Bohrschablone präzise an einer vordefinierten Position auf bzw. an der Gingiva des Patienten zu fixieren. Dies könnte den Vorteil haben, dass obwohl die Gingiva für die Bohrschablone lediglich eine weiche Auflagefläche bereitstellt, dennoch eine stabile druckunabhängige Fixierung der Bohrschablone während des Einbringens der Implantatbohrungen sichergestellt werde kann. Dies kann es beispielsweise ermöglichen, dass der Patient seinen Mund öffnen kann, ohne dass die Bohrschablone verrutscht.

Beispielsweise erfolgt die Planung des Positionierungselements und der Bohrschablone auf Basis der relativen Position der Kiefer zueinander während des Scans der Knochenstrukturen, d.h. der Erfassungsposition der Kiefer. Dabei kann beispielsweise die für die Erfassung verwendete relative Position der Kiefer zueinander von untergeordneter Bedeutung sein. Beispielsweise kommt es bei der Wahl des Abstands der Kiefer während der Erfassung nicht auf eine millimetergenaue Einstellung eines vordefinierten Abstands zwischen den Kiefer an. Beispielsweise kann für die Wahl der Abmessungen des Bissregistrats und damit für die Anordnung der Kiefer in der Erfassungsposition vernachlässigt werden, wie potentielle, von einem Zahnersatz bereitgestellte Zähne in der für die Aufnahme gewählten Position der Kiefer zueinanderstehen würden. So ist es beispielsweise nicht notwendig, dass für die Erfassungsposition eine Okklusion mit den entsprechenden potentiellen Zähnen geben ist.

Beispielsweise erfolgt die Planung der Fixierungsbohrungen für die Erfassungsposition der Kiefer. Beispielsweise erfolgt die Planung der Implantatbohrungen für die Erfassungsposition der Kiefer. Beispielsweise wird für die Planung der Implantatbohrungen der mit den Implantaten zu befestigende Zahnersatz berücksichtigt.

Beispielsweise können die Abmessungen des Bissregistrats und damit die Anordnung der Kiefer in der Erfassungsposition so gewählt werden, dass die Kiefer weit genug geöffnet sind, um ein Einbringen der Implantatbohrungen zu ermöglichen. Wird diese Kieferstellung mit dem Positionierungselement nachgestellt, könnte es ermöglicht werden, die Implantatbohrungen in den Kiefer einzubringen, ohne das Positionierungselement zu entfernen. Beispielsweise weist das Positionierungselement Durchgangsöffnungen auf, durch welche die Implantatbohrungen ausgeführt werden können. Beispielsweise weist das Positionierungselement eine Gitterstruktur mit einer Mehrzahl von Stützelementen auf, zwischen denen entsprechende Durchgangsöffnungen angeordnet sind.

Beispielsweise entsprechen die Implantatbohrungen, welche zum Verankern der Implantate verwendet werden, nicht notwendigerweise jeweils einem Zahn. Beispielsweise werden Positionierungselement und Bohrschablone so geplant, dass die Implantatbohrungen exakt an den geplanten Positionen relativ zu dem jeweiligen Kieferknochen eingebracht werden, welche jeweils die ein oder mehreren Positionierungskriterien erfüllen. Demgegenüber kann die Positionierung relativ zu den später zu ankernden Zähnen bzw. dem Zahnersatz beispielsweise eine untergeordnete Rolle spielen und/oder vernachlässigt werden. Mithin könnte bei der Planung der Implantatbohrungen beispielsweise eine Positionspriorität für eine sichere und stabile Verankerung gelten, welche höher eingestuft werden könnte als eine millimetergenaue Positionierung und/oder Ausrichtung an zukünftigen Zähnen.

In Block 406 wird das Bissregistrat mit dem Abdruck der Gingiva als Auflageflächen für die Schablonenkontaktfläche der zu erstellende Bohrschablone unter Verwendung eines zweiten Scan-Verfahrens gescannt und eine dreidimensionale digitale Darstellung (D2) des Bissregistrats erstellt. Beispielsweise handelt es sich bei der Darstellung D2 um ein dreidimensionales digitales Modell des Bissregistrats. Die zweite Darstellung D2 umfasst beispielsweise die Auflageflächen für die Bohrschablonen auf bzw. an der Gingiva, welche die Schablonenkontaktfläche der Bohrschablone definiert.

Sollen Bohrschablonen für beide Kiefer des Patienten erstellt werden, umfasst das Bissregistrat beispielsweise Abdrücke der Gingivae beider Kiefer, welche jeweils eine Auflagefläche für eine Schablonenkontaktfläche einer der Bohrschablonen definieren.

Das zweiten Scan-Verfahrens umfasst beispielsweise einen optischen Scan, eine auf die Densität des Bissregistrats eingestellte Computertomographie, und/oder einen haptischen Scan.

Falls einer der Kiefer beispielsweise Zähne oder einen bereits vorhandenen Zahnersatz umfasst, könnten diese mittels des ersten Scan-Verfahrens erfasst und in dem dreidimensionale digitale Modell D1 der Kieferstrukturen umfasst sein. Beispielsweise werden diese vorhandenen Zähne und/oder der Zahnersatz als Referenzstrukturen verwendet werden, um das auf dem zweiten Scan-Verfahren basierende dreidimensionale digitale Modell D2 des Bissregistrat relative zu dem auf dem ersten Scan-Verfahren basierenden dreidimensionalen digitalen Modell D1 der Kiefer in derselben Position auszurichten, welche das Bissregistrat beim Erfassen der Strukturdaten zwischen den Kieferknochen eingenommen hat. Somit kann digital die relative Position und Ausrichtung des Bissregistrat gegenüber den Kieferknochen während des ersten Scan-Verfahrens präzise rekonstruiert werden. Die entsprechenden Referenzstrukturen können von dem Bissregistrat als Abdrücke und damit auch von dem dreidimensionale digitale Modell D2 des entsprechenden Bissregistrats umfasst sein. Somit können die entsprechenden Referenzstrukturen, wenn sie von beiden Modellen D1 und D2 umfasst sind, ein relatives Ausrichten der beiden Modelle ermöglichen.

Beispielsweise könnte ein relatives Ausrichten der beiden Modelle D1 und D2 zueinander unter Verwendung von opaken Markern erfolgen, welche das Bissregistrat umfasst. Die entsprechenden Marker sind beispielsweise opak, d.h. sichtbar, sowohl für das erste Scan-Verfahren zum Erfassen der Strukturdaten der Kiefer als auch für das zweite Scan-Verfahren zum Erfassen des Bissregistrats. Falls die Marker für beide Scan-Verfahren sichtbar sind, können beispielsweise beide Modelle D1 und D2 die entsprechenden Marker umfassen. Beispielsweise indem die Marker der beiden Modelle zur Deckung gebracht werden, können die beiden Modelle D1 und D2 in derselben Position relativ zueinander auszurichten, welche das Bissregistrat und die Kieferknochen während des Erfassens der Strukturdaten eingenommen haben. Somit kann digital die relative Position und Ausrichtung des Bissregistrat gegenüber den Kieferknochen während des ersten Scan-Verfahrens präzise rekonstruiert werden.

Die Ausrichtung der beiden Modelle D1 und D2 zueinander resultiert beispielsweise in einem kombinierten 3D-Modell, welches die Kieferknochenstruktur und die Oberfläche der Gingiva zumindest eines Kiefers, bei Berücksichtigung beider Kiefer, die Oberflächen der Gingivae beider Kiefer umfasst. In diesem kombinierten Modell entspricht die relative Positionierung und Ausrichtung der von dem Bissregistrat definierten Auflagefläche der Gingiva zu dem Kieferknochen beispielsweise exakt der tatsächlichen relativen Positionierung und Ausrichtung von Gingivaoberfläche und Kieferknochen während der Erfassung der Strukturdaten, d.h. mit dem Bissregistrat im Mund des Patienten.

Dies könnte den Vorteil haben, dass beispielsweise ein optischer Scan des Bissregistrat ausreichen kann, aus welchem eine 3D-Netz-, Punkt- oder Voxelstruktur der Auflageflächen der Gingiva und/oder der Zähne für die herzustellende Bohrschablone erstellt werden kann. Beispielsweise kann auf einen komplizierten CT-Scan verzichtet werden, falls optisch erfassbare Referenzpunkte, wie beispielsweise Zähne, Zahnersatz oder Marker vorhanden sind.

Bei dem Netz (engl.: "Mesh") handelt es sich beispielsweise um ein Polygonnetz, dessen einzelnen Netzmaschen die Form von Polygonen aufweisen und welches die Gestalt eines Polyeders definiert. Ein Polygonnetz umfasst Eckpunkte, Kanten und zwischen den Kanten aufgespannte Facetten, welche die Gestalt eines polyedrischen Objekts in 3D-Computergrafiken und Volumenmodellen definiert.

Das aus dem zweiten Scan erzeugte 3D-Modell umfasst im Falle eines mit Zähnen bestückten Gebisses beispielsweise einen genauen Abdruck der Antagonisten des Gegenkiefers, welche als Referenzstrukturen für eine genaue Positionierung des Modells relativ zu der Kieferknochenstruktur verwendet werden kann. Dies könnte den Vorteil haben, dass im Falle einer Verwendung von Zähnen als Referenzstrukturen das DICOM-Format der ersten 3D-Darstellung D1 leicht mit dem von der zweiten 3D-Darstellung bereitgestellten bzw. extrahierten Modell in Relation gebracht werden kann.

Im Falle eines vollständig zahnlosen Gebisses umfasst das Bissregistrat beispielsweise Abdrücke der zahnlosen Gingivae beider Kiefer. Das Bissregistrat wird gescannt und digitalisiert um ein zweites 3D-Modell D2 zu erzeugen. Diese zweite 3D-Modell D2 umfasst beispielsweise Marker des Bissregistrats, welche ebenfalls von dem ersten 3D-Modell D1 umfasst sind. Diese Marker können als Referenzpunkte und/oder Referenzstrukturen für ein Ausrichten der beiden Modelle D1 und D2 verwendet werden, wenn keine Zähne vorhanden sind, welche als Referenzpunkte verwendet werden können. Dies könnte den Vorteil haben, dass unter Verwendung der Marker das zweite Modell D2 relativ zu dem ersten Modell D1 in exakter Übereinstimmung mit der Erfassungssituation beim Ausführen des ersten Scans positioniert werden kann.

Das zweite Modell D2 umfasst beispielsweise die aus den Scan-Daten des zweiten Scans extrahierten Abdrücke der Auflageflächen der Gingivae, die von dem Bissregistrat aufgenommen wurden und die Schablonenkontaktflächen für die herzustellenden Bohrschablonen definieren. Das relative Positionieren und Ausrichten der Modelle zueinander resultiert beispielsweise in einem kombinierten Modell, in welchem die von dem Bissregistrat aufgenommenen Auflageflächen der Gingivae in exakter Übereinstimmung mit der Erfassungssituation relativ zu den Kieferknochen dargestellt werden.

Beispielsweise sind die Marker sowohl für das erste als auch für das zweite Scan-Verfahren opak. Beispielsweise handelt es sich bei dem ersten und dem zweiten Scan-Verfahren um die gleichen Scan-Verfahren mit unterschiedlichen Parametereinstellungen. Beispielsweise handelt es sich bei beiden Scan-Verfahren um ein Computertomographie-Verfahren, welches im Falle des ersten Scans auf die Densität der Kieferknochen, im Falle des zweiten Scans auf die Densität des Bissregistrats eingestellt ist. Dies könnte den Vorteil haben, dass in beiden Fällen jeweils die Marker erfasst werden können. Im zweiten Fall könnten zugleich die Auflageflächen der Gingivae, d.h. die Oberflächen des Bissregistrats, präzise erfasst werden.

Beispielsweise handelt es sich bei dem ersten und dem zweiten Scan-Verfahren um unterschiedliche Scan-Verfahren. Beispielsweise sind die Marker nicht nur für das erste Scan-Verfahren, wie etwa ein Computertomographie-Verfahren, ein Volumentomographie-Verfahren oder ein Magnetresonanztomographie-Verfahren, erfassbar, sondern auch für das zweite Scan-Verfahren, etwa ein optisches Scan-Verfahren im optischen Frequenzbereich oder ein haptisches Scan-Verfahren.

In Block 408 werden die beiden Modelle D1 und D2 der Kieferknochen und des Bissregistrats beispielsweise zusammengefügt.

Das erste Modell D1, beispielsweise in Form eines 3D-DICOM, des Kiefers wird mit dem zweiten Modell D2, beispielsweise in Form einer 3D-Netz-, Punkt- oder Voxelstruktur, der Gingivaoberfläche unter Verwendung der Referenzpunkte und/oder Referenzstrukturen relativ zueinander gemäß der Ausrichtung beim Erfassen der Strukturdaten gekoppelt. Diese Referenzpunkte und/oder Referenzstrukturen können in den beiden Modellen D1 und D2 beispielsweise von Hand oder automatisch per Computer, beispielsweise unter Verwendung eines Bilderkennungsalgorithmus, identifiziert und/oder selektiert werden. Beispielsweise handelt es sich bei den Referenzpunkte und/oder Referenzstrukturen um Zähne, Zahnersatz oder Marker. Beispielsweise weisen die Marker eine spezifische geometrische Form auf.

Dies könnte den Vorteil haben, dass die Auflageflächen der Gingivae exakt relativ zu den Kieferknochen positioniert und extrahiert werden können. Die extrahierten Auflageflächen an den exakten relativen Positionen zu den Kieferknochen können zur Erstellung einer 3D-Darstellung verwendet werden, welche die geplanten Bohrungen und die Schablonenkontaktflächen der Bohrschablonen in exakter Relation zu einander umfasst.

In Block 410 werden beispielsweise Fixierungspositionen in den Auflageflächen der Gingiva bzw. Gingivae zum Einbringen von Fixierungsbohrungen in den Kiefer und/oder Implantatpositionen in den Auflageflächen der Gingiva bzw. Gingivae zum Einbringen von Implantatbohrungen in den Kiefer unter Verwendung des kombinierten Modells festgelegt.

In Block 412 werden beispielsweise die Auflageflächen der Gingiva bzw. Gingivae mit den Positionen der geplanten Bohrungen, d.h. Fixierungsbohrungen und/oder Implantatbohrungen, extrahiert und eine oder zwei Bohrschablonen erstellt, welche jeweils ein Negativ der Auflagefläche als Schablonenkontaktfläche umfassen und so exakt an die entsprechende Gingiva angepasst sind.

In die zu erstellende Bohrschablone werden an den Implantatpositionen jeweils Durchgangsöffnungen in einer Linie, d.h. koaxial, mit den geplanten Implantatbohrungen eingeplant. Dies könnte den Vorteil haben, dass so eine führende Bohrschablone zur Durchführung von Implantatbohrungen für dentale Implantate bereitgestellt werden kann. Unter Verwendung dieser Bohrschablone können die Positionen der Implantatbohrungen exakt bestimmt werden.

Die Bohrschablone könnte zusätzlich zu den Durchgangsöffnungen, welche die Positionen der zu erstellenden Fixierungsbohrungen definieren bzw. identifizieren, beispielsweise Bohrführungselemente umfassen, mit welchen die entsprechenden Durchgangsöffnungen versehen sind und die dazu konfiguriert sind, ein beim Einbringen der Implantatbohrungen verwendetes Bohrwerkzeug zu führen und/oder die Bohrtiefe der Implantatbohrungen zu begrenzen.

Beispielsweise können in analoger Weise zusätzliche Durchgangsöffnungen zum Erstellen, beispielsweise seitlich angeordneter, Fixierungsbohrungen in die Bohrschablone eingeplant werden. Die Bohrschablone könnte zusätzlich zu den Durchgangsöffnungen, welche die Positionen der zu erstellenden Fixierungsbohrungen definieren bzw. identifizieren, beispielsweise Bohrführungselemente umfassen, mit welchen die Durchgangsöffnungen versehen sind und die dazu konfiguriert sind, ein beim Einbringen der Fixierungsbohrungen verwendetes Bohrwerkzeug zu führen und/oder die Bohrtiefe der Fixierungsbohrungen zu begrenzen.

In Block 414 wird beispielsweise ein Positionierungselement erstellt, welches dazu konfiguriert ist zwischen den Kiefern auf der Bohrschablone bzw. zwischen den Bohrschablonen angeordnet zu werden und eine druckbasierte Fixierung der Bohrschablone bereitzustellen. Übt der Patient mit seinen Kiefern Druck auf das Positionierungselement aus, d.h. beißt er auf dieses, so wird die Bohrschablone bzw. werden die Bohrschablonen beispielsweise jeweils zwischen dem Positionierungselement und einem Kiefern des Patienten auf bzw. an welchem die Bohrschablone angeordnet ist, fixiert. Genauer gesagt kann das Positionierungselement dazu konfiguriert sein, die Bohrschablone in einer vordefinierten Position auf bzw. an der Gingiva zu fixieren, wobei die vordefinierte Position mit der Position des Bissregistrats während des ersten Scan-Verfahrens zum Erfassen von Strukturdaten übereinstimmt. Dies könnte ferner den Vorteil haben, dass es ermöglicht wird mit beiden Kiefern Druck auf die Bohrschablone auszuüben.

Beispielsweise wird zum Fixieren der Bohrschablone genau ein Positionierungselement verwendet. Beispielsweise wird für jede Bohrschablone jeweils ein Positionierungselement verwendet. Beispielsweise ist das Positionierungselement jeweils einstückig ausgestaltet. Beispielsweise werden die Positionierungselemente jeweils von einer Bohrschablone umfasst. Beispielsweise wird das genau eine Positionierungselement im Falle zweier Bohrschablonen von beiden Bohrschablonen gemeinsam umfasst.

Das Positionierungselement könnte den Vorteil haben, dass ein Positionieren und druckbasiertes Fixieren der Bohrschablone erleichtert wird. Dabei wird die Bohrschablone beispielsweise in korrekter räumlicher Relation zu dem Kieferknochen gehalten. Beispielweise kann das Positionierungselement die Bohrschablone in korrekter räumlicher Korrelation zum Knochen des Gegenkiefers halten. Beispielsweise kann das Positionierungselement im Falle zweier Bohrschablonen die beiden Bohrschablonen in korrekter räumlicher Relation zueinander halten.

Beispielsweise sind die Positionierungselemente jeweils mittels einer zerstörungsfrei lösbaren Verbindung mit den Bohrschablonen verbunden. Beispielsweise ist das genau eine Positionierungselement mit jeder der beiden Bohrschablonen jeweils mit einer zerstörungsfrei lösbaren Verbindung verbunden. Dies könnte den Vorteil haben, dass das Positionierungselement nach dem Einbringen der Fixierungsbohrungen und der Fixierungselemente entfernt werden kann und die Bohrschablone mittels der Fixierungselemente in der vordefinierten Position an der Gingiva gehalten wird. Dadurch könnte ein besserer Zugang zu den Durchgangsöffnungen in der Bohrschablone zum Einbringen der Implantatbohrungen bereitgestellt werden.

Dies könnte ferner den Vorteil haben, dass relative Verschiebungen, insbesondere seitliche Verschiebungen, zwischen dem Positionierungselement und der/den Bohrschablone verhindert werden können.

Beispielsweise wird die zerstörungsfrei lösbare Verbindung zwischen den Positionierungselementen unter Verwendung ineinandergreifender Strukturen bereitgestellt. Beispielsweise bilden die ineinandergreifenden Strukturen einen Formschluss. Beispielsweise bilden die ineinandergreifenden Strukturen eine Steckverbindung. Beispielsweise weist das Positionierungselement weibliche Teile zur Aufnahme männlicher Teile der Bohrschablone und/oder männliche Teile zur Aufnahme in weiblichen Teilen der Bohrschablone auf. Beispielsweise weist die Bohrschablone weibliche Teile zur Aufnahme männlicher Teile des Positionierungselements und/oder männliche Teile zur Aufnahme in weiblichen Teilen des Positionierungselements auf.

Beispielsweise umfasst das Positionierungselement eine Kieferkontaktfläche für den Gegenkiefer, deren Form dem unter Verwendung des Bissregistrats erfassten Abdruck des Gegenkiefers bzw. von Gingiva, Zähnen und/oder Zahnersatzteilen, wie etwa Implantaten und/oder Prothesen, des Gegenkiefers entspricht.

Beispielsweise umfasst die Kombination aus Positionierungselement und Bohrschablone(n) zwei Kontaktflächen, etwa zwei Schablonenkontaktflächen oder eine Schablonenkontaktfläche und eine Kieferkontaktfläche des Positionierungselements, für die beiden Kiefer, deren Formen den unter Verwendung des Bissregistrats erfassten Abdrücken der beiden Kiefer entsprechen. Die relative Ausrichtung und der Abstand der beiden Kontaktflächen zueinander entspricht beispielsweise der relativen Ausrichtung und dem Abstand der beiden von dem Bissregistrat als Abdrücke erfassten Auflageflächen der Gingivae.

Unter Verwendung der Kombination aus Positionierungselement und Bohrschablone(n) lässt sich beispielsweise die Position und Ausrichtung der Kiefer während der ersten Scan-Verfahrens exakt rekonstruiert. Anhand des Ergebnisses dieses ersten Scan-Verfahrens wurden die Fixierungspositionen der Fixierungsbohrungen und/oder die Implantatpositionen der Implantatbohrungen festgelegt. Mithin kann sichergestellt werden, dass die Bohrschablone und die von ihr festgelegten Fixierungsbohrungen und/oder Implantatbohrungen exakt wie geplant positioniert werden können. Beispielsweise kann die Bohrschablone unter Verwendung der Fixierungsbohrungen exakt an der geplanten Position auf der Gingiva angeordnet werden zum Einbringen der Implantatbohrungen exakt an den geplanten Implantatpositionen.

Beispielsweise umfassen im Falle zweier Bohrschablonen beide Bohrschablonen jeweils ein Positionierungselement, was den Vorteil haben könnte, dass die beiden Bohrschablonen sich jeweils gegenseitig in einer vorbestimmten Position relativ zu den beiden Kiefern halten. Ferner könnte dies den Vorteil haben, dass kein zusätzliches Element zum Positionieren benötigt wird.

Beispielsweise umfassen im Falle zweier Bohrschablonen die beiden Bohrschablonen jeweils eine Schablonenkontaktfläche, deren Form jeweils einem der unter Verwendung des Bissregistrats erfassten Abdrücke der Gingivae entspricht. Die durch ein Anordnen von ein oder zwei Positionierungselementen zwischen den Bohrschablonen resultierende relative Ausrichtung und der Abstand der beiden Schablonenkontaktflächen zueinander entspricht beispielsweise der relativen Ausrichtung und dem Abstand der beiden von dem Bissregistrat als Abdrücke erfassten Auflageflächen.

Beispielsweise umfassen im Falle einer Bohrschablone die Bohrschablone ein Schablonenkontaktfläche und das Positionierungselement eine Kieferkontaktfläche, deren Form einem unter Verwendung des Bissregistrats erfassten Abdruck der Gingiva des Kiefers bzw. einem unter Verwendung des Bissregistrats erfassten Abdruck des Gegenkiefers entspricht. Die unter Verwendung des Positionierungselements resultierende relative Ausrichtung und der Abstand der Schablonenkontaktfläche und Kieferkontaktfläche zueinander entspricht beispielsweise der relativen Ausrichtung und dem Abstand der beiden von dem Bissregistrat als Abdrücke erfassten Auflageflächen.

Dies könnte den Vorteil haben, dass die Kiefer bei einer Verwendung der Bohrschablone in Kombination mit den ein oder zwei Positionierungselementen in der Erfassungsposition angeordnet werden und mit den Kiefern Druck auf die Bohrschablone ausgeübt werden kann. Durch diesen Druck können die Positionierungselementen an den Kiefern, beispielsweise zum Einbringen der Fixierungsbohrungen, vorläufig fixiert werden.

Beispielsweise umfassen im Falle zweier Bohrschablonen mit jeweils einem Positionierungselement die beiden Positionierungselemente ineinandergreifende Strukturen zum Bilden einer zerstörungsfrei lösbaren Verbindung auf. Dies könnte den Vorteil haben, dass eine relative Ausrichtung der beiden Bohrschablonen erleichtert wird. Die Bohrschablonen könnten sich mittels der Positionierungselemente gegenseitig in korrekter räumlicher Korrelation zu den Kiefern halten. Ferner könnten sich die Bohrschablonen mittels der Positionierungselemente gegenseitig in korrekter räumlicher Relation zueinander halten. Dies könnte den Vorteil haben, dass relative Verschiebungen, insbesondere seitliche Verschiebungen, zwischen den Bohrschablonen verhindert werden können.

Beispielsweise bilden die ineinandergreifenden Strukturen einen Formschluss. Beispielsweise bilden ineinandergreifenden Strukturen eine Steckverbindung. Beispielsweise weist das Positionierungselement einer ersten der beiden Bohrschablonen weibliche Teile zur Aufnahme männlicher Teile des Positionierungselements der zweiten Bohrschablone und/oder männliche Teile zur Aufnahme in weiblichen Teilen des Positionierungselements der zweiten Bohrschablone auf. Beispielsweise weist das Positionierungselement der zweiten Bohrschablone weibliche Teile zur Aufnahme männlicher Teile des Positionierungselements der ersten Bohrschablone und/oder männliche Teile zur Aufnahme in weiblichen Teilen des Positionierungselements der ersten Bohrschablone auf.

In Block 416 werden beispielsweise die ein oder zwei Bohrschablonen und die ein oder zwei Positionierungselemente hergestellt. Beispielsweise werden die Bohrschablonen und Positionierungselemente mit einem 3D-Drucker gedruckt oder mittels eines spanabhebenden Verfahrens aus einem Rohling geschnitten.

In Block 418 werden die hergestellten ein oder zwei Bohrschablonen und die ein oder zwei Positionierungselemente in einem Mundraum eines Patienten angeordnet und mittels Drucks, welchen der Patient mit seinen Kiefern ausübt vorläufig fixiert.

In Block 420 werden beispielsweise Fixierungsbohrungen unter Verwendung der entsprechenden Bohrschablone in einen oder beide Kiefer des Patienten eingebracht. In diese Fixierungsbohrungen werden Fixierungselemente eingebracht, um die entsprechenden Bohrschablone unabhängig von einem mit den Kiefern aufgebrachten Druck an den vordefinierten Positionen zu fixieren für welche die Implantatpositionen festgelegt wurden.

In Block 422 werden die ein oder zwei Positionierungselemente, falls sie lösbare Verbindungen zu den Bohrschablonen aufweisen, aus dem Mundraum des Patienten entfernt und die Implantatbohrungen werden unter Verwendung der mittels den Fixierungsbohrungen an den Kiefern fixierten Bohrschablonen an den geplanten Positionen in die Kiefer eingebracht.

Falls die Positionierungselemente von den Bohrschablonen umfasst und nicht für eine Entnahme aus dem Mundraum des Patienten vorgesehen sind, könnten die Implantatbohrungen auch ohne Entnahme der Positionierungselemente eingebracht werden. Hierzu weisen die Positionierungselemente beispielsweise Zugangsöffnungen auf, welche einen Zugang zu den Durchgangsöffnungen der Bohrschablonen zum Einbringen der Implantatbohrungen bereitstellen. Beispielsweise könnten die Implantatbohrungen in diesem Fall auch ohne vorangehende Fixierung mittels Fixierungselementen, d.h. ohne den Schritt gemäß Block 420, eingebracht werden, wobei die Bohrschablonen mittels des Drucks der Kiefer in der korrekten Position fixiert werden.

Beispielsweise umfassen das Bissregistrat, die Bohrschablone und/oder das Positionierungselement einen Druckmesser und/oder eine Druckanzeige. Dies könnte den Vorteil haben, das während des ersten Scan-Verfahrens der von den Kiefern des Patienten auf das Bissregistrat aufgebrachte Druck gemessen und beispielsweise auf einen vordefinierten Wert eingestellt. Ferner könnte dieser während des ersten Scan-Verfahren aufgebrachte Druck unter Verwendung des Positionierungselements exakt simuliert bzw. nachgestellt werden. Somit kann beispielsweise während des Einbringens der Fixierungsbohrungen derselbe Druck der Kiefer auf die Bohrschablone eingestellt werden, unter welchem auch die Abdrücke mit dem Bissregistrats genommen wurden.

Beispielsweise umfasst das Bissregistrat und/oder das Positionierungselement ein Material, welches druckabhängige Verformungseigenschaften und/oder Farbeigenschaften aufweist, anhand derer ein auf das Bissregistrat und/oder Positionierungselement ausgeübter Druck abgelesen werden kann.

Dies könnte den Vorteil haben, dass sie eine Fixierung der Bohrschablone in einer vordefinierten Position zum Einbringen von Fixierungsbohrungen ermöglichen, welche exakt der Position entspricht, für welche die Knochenstruktur des Kiefers erfasst und die Bohrschablone geplant wurde. Insbesondere könnte der Druck auf die weiche Gingiva exakt an die Druckverhältnisse angepasst werden, unter welchen die Abdrücke genommen wurden.

Dies könnte den Vorteil haben, dass eine vorläufige druckbasierte Fixierung der Bohrschablone in einer Position auf der weichen Gingiva des Kiefers ermöglicht wird, welche exakt der Position des Bissregistrats auf der Gingiva während des ersten Scan-Verfahrens entspricht. In dieser Position können beispielsweise die Fixierungsbohrungen eingebracht werden.

Die druckabhängige Fixierung der Bohrschablone erfolgt dabei beispielsweise unter Verwendung eines entgegengesetzten Drucks auf die Bohrschablone in Richtung auf die Schablonenauflagefläche und die Kontaktfläche der Gingiva. Dieser Druck resultiert beispielsweise aus einem Druck, welcher durch die beiden Kiefer auf die Bohrschablone ausgeübt wird.

Die druckunabhängige Fixierung erfolgt beispielsweise unter Verwendung von Fixierungselementen, welche in die Fixierungsbohrungen eingebracht werden. Diese druckunabhängige Fixierung erlaubt beispielsweise ein Öffnen der Kiefer für ein Einbringen der Implantatbohrungen, ohne dass die Bohrschablone verrutscht. Dies könnte den Vorteil haben, dass insbesondere die ursprüngliche Position des Bissregistrat bei Aufnahme der Auflagefläche berücksichtigt wird. Ausführungsformen können mithin den Vorteil haben, dass falsche Positionierungen vermieden werden können.

Somit könnte die Position des ursprünglichen Bissregistrats simuliert bzw. reproduziert werden, obwohl nicht mehr das Bissregistrat, sondern die Bohrschablone bzw. die Bohrschablonen in Kombination mit ein oder zwei Positionierungselementen verwendet werden. Eine solche Simulation des Bissregistrats unter Verwendung eines Positionierungselements kann bewirken, dass nicht nur die Winkel der Auflagefläche der Bohrschablone relativ zu dem Kieferknochen exakt den Winkeln der mit dem Bissregistrat erfassten Auflagefläche relativ zu dem Kieferknochen entsprechen, sondern dass zudem Größe und Richtung des Drucks auf das weiche Gewebe der Gingiva zur druckabhängigen Fixierung der Bohrschablone während des Einbringens der Fixierungsbohrungen identisch oder zumindest sehr ähnlich zu dem Druck eingestellt bzw. nachgestellt werden kann, mit welchem der Abdruck des Bissregistrats während des ersten Scan-Verfahrens genommen wurde.

Dies könnte den Vorteil haben, dass Verformungen des Weichgewebes der Kiefer, z.B. der Gingiva, während des Einbringens der Fixierungsbohrungen identisch oder zumindest sehr ähnlich zu Verformungen während des ersten Scan-Verfahrens und dem Erstellen des Bissregistrats nachgebildet werden können.

Dies könnte den Vorteil haben, dass ein einfaches und präzises Nachstellen räumlichen Verhältnisse der Kiefer ermöglicht wird, unter welchen die Abdrücke mit dem Bissregistrat genommen und das erste Scan-Verfahren ausgeführt wurde.

Dies könnte den Vorteil haben, dass hierfür nur eine geringe Anzahl an beispielsweise ein, zwei, drei oder vier Elemente notwendig sind, d.h. beispielsweise ein bis zwei Bohrschablonen und ein bis zwei Positionierungselemente, wobei die Positionierungselemente von den Bohrschablonen umfasst oder mit diesen lösbar verbunden sein können.

Diese Elemente simulieren in Kombination miteinander das Bissregistrat. Dabei können dieselbe Druck- und Positionsverhältnisse nachgestellt werden, unter denen das Bissregistrat erstellt und das erste Scan-Verfahren zur Erfassung der Strukturdaten ausgeführt wurde. Dies könnte den Vorteil haben, dass die Fixierungsbohrungen für die Fixierung der Bohrschablone an dem Kiefer des Patienten mit hoher Präzision an der geplanten Position des Kiefers des Patienten ausgeführt werden können.

### Bezugszeichenliste

- 10: Computersystem
- 11: Herstellungsvorrichtung
- 24: Bildschirm
- 50: Nutzerschnittstelle
- 52: Steuerelement
- 54: Hardwarekomponente
- 56: externes Gerät
- 58: externes Gerät
- 60: 3D-Drucker
- 62: Druckelement
- 64: Bearbeitungsgerät
- 65: Bearbeitungswerkzeug
- 66: Haltevorrichtung
- 67: Rohling
- 68: Material
- 70: Scan-Vorrichtung
- 72: Scan-Vorrichtung
- 120: Kieferknochen
- 121: Gegenkieferknochen
- 122: Gingiva
- 123: Gingiva
- 124: Gingivaoberfläche
- 125: Gingivaoberfläche
- 126: dentales Objekt
- 130: Bissregistrat
- 132: Abdruck
- 133: Abdruck
- 134: Marker
- 136: Druckerfassungselement
- 137: Druckanzeigeelement
- 140: Scan-Daten Kieferknochen
- 141: Scan-Daten Gegenkieferknochen
- 142: Scan-Daten Marker
- 144: Scan-Daten dentales Objekt
- 150: Scan-Daten Bissregistrat
- 152: Scan-Daten Marker
- 154: Scan-Daten Abdruck
- 155: Scan-Daten Abdruck
- 160: digitales Modell Kiefer/Bissregistrat
- 170: digitales Modell Positionierungselement/Bohrschablone
- 200: Positionierungselement
- 201: Positionierungselement
- 202: Kontaktfläche
- 203: Kontaktfläche
- 204: Verbindungselement
- 205: Verbindungselement
- 206: Verbindungselement
- 207: Verbindungselement
- 208: Verbindungselement
- 209: Verbindungselement
- 210: Zugangsöffnung
- 211: Zugangsöffnung
- 212: Kontaktfläche
- 214: Verbindungselement
- 216: Druckerfassungselement
- 217: Druckanzeigeelement
- 218: Kontaktfläche
- 219: Kontaktfläche
- 220: Bohrschablone
- 221: Bohrschablone
- 222: Schablonenkontaktfläche
- 223: Schablonenkontaktfläche
- 224: Durchgangsöffnung
- 225: Durchgangsöffnung
- 226: Durchgangsöffnung
- 227: Durchgangsöffnung
- 228: Verbindungselement
- 229: Verbindungselement
- 230: Bohrschablonenoberfläche
- 231: Bohrschablonenoberfläche

## Patentansprüche

1. Verfahren zum Herstellen eines ersten Positionierungselements (200) zum Fixieren einer ersten Bohrschablone (220) auf einer Gingiva (122) eines Kiefers eines Patienten in einer ersten Position relativ zu einem Kieferknochen (120) des Kiefers,
wobei das erste Positionierungselement (200) konfiguriert ist, die erste Bohrschablone (220) mittels Drucks, welchen der Patient durch ein Schließen des Kiefers und eines Gegenkiefers auf das erste Positionierungselement (200) aufbringt, in der ersten Position zu fixieren zum Einbringen von ersten Implantatbohrungen in den Kiefer für erste dentale Implantate,
wobei die erste Bohrschablone (220) eine erste Schablonenkontaktfläche (222) zum Herstellen eines Kontakts mit einer Oberfläche (124) der Gingiva (122) des Kiefers und erste Durchgangsöffnungen (224) umfasst, wobei die ersten Durchgangsöffnungen (224) jeweils eine erste Implantatposition zum Einbringen einer der ersten Implantatbohrungen definieren,
wobei das Verfahren umfasst:
• Erfassen von ersten Formdaten (154) eines ersten Abdrucks (132) der Oberfläche (124) der Gingiva (122) des Kiefers unter Verwendung eines zwischen den beiden Kiefern des Patienten angeordneten Bissregistrats (130), wobei sich die beiden Kiefer in einer Erfassungsposition relativ zueinander befinden, wobei sich der erste Abdruck (132) in der ersten Position relativ zu dem Kieferknochen (120) befindet und wobei der erste Abdruck (132) die erste Schablonenkontaktfläche (222) definiert,
• Erfassen eines auf das zwischen den beiden Kiefern des Patienten angeordnete Bissregistrat (130) aufgebrachten Erfassungsdrucks,
• Erfassen von ersten Strukturdaten (140) des Kieferknochens (120), während das Bissregistrat (130) zwischen den beiden Kiefern in der Erfassungsposition angeordnet ist,
• Erfassen von Positionsdaten des Bissregistrats (130) relativ zu dem Kieferknochen (120), während das Bissregistrat (130) zwischen den beiden Kiefern in der Erfassungsposition angeordnet ist,
• Erstellen eines ersten dreidimensionalen digitalen Modells (160) unter Verwendung der erfassten ersten Formdaten (154), ersten Strukturdaten (140) und Positionsdaten, wobei das erste Modell (160) die Kieferknochenstruktur (140) und die Oberfläche (124) der Gingiva (122) des Kiefers umfasst,
• Festlegen der ersten Implantatpositionen zum Einbringen der ersten Implantatbohrungen in den Kiefer unter Verwendung des ersten Modells (160),
• Erstellen eines zweiten dreidimensionalen digitalen Modells (170) der ersten Bohrschablone (220) und des ersten Positionierungselements (200) unter Verwendung des ersten Modells (160) und der festgelegten ersten Implantatpositionen, wobei Form und Abmessungen des ersten Positionierungselements (200) so bestimmt werden, dass die beiden Kiefer beim Aufbringen des Erfassungsdrucks auf das erste Positionierungselement (200) in der Erfassungsposition und die erste Bohrschablone (220) in der ersten Position relativ zu dem Kieferknochen (120) angeordnet werden,
• Herstellen des ersten Positionierungselements (200) unter Verwendung des zweiten dreidimensionalen digitalen Modells (170), wobei das erste Positionierungselement (200) ein Druckerfassungselement (216) umfasst zum Prüfen, ob ein in der Erfassungsposition der beiden Kiefer auf das erste Positionierungselement (200) aufgebrachter Druck mit dem Erfassungsdruck übereinstimmt.

2. Verfahren nach Anspruch 1, wobei es sich bei dem Kiefer des Patienten um einen zahnlosen Kiefer handelt, und/oder
wobei das erste Positionierungselement (200) konfiguriert ist, die erste Bohrschablone (220) in der ersten Position zum Einbringen von ersten Fixierungsbohrungen in den Kiefer für erste Fixierungselemente zu fixieren,
wobei die erste Bohrschablone (220) ferner zweite Durchgangsöffnungen (226) umfasst, wobei die zweiten Durchgangsöffnungen (226) jeweils eine erste Fixierungsposition zum Einbringen einer der ersten Fixierungsbohrungen definieren, wobei die ersten Fixierungselemente konfiguriert sind zum kieferdruckunabhängigen Fixieren der ersten Bohrschablone (220) in der ersten Position zum Einbringen der ersten Implantatbohrungen in den Kiefer für die ersten dentalen Implantate,
wobei das Verfahren ferner ein Festlegen der ersten Fixierungspositionen zum Einbringen der ersten Fixierungsbohrungen in den Kiefer unter Verwendung des ersten Modells (160) umfasst und das zweite dreidimensionale digitale Modell (170) der ersten Bohrschablone (220) und des ersten Positionierungselements (200) ferner unter Verwendung festgelegten ersten Fixierungspositionen erstellt wird, und/oder
wobei das Verfahren ferner ein Herstellen der ersten Bohrschablone (220) unter Verwendung des zweiten dreidimensionalen digitalen Modells (170) umfasst.

3. Verfahren nach einem der vorangehenden Ansprüche, wobei das erste Positionierungselement (200) ein oder mehrere erste Verbindungselemente (204) umfasst, welche konfiguriert sind zum Herstellen und Lösen einer zerstörungsfrei lösbaren ersten Verbindung zwischen dem ersten Positionierungselement (200) und der ersten Bohrschablone (220) im Mundraum des Patienten, oder
wobei die erste Bohrschablone (220) das erste Positionierungselement (200) umfasst, wobei das erste Positionierungselement (200) erste Zugangsöffnungen (210) zu den ersten Durchgangsöffnungen (224) der ersten Bohrschablone (220) umfasst.

4. Verfahren nach einem der vorangehenden Ansprüche, wobei unter Verwendung des zwischen den beiden Kiefern in der Erfassungsposition angeordneten Bissregistrats (130) zweite Formdaten (155) eines zweiten Abdrucks (133) einer Oberfläche des Gegenkiefers erfasst werden, welche zum Erstellen des ersten dreidimensionalen digitalen Modells (160) verwendet werden, welches ferner die zweite Oberfläche des Gegenkiefers umfasst.

5. Verfahren nach Anspruch 4, wobei das erste Positionierungselement (200) eine Kieferkontaktfläche (218) zum Herstellen eines Kontakts mit der Oberfläche des Gegenkiefers umfasst, wobei der zweite Abdruck (133) die Kieferkontaktfläche (218) definiert.

6. Verfahren nach Anspruch 4, wobei das Verfahren ferner ein Erfassen von zweiten Strukturdaten (141) eines Gegenkieferknochens (121) des Gegenkiefers, während das Bissregistrat (130) zwischen den beiden Kiefern in der Erfassungsposition angeordnet ist, umfasst,
wobei die zweiten Strukturdaten (141) zum Erstellen des ersten dreidimensionalen digitalen Modells (160) verwendet werden, welches ferner die Gegenkieferknochenstruktur (141) umfasst,
wobei das Verfahren ferner ein Herstellen einer zweiten Bohrschablone (221) umfasst, welche eine zweite Schablonenkontaktfläche (223) zum Herstellen eines Kontakts mit der Oberfläche des Gegenkiefers umfasst, wobei der zweite Abdruck (133) die zweite Schablonenkontaktfläche (223) definiert, wobei die Oberfläche des Gegenkiefers eine Oberfläche (125) der Gingiva (123) des Gegenkiefers ist,
wobei die zweite Bohrschablone (221) ferner dritte Durchgangsöffnungen (225) umfasst, wobei die dritten Durchgangsöffnungen (225) jeweils eine zweite Implantatposition zum Einbringen einer zweiten Implantatbohrung in den Gegenkiefer für zweite dentale Implantate definieren,
wobei die erfassten Positionsdaten Positionsdaten des Bissregistrats (130) relativ zu dem Gegenkieferknochen (121) umfassen,
wobei das Verfahren ferner ein Festlegen der zweiten Implantatpositionen zum Einbringen der zweiten Implantate in den Gegenkiefer unter Verwendung des ersten Modells (160) umfasst,
wobei das zweite Modell (170) die zweite Bohrschablone (221) umfasst und wobei zum Erstellen des zweiten Modells (170) ferner die festgelegten zweiten Implantatpositionen verwendet werden.

7. Verfahren nach Anspruch 6, wobei es sich bei dem Gegenkiefer des Patienten um einen zahnlosen Gegenkiefer handelt, und/oder
wobei das erste Positionierungselement (200) ferner konfiguriert ist, die zweite Bohrschablone (221) in der zweiten Position mittels Drucks, welchen der Patient durch ein Schließen der beiden Kiefer auf das erste Positionierungselement (200) aufbringt, zu fixieren, wobei Form und Abmessungen des ersten Positionierungselements (200) ferner so bestimmt sind, dass die zweite Bohrschablone (221) in der zweiten Position relativ zum Gegenkiefer angeordnet wird.

8. Verfahren nach einem der Ansprüche 6 bis 7, wobei das erste Positionierungselement (200) konfiguriert ist, die zweite Bohrschablone (220) in der zweiten Position zum Einbringen von zweiten Fixierungsbohrungen in den Gegenkiefer für zweite Fixierungselemente zu fixieren,
wobei die zweite Bohrschablone (221) ferner vierte Durchgangsöffnungen (227) umfasst, wobei die vierten Durchgangsöffnungen (227) jeweils eine zweite Fixierungsposition zum Einbringen einer der zweiten Fixierungsbohrungen definieren, wobei die zweiten Fixierungselemente konfiguriert sind zum kieferdruckunabhängigen Fixieren der zweiten Bohrschablone (221) in einer zweiten Position relativ zum Gegenkieferknochen (121) zum Einbringen der zweiten Implantatbohrungen in den Gegenkiefer für die zweiten dentalen Implantate,
wobei das Verfahren ferner ein Festlegen der zweiten Fixierungspositionen zum Einbringen der zweiten Fixierungselemente in den Gegenkiefer unter Verwendung des ersten Modells (160) umfasst,
wobei zum Erstellen des zweiten Modells (170) ferner die festgelegten zweiten Fixierungspositionen verwendet werden.

9. Verfahren nach Anspruch 8, wobei das erste Positionierungselement (200) ferner konfiguriert ist, die zweite Bohrschablone (221) in der zweiten Position zum Einbringen der zweiten Fixierungsbohrungen in den Gegenkiefer für die zweiten Fixierungselemente zu fixieren.

10. Verfahren nach einem der Ansprüche 8 bis 9, wobei das erste Positionierungselement (200) ferner ein oder mehrere zweite Verbindungselemente (214) umfasst, welche konfiguriert sind zum Herstellen und Lösen einer zerstörungsfrei lösbaren zweiten Verbindung zwischen dem ersten Positionierungselement (200) und der zweiten Bohrschablone (221) im Mundraum des Patienten, oder
wobei die zweite Bohrschablone (221) das erste Positionierungselement (200) umfasst, wobei das erste Positionierungselement (200) zweite Zugangsöffnungen (211) zu den dritten Durchgangsöffnungen (225) der zweiten Bohrschablone (221) umfasst,
wobei die erste und zweite Bohrschablone (220, 221) das erste Positionierungselement (200) beispielsweise gemeinsam umfassen.

11. Verfahren nach einem der Ansprüche 6 bis 7, wobei das Herstellen der zweite Bohrschablone (221) ferner ein Herstellen eines zweiten Positionierungselements (201) umfasst, wobei das zweite Positionierungselement (201) in Kombination mit dem ersten Positionierungselement (200) konfiguriert ist, die zweite Bohrschablone (221) in der zweiten Position mittels Drucks, welchen der Patient durch ein Schließen der Kiefer auf das erste und zweite Positionierungselement (200, 201) aufbringt, zu fixieren, wobei Form und Abmessungen des zweiten Positionierungselements (201) so bestimmt werden, dass die beiden Kiefer beim Aufbringen des Drucks auf das erste und zweite Positionierungselement (200, 201) in der Erfassungsposition und die zweite Bohrschablone (221) in der zweiten Position relativ zum Gegenkiefer angeordnet werden,
wobei das zweite Positionierungselement (201) beispielsweise ein oder mehrere dritte Verbindungselemente (205) umfasst, welche konfiguriert sind zum Herstellen und Lösen einer zerstörungsfrei lösbaren dritten Verbindung zwischen dem zweiten Positionierungselement (201) und der zweiten Bohrschablone (221) im Mundraum des Patienten.

12. Verfahren nach einem der Ansprüche 6 bis 11, wobei die zweiten Implantatpositionen der zweiten Implantatbohrungen und/oder zweiten Fixierungspositionen der zweiten Fixierungsbohrungen jeweils unter Erfüllung eines oder mehrerer der folgenden ersten Positionierungskriterien festgelegt werden: Erstrecken der zweiten Implantatbohrungen und/oder zweiten Fixierungsbohrungen jeweils durch eine Knochenwand der Gegenkieferknochenstruktur (121) mit einer vordefinierten ersten Mindeststärke, Einhalten jeweils eines vordefinierten ersten Mindestabstands der zweiten Implantatbohrungen und/oder zweiten Fixierungsbohrungen von Hauptnerven im Gegenkiefer, Einhalten jeweils eines vordefinierten zweiten Mindestabstands der zweiten Implantatbohrungen und/oder zweiten Fixierungsbohrungen von Adern im Gegenkiefer, und/oder
wobei die zweite Bohrschablone (221) für die dritten Durchgangsöffnungen (225) und/oder vierten Durchgangsöffnungen (227) jeweils mit einem Bohrführungselement versehen ist, welches dazu konfiguriert ist, ein Bohrwerkzeug beim Einbringen der zweiten Implantatbohrungen und/oder zweiten Fixierungsbohrungen zu führen und/oder eine erste Bohrtiefe der zweiten Implantatbohrungen und/oder zweiten Fixierungsbohrungen zu begrenzen, und/oder
wobei das erste und/oder das zweite Positionierungselement (200; 201) für die dritten Durchgangsöffnungen (225) und/oder vierten Durchgangsöffnungen (227) jeweils mit einem dritten Bohrführungselement versehen sind, welches dazu konfiguriert ist, das Bohrwerkzeug beim Einbringen der zweiten Implantatbohrungen und/oder zweiten Fixierungsbohrungen zu führen und/oder die erste Bohrtiefe der zweiten Implantatbohrungen und/oder zweiten Fixierungsbohrungen zu begrenzen, und/oder
wobei das zweite Positionierungselement (201) ein Druckerfassungselement (216) umfasst zum Prüfen, ob der in der Erfassungsposition der beiden Kiefer auf das zweite Positionierungselement (201) aufgebrachte Druck mit dem Erfassungsdruck übereinstimmt.

13. Verfahren nach einem der vorangehenden Ansprüche, wobei die ersten Implantatpositionen der ersten Implantatbohrungen und/oder ersten Fixierungspositionen der ersten Fixierungsbohrungen jeweils unter Erfüllung eines oder mehrerer der folgenden zweiten Positionierungskriterien festgelegt werden: Erstrecken der ersten Implantatbohrungen und/oder ersten Fixierungsbohrungen jeweils durch eine Knochenwand der Kieferknochenstruktur (120) mit einer vordefinierten zweiten Mindeststärke, Einhalten jeweils eines vordefinierten dritten Mindestabstands der ersten Implantatbohrungen und/oder ersten Fixierungsbohrungen von Hauptnerven im Kiefer, Einhalten jeweils eines vordefinierten vierten Mindestabstands der ersten Implantatbohrungen und/oder ersten Fixierungsbohrungen von Adern im Kiefer, und/oder
wobei die erste Bohrschablone (220) für die ersten Durchgangsöffnungen (224) und/oder zweiten Durchgangsöffnungen (226) jeweils mit einem dritten Bohrführungselement versehen ist, welches dazu konfiguriert ist, das Bohrwerkzeug beim Einbringen der ersten Implantatbohrungen und/oder ersten Fixierungsbohrungen zu führen und/oder eine zweite Bohrtiefe der ersten Implantatbohrungen und/oder ersten Fixierungsbohrungen zu begrenzen, und/oder
wobei das erste Positionierungselement (200) für die ersten Durchgangsöffnungen (224) und/oder zweiten Durchgangsöffnungen (226) jeweils mit einem vierten Bohrführungselement versehen ist, welches dazu konfiguriert ist, das Bohrwerkzeug beim Einbringen der ersten Implantatbohrungen und/oder ersten Fixierungsbohrungen zu führen und/oder die zweite Bohrtiefe der ersten Implantatbohrungen und/oder ersten Fixierungsbohrungen zu begrenzen, und/oder
wobei die ersten und/oder zweiten Strukturdaten (140; 141) unter Verwendung eines ersten Scan-Verfahren erfasst werden, bei welchem es sich um eines der folgenden Verfahren handelt: ein Computertomographie-Verfahren, ein Volumentomographie-Verfahren oder ein Magnetresonanztomographie-Verfahren,
wobei die Positionsdaten des Bissregistrats (130) beispielsweise unter Verwendung von Markern (134) erfasst werden, welche das Bissregistrat (130) umfasst und welche opak für das erste Scan-Verfahren sind, und/oder
wobei die ersten und/oder zweiten Formdaten (155) unter Verwendung eines zweiten Scan-Verfahren erfasst werden, bei welchem es sich um eines der folgenden Verfahren handelt: ein optisches Verfahren, ein Computertomographie-Verfahren, ein haptisches Verfahren.

14. Kombination aus einer ersten Bohrschablone (220) und einem Positionierungselement (200) zum Fixieren der ersten Bohrschablone (220) auf einer Gingiva (122) eines Kiefers eines Patienten in einer ersten Position relativ zu einem Kieferknochen (120) des Kiefers,
wobei das Positionierungselement (200) konfiguriert ist, die erste Bohrschablone (220) mittels Drucks, welchen der Patient durch ein Schließen des Kiefers und eines Gegenkiefers auf das Positionierungselement (200) aufbringt, in der ersten Position zu fixieren zum Einbringen von ersten Implantatbohrungen in den Kiefer für erste dentale Implantate,
wobei die Kombination hergestellt ist unter Verwendung eines Verfahrens, welches umfasst:
• Erfassen von ersten Formdaten (154) eines ersten Abdrucks (132) der Oberfläche (124) der Gingiva (122) des Kiefers unter Verwendung eines zwischen den beiden Kiefern des Patienten angeordneten Bissregistrats (130), wobei sich die beiden Kiefer in einer Erfassungsposition relativ zueinander befinden, wobei sich der erste Abdruck (132) in der ersten Position relativ zu dem Kieferknochen (120) befindet und wobei der erste Abdruck (132) eine erste Schablonenkontaktfläche (222) der ersten Bohrschablone (220) zum Herstellen eines Kontakts mit einer Oberfläche (124) der Gingiva (122) des Kiefers definiert,
• Erfassen eines auf das zwischen den beiden Kiefern des Patienten angeordnete Bissregistrat (130) aufgebrachten Erfassungsdrucks,
• Erfassen von ersten Strukturdaten (140) des Kieferknochens (120), während das Bissregistrat (130) zwischen den beiden Kiefern in der Erfassungsposition angeordnet ist,
• Erfassen von Positionsdaten des Bissregistrats (130) relativ zu dem Kieferknochen (120), während das Bissregistrat (130) zwischen den beiden Kiefern in der Erfassungsposition angeordnet ist,
• Erstellen eines ersten dreidimensionalen digitalen Modells (160) unter Verwendung der erfassten ersten Formdaten (154), ersten Strukturdaten (140) und Positionsdaten, wobei das erste Modell (160) die Kieferknochenstruktur (140) und die Oberfläche (124) der Gingiva (122) des Kiefers umfasst,
• Festlegen erster Implantatpositionen zum Einbringen der ersten Implantatbohrungen in den Kiefer unter Verwendung des ersten Modells (160),
• Erstellen eines zweiten dreidimensionalen digitalen Modells (170) der ersten Bohrschablone (220) und des Positionierungselements (200) unter Verwendung des ersten Modells (160) und der festgelegten ersten Implantatpositionen, wobei Form und Abmessungen des Positionierungselements (200) so bestimmt werden, dass die beiden Kiefer beim Aufbringen des Erfassungsdrucks auf das Positionierungselement (200) in der Erfassungsposition und die erste Bohrschablone (220) in der ersten Position relativ zu dem Kieferknochen (120) angeordnet werden, wobei die erste Bohrschablone (220) die erste Schablonenkontaktfläche (222) und erste Durchgangsöffnungen (224) umfasst, wobei die ersten Durchgangsöffnungen (224) jeweils eine der ersten Implantatpositionen definieren,
wobei das zweite dreidimensionale digitale Modell (170) zum Herstellen des Positionierungselements (200) und der ersten Bohrschablone (220) verwendet wird,
wobei das Positionierungselement (200) ein Druckerfassungselement (216) umfasst zum Prüfen, ob ein in der Erfassungsposition der beiden Kiefer auf das Positionierungselement (200) aufgebrachter Druck mit dem Erfassungsdruck übereinstimmt.

15. Kombination nach Anspruch 14, welche ferner eine zweite Bohrschablone (221) umfasst, welche eine zweite Schablonenkontaktfläche (223) zum Herstellen eines Kontakts mit der Oberfläche des Gegenkiefers umfasst, wobei der zweite Abdruck (133) die zweite Schablonenkontaktfläche (223) definiert, wobei die Oberfläche des Gegenkiefers eine Oberfläche der Gingiva (125) des Gegenkiefers (123) ist,
wobei die zweite Bohrschablone (221) ferner dritte Durchgangsöffnungen (225) umfasst, wobei die dritten Durchgangsöffnungen (225) jeweils eine zweite Implantatposition zum Einbringen einer der zweiten Implantatbohrungen definieren.

## Claims

1. A method for producing a first positioning element (200) for fixing a first drilling template (220) on a gingiva (122) of a patient's jaw in a first position relative to a jawbone (120) of the jaw,
wherein the first positioning element (200) is configured to fix the first drilling template (220), by means of pressure, which the patient applies to the first positioning element (200) by closing the jaw and an opposing jaw, in the first position for making first implant holes in the jaw for first dental implants,
wherein the first drilling template (220) comprises a first template contact surface (222) for making contact with a surface (124) of the gingiva (122) of the jaw and comprises first through-openings (224), wherein the first through-openings (224) each define a first implant position for making one of the first implant holes,
wherein the method comprises:
• recording first shape data (154) of a first impression (132) of the surface (124) of the gingiva (122) of the jaw, using a bite register (130) arranged between the patient's two jaws, wherein the two jaws are located in a recording position relative to each other, wherein the first impression (132) is located in the first position relative to the jawbone (120), and wherein the first impression (132) defines the first template contact surface (222),
• sensing a grip pressure applied to the bite register (130) arranged between the two jaws of the patient,
• recording first structure data (140) of the jawbone (120) while the bite register (130) is arranged between the two jaws in the recording position,
• recording position data of the bite register (130) relative to the jawbone (120) while the bite register (130) is arranged between the two jaws in the recording position,
• creating a first three-dimensional digital model (160), using the recorded first shape data (154), first structure data (140) and position data, wherein the first model (160) comprises the jawbone structure (140) and the surface (124) of the gingiva (122) of the jaw,
• defining the first implant positions for making the first implant holes in the jaw, using the first model (160),
• creating a second three-dimensional digital model (170) of the first drilling template (220) and of the first positioning element (200), using the first model (160) and the defined first implant positions, wherein the shape and dimensions of the first positioning element (200) are determined such that, when pressure is applied to the first positioning element (200), the two jaws are arranged in the recording position and the first drilling template (220) is arranged in the first position relative to the jawbone (120),
• producing the first positioning element (200), using the second three-dimensional digital model (170), wherein the first positioning element (200) comprises a pressure sensing element (216) for checking whether a pressure applied to the first positioning element (200) in the recording position of the two jaws matches the grip pressure.

2. The method according to claim 1, wherein the patient's jaw is a toothless jaw and/or
wherein the first positioning element (200) is configured to fix the first drilling template (220) in the first position for making first fixing holes in the jaw for first fixing elements,
wherein the first drilling template (220) further comprises second through-openings (226), wherein the second through-openings (226) each define a first fixing position for making one of the first fixing holes, wherein the first fixing elements are configured to fix the first drilling template (220), in a jaw pressure-independent manner, in the first position for making the first implant holes in the jaw for the first dental implants,
wherein the method further comprises defining the first fixing positions for making the first fixing holes in the jaw, using the first model (160), and the second three-dimensional digital model (170) of the first drilling template (220) and of the first positioning element (200) is also created using defined first fixing positions, and/or
wherein the method further comprises a production of the first drilling template (220) using the second three-dimensional digital model (170).

3. The method according to any one of the preceding claims, wherein the first positioning element (200) comprises one or more first connecting elements (204), which are configured to establish and to release a non-destructively releasable first connection between the first positioning element (200) and the first drilling template (220) in the patient's oral cavity, or
wherein the first drilling template (220) comprises the first positioning element (200), wherein the first positioning element (200) comprises first access openings (210) to the first through-openings (224) of the first drilling template (220).

4. The method according to any one of the preceding claims, wherein second shape data (155) of a second impression (133) of a surface of the opposing jaw are recorded, using the bite register (130) arranged between the two jaws in the recording position, said second shape data being used to create the first three-dimensional digital model (160), which further comprises the second surface of the opposing jaw.

5. The method according to claim 4, wherein the first positioning element (200) comprises a jaw contact surface (218) for making contact with the surface of the opposing jaw, wherein the second impression (133) defines the jaw contact surface (218).

6. The method according to claim 4, wherein the method further comprises recording second structure data (141) of an opposing jawbone (121) of the opposing jaw while the bite register (130) is arranged between the two jaws in the recording position,
wherein the second structure data (141) are used to create the first three-dimensional digital model (160), which further comprises the opposing jawbone structure (141),
wherein the method further comprises producing a second drilling template (221) which comprises a second template contact surface (223) for making contact with the surface of the opposing jaw, wherein the second impression (133) defines the second template contact surface (223), wherein the surface of the opposing jaw is a surface (125) of the gingiva (123) of the opposing jaw,
wherein the second drilling template (221) further comprises third through-openings (225), wherein the third through-openings (225) each define a second implant position for making one of the second implant holes in the opposing jaw for second dental implants,
wherein the recorded position data comprise position data of the bite register (130) relative to the opposing jawbone (121),
wherein the method further comprises defining the second implant positions for inserting the second implants into the opposing jaw, using the first model (160),
wherein the second model (170) comprises the second drilling template (221), and wherein the defined second implant positions are also used to create the second model (170).

7. The method according to claim 6, wherein the patient's opposing jaw is a toothless opposing jaw and/or
wherein the first positioning element (200) is further configured to fix the second drilling template (221) in the second position by means of pressure, which the patient applies to the first positioning element (200) by closing the two jaws, wherein the shape and dimensions of the first positioning element (200) are further determined such that the second drilling template (221) is arranged in the second position relative to the opposing jaw.

8. The method according to any one of claims 6 to 7, wherein the first positioning element (200) is configured to fix the second drilling template (220) in the second position for making second fixing holes in the opposing jaw for second fixing elements,
wherein the second drilling template (221) further comprises fourth through-openings (227), wherein the fourth through-openings (227) each define a second fixing position for making one of the second fixing holes, wherein the second fixing elements are configured to fix the second drilling template (221), in a jaw pressure-independent manner, in a second position relative to the opposing jawbone (121) for making second implant holes in the opposing jaw for second dental implants,
wherein the method further comprises defining the second fixing positions for inserting the second fixing elements in the opposing jaw, using the first model (160),
wherein the defined second fixing positions are also used to create the second model (170).

9. The method according to claim 8, wherein the first positioning element (200) is further configured to fix the second drilling template (221) in the second position for making the second fixing holes in the opposing jaw for the second fixing elements.

10. The method according to any one of claims 8 to 9, wherein the first positioning element (200) further comprises one or more second connecting elements (214), which are configured to establish and to release a non-destructively releasable second connection between the first positioning element (200) and the second drilling template (221) in the patient's oral cavity, or
wherein the second drilling template (221) comprises the first positioning element (200), wherein the first positioning element (200) comprises second access openings (211) to the third through-openings (225) of the second drilling template (221),
wherein the first and second drilling template (220, 221) exemplarily jointly comprise the first positioning element (200).

11. The method according to any one of claims 6 to 7, wherein the production of the second drilling template (221) further comprises producing a second positioning element (201), wherein the second positioning element (201) in combination with the first positioning element (200) is configured to fix the second drilling template (221) in the second position by means of pressure, which the patient applies to the first and second positioning element (200, 201) by closing the jaws, wherein the shape and dimensions of the second positioning element (201) are determined such that, when the pressure is applied to the first and second positioning element (200, 201), the two jaws are arranged in the recording position and the second drilling template (221) is arranged in the second position relative to the opposing jaw,
wherein the second positioning element (201) exemplarily comprises one or more third connecting elements (205), which are configured to establish and to release a non-destructively releasable third connection between the second positioning element (201) and the second drilling template (221) in the patient's oral cavity.

12. The method according to any one of claims 6 to 11, wherein the second implant positions of the second implant holes and/or second fixing positions of the second fixing holes are each defined in such a way as to satisfy one or more of the following first positioning criteria: extending the second implant holes and/or second fixing holes through a bone wall of the opposing jawbone structure (121) having a predefined first minimum thickness, maintaining a predefined first minimum distance of the second implant holes and/or second fixing holes from main nerves in the opposing jaw, maintaining a predefined second minimum distance of the second implant holes and/or second fixing holes from blood vessels in the opposing jaw, and/or
wherein the second drilling template (221) for the third through-openings (225) and/or fourth through-openings (227) is provided in each case with a drilling guide element, which is configured to guide a drilling tool when making the second implant holes and/or second fixing holes and/or to limit a first drilling depth of the second implant holes and/or second fixing holes, and/or
wherein the first and/or the second positioning element (200; 201) for the third through-openings (225) and/or fourth through-openings (227) are provided in each case with a third drilling guide element, which is configured to guide the drilling tool when making the second implant holes and/or second fixing holes and/or to limit the first drilling depth of the second implant holes and/or second fixing holes, and/or
wherein the second positioning element (201) comprises a pressure sensing element (216) for checking whether a pressure applied to the second positioning element (201) in the recording position of the two jaws matches the grip pressure.

13. The method according to any one of the preceding claims, wherein the first implant positions of the first implant holes and/or first fixing positions of the first fixing holes are each defined in such a way as to satisfy one or more of the following second positioning criteria: extending the first implant holes and/or first fixing holes through a bone wall of the jawbone structure (120) having a predefined second minimum thickness, maintaining a predefined third minimum distance of the first implant holes and/or first fixing holes from main nerves in the jaw, maintaining a predefined fourth minimum distance of the first implant holes and/or first fixing holes from blood vessels in the jaw, and/or
wherein the first drilling template (220) for the first through-openings (224) and/or second through-openings (226) is provided in each case with a third drilling guide element, which is configured to guide the drilling tool when making the first implant holes and/or first fixing holes and/or to limit a second drilling depth of the first implant holes and/or first fixing holes and/or
wherein the first positioning element (200) for the first through-openings (224) and/or second through-openings (226) is provided in each case with a fourth drilling guide element, which is configured to guide the drilling tool when making the first implant holes and/or first fixing holes and/or to limit the second drilling depth of the first implant holes and/or first fixing holes, and/or
wherein the first and/or second structure data (140; 141) are recorded using a first scanning method, which is one of the following methods: a computed tomography method, a volumetric tomography method or a magnetic resonance imaging method,
wherein the position data of the bite register (130) are exemplarily recorded using markers (134), which are comprised by the bite register (130) and are opaque for the first scanning method, and/or
wherein the first and/or second shape data (155) are recorded using a second scanning method, which is one of the following methods: an optical method, a computed tomography method, a haptic method.

14. A combination of a first drilling template (220) and a positioning element (200) for fixing the first drilling template (220) on a gingiva (122) of a patient's jaw in a first position relative to a jawbone (120) of the jaw,
wherein the positioning element (200) is configured to fix the first drilling template (220), by means of pressure, which the patient applies to the positioning element (200) by closing the jaw and an opposing jaw, in the first position for making first implant holes in the jaw for first dental implants,
wherein the combination is produced using a method which comprises:
• recording first shape data (154) of a first impression (132) of the surface (124) of the gingiva (122) of the jaw, using a bite register (130) arranged between the patient's two jaws, wherein the two jaws are located in a recording position relative to each other, wherein the first impression (132) is located in the first position relative to the jawbone (120), and wherein the first impression (132) defines a first template contact surface (222) of a first drilling template (220) for making contact with a surface (124) of the gingiva (122) of the jaw,
• sensing a grip pressure applied to the bite register (130) arranged between the patient's two jaws,
• recording first structure data (140) of the jawbone (120) while the bite register (130) is arranged between the two jaws in the recording position,
• recording position data of the bite register (130) relative to the jawbone (120) while the bite register (130) is arranged between the two jaws in the recording position,
• creating a first three-dimensional digital model (160), using the recorded first shape data (154), first structure data (140) and position data, wherein the first model (160) comprises the jawbone structure (140) and the surface (124) of the gingiva (122) of the jaw,
• defining first implant positions for making the first implant holes in the jaw, using the first model (160),
• creating a second three-dimensional digital model (170) of the first drilling template (220) and of the positioning element (200), using the first model (160) and the defined first implant positions, wherein the shape and dimensions of the positioning element (200) are determined such that, when pressure is applied to the positioning element (200), the two jaws are arranged in the recording position and the first drilling template (220) is arranged in the first position relative to the jawbone (120), wherein the first drilling template (220) comprises the first template contact surface (222) and first through-openings (224), wherein the first through-openings (224) each define one of the first implant positions,
wherein the second three-dimensional digital model (170) is used to produce the positioning element (200) and the first drilling template (220), wherein the positioning element (200) comprises a pressure sensing element (216) for checking whether a pressure applied to the positioning element (200) in the recording position of the two jaws matches the grip pressure.

15. The combination according to claim 14, further comprising a second drilling template (221) which comprises a second template contact surface (223) for making contact with the surface of the opposing jaw, wherein the second impression (133) defines the second template contact surface (223), wherein the surface of the opposing jaw is a surface of the gingiva (125) of the opposing jaw (123),
wherein the second drilling template (221) further comprises third through-openings (225), wherein the third through-openings (225) each define a second implant position for making one of the second implant holes.

## Revendications

1. Procédé de fabrication d'un premier élément de positionnement (200) permettant la fixation d'un premier gabarit de perçage (220) sur une gencive (122) d'un maxillaire d'un patient dans une première position par rapport à un os de maxillaire (120) du maxillaire,
dans lequel le premier élément de positionnement (200) est conçu pour fixer le premier gabarit de perçage (220) au moyen d'une pression, laquelle exerce le patient par une fermeture du maxillaire et du maxillaire complémentaire sur le premier élément de positionnement (200), dans la première position, pour la mise en place de premiers perçages d'implant dans le maxillaire destinés à des premiers implants dentaires,
dans lequel le premier gabarit de perçage (220) comprend une première surface de contact de gabarit (222) permettant d'établir un contact avec une surface (124) de la gencive (122) du maxillaire et des premiers orifices de passage (224), où les premiers orifices de passage (224) définissent respectivement une première position d'implant permettant la mise en place d'un des premiers perçages d'implant,
le procédé comprenant :
• la détection de premières données de forme (154) d'une première empreinte (132) de la surface (124) de la gencive (122) du maxillaire moyennant l'emploi d'un enregistrement d'occlusion (130) disposé entre les deux maxillaires du patient, où les deux maxillaires se trouvent dans une position de détection l'un par rapport à l'autre, où la première empreinte (132) se trouve dans la première position par rapport à l'os de maxillaire (120) et où la première empreinte (132) définit la première surface de contact de gabarit (222),
• la détection d'une pression de détection exercée sur l'enregistrement d'occlusion (130) disposé entre les deux maxillaires du patient,
• la détection de premières données de structure (140) de l'os de maxillaire (120) pendant que l'enregistrement d'occlusion (130) est disposé dans la position de détection entre les deux maxillaires,
• la détection de données de position de l'enregistrement d'occlusion (130) par rapport à l'os de maxillaire (120) pendant que l'enregistrement d'occlusion (130) est disposé dans la position de détection entre les deux maxillaires,
• l'élaboration d'un premier modèle numérique tridimensionnel (160) moyennant l'emploi des premières données de forme (154), de premières données de structure (140) et de données de position détectées, où le premier modèle (160) comprend la structure de l'os de maxillaire (140) et la surface (124) de la gencive (122) du maxillaire,
• l'établissement des premières positions d'implant pour la mise en place des premiers perçages d'implant dans le maxillaire moyennant l'emploi du premier modèle (160),
• l'élaboration d'un deuxième modèle numérique tridimensionnel (170) du premier gabarit de perçage (220) et du premier élément de positionnement (200) moyennant l'emploi du premier modèle (160) et des premières positions d'implant établies, où la forme et les dimensions du premier élément de positionnement (200) sont déterminées de telle manière que les deux maxillaires, lors de l'exercice de la pression de détection, se trouve disposés sur le premier élément de positionnement (200) dans la position de détection et le premier gabarit de perçage (220) dans la première position par rapport à l'os de maxillaire (120),
• l'élaboration du premier élément de positionnement (200) moyennant l'emploi du deuxième modèle numérique tridimensionnel (170), où le premier élément de positionnement (200) comprend un élément de détection de pression (216) permettant de vérifier si une pression exercée sur le premier élément de positionnement (200) lors de la position de détection des deux maxillaires correspond avec la pression de détection.

2. Procédé selon la revendication 1, dans lequel, dans le cas du maxillaire du patient il s'agit d'un maxillaire dépourvu de dents, et/ou
dans lequel le premier élément de positionnement (200) est conçu pour fixer le premier gabarit de perçage (220) dans la première position pour la mise en place de premiers perçages de fixation dans le maxillaire pour des premiers éléments de fixation,
dans lequel le premier gabarit de perçage (220) comprend en outre des deuxièmes orifices de passage (226), où les deuxièmes orifices de passage (226) définissent respectivement une première position de fixation pour la mise en place d'un des premiers perçages de fixation, où les premiers éléments de fixation sont conçus pour la fixation indépendante de la pression des maxillaires du premier gabarit de perçage (220) dans la première position pour la mise en place des premiers perçages d'implant dans les maxillaires pour les premiers implants dentaires,
le procédé comprenant en outre un établissement des premières positions de fixation permettant la mise en place des premiers perçages de fixation dans les maxillaires moyennant l'emploi du premier modèle (160) et le deuxième modèle numérique tridimensionnel (170) du premier gabarit de perçage (220) et du premier élément de positionnement (200) est établi en outre moyennant l'emploi de premières positions de positionnement déterminées, et/ou
le procédé comprenant en outre l'élaboration du premier gabarit de perçage (220) moyennant l'emploi du deuxième modèle numérique tridimensionnel (170).

3. Procédé selon l'une des revendications précédentes, dans lequel le premier élément de positionnement (200) comprend un ou plusieurs premiers éléments de liaison (204), lesquels sont conçus pour l'établissement et le détachement d'une première liaison amovible sans destruction entre le premier élément de positionnement (200) et le premier gabarit de perçage (220) dans l'espace buccal du patient, ou
dans lequel le premier gabarit de perçage (220) comprend le premier élément de positionnement (200), où le premier élément de positionnement (200) comprend des premiers orifices d'accès (210) vers les premiers orifices de passage (224) du premier gabarit de perçage (220).

4. Procédé selon l'une des revendications précédentes, dans lequel, moyennant l'emploi de l'enregistrement d'occlusion (130) disposé entre les deux maxillaires dans la position de détection, des deuxièmes données de forme (155) d'une deuxième empreinte (133) d'une surface du maxillaire complémentaire sont détectées, lesquelles sont employées pour l'établissement du premier modèle numérique tridimensionnel (160), lequel comprend en outre la deuxième surface du maxillaire complémentaire.

5. Procédé selon la revendication 4, dans lequel le premier élément de positionnement (200) comprend une surface de contact de maxillaire (218) permettant d'établir un contact avec la surface du maxillaire complémentaire, où la deuxième empreinte (133) définit la surface de contact du maxillaire (218).

6. Procédé selon la revendication 4, le procédé comprenant en outre une détection de deuxièmes données de structure (141) d'un os de maxillaire complémentaire (121) du maxillaire complémentaire pendant que l'enregistrement d'occlusion (130) est disposé entre les deux maxillaires dans la position de détection,
dans lequel les deuxièmes données de structure (141) sont employées pour l'élaboration du premier modèle numérique tridimensionnel (160), lequel comprend en outre la structure de l'os de maxillaire complémentaire (141),
le procédé comprenant en outre l'élaboration d'un deuxième gabarit de perçage (221), lequel comprend une deuxième surface de contact de gabarit (223) permettant l'établissement d'un contact avec la surface du maxillaire complémentaire, où la deuxième empreinte (133) définit la deuxième surface de contact de gabarit (223), où la surface du maxillaire complémentaire est une surface (125) de la gencive (123) du maxillaire complémentaire,
dans lequel le deuxième gabarit de perçage (221) comprend en outre des troisièmes orifices de passage (225), où les troisièmes orifices de passage (225) définissent respectivement une deuxième position d'implant pour la mise en place d'un deuxième perçage d'implant dans le maxillaire complémentaire pour des deuxièmes implants dentaires,
dans lequel les données de position détectées comprennent des données de position de l'enregistrement d'occlusion (130) par rapport à l'os du maxillaire complémentaire (121),
le procédé comprenant en outre un établissement des deuxièmes positions d'implant pour la mise en place des deuxièmes implants dans le maxillaire complémentaire moyennant l'emploi du premier modèle (160),
dans lequel le deuxième modèle (170) comprend le deuxième gabarit de perçage (221) et dans lequel les deuxièmes positions d'implant déterminées sont en outre employées pour l'élaboration du deuxième modèle (170).

7. Procédé selon la revendication 6, dans lequel, dans le cas du maxillaire complémentaire du patient, il s'agit d'un maxillaire complémentaire dépourvu de dents, et/ou
dans lequel le premier élément de positionnement (200) est en outre conçu pour fixer le deuxième gabarit de perçage (221) dans la deuxième position au moyen d'une pression, laquelle exerce le patient par une fermeture des deux maxillaires sur le premier élément de positionnement (200), dans lequel la forme et les dimensions du premier élément de positionnement (200) sont en outre déterminées de telle manière que le deuxième gabarit de perçage (221) est disposé dans la deuxième position par rapport au maxillaire complémentaire.

8. Procédé selon l'une des revendications 6 à 7, dans lequel le premier élément de positionnement (200) est conçu de manière à fixer le deuxième gabarit de perçage (220) dans la deuxième position, permettant la mise en place de deuxièmes perçages de fixation dans le maxillaire complémentaire pour des deuxièmes éléments de fixation,
dans lequel le deuxième gabarit de perçage (221) comprend en outre des quatrièmes orifices de passage (227), où les quatrièmes orifices de passage (227) définissent respectivement une deuxième position de fixation pour la mise en place d'un des deuxièmes perçages de fixation, où les deuxièmes éléments de fixation sont conçus pour la fixation indépendante de la pression des maxillaires du deuxième gabarit de perçage (221) dans une deuxième position par rapport à l'os de maxillaire complémentaire (121) pour la mise en place des deuxièmes perçages d'implant dans le maxillaire complémentaire pour des deuxièmes implants dentaires,
le procédé comprenant en outre un établissement des deuxièmes positions de fixation pour la mise en place des deuxièmes éléments de fixation dans le maxillaire complémentaire moyennant l'emploi du premier modèle (160),
dans lequel, pour l'élaboration du deuxième modèle (170), on emploie en outre les deuxièmes positions de fixation déterminées.

9. Procédé selon la revendication 8, dans lequel le premier élément de positionnement (200) est en outre conçu pour fixer le deuxième gabarit de perçage (221) dans la deuxième position pour la mise en place des deuxièmes perçages de fixation dans le maxillaire complémentaire pour les deuxièmes éléments de fixation.

10. Procédé selon l'une des revendications 8 à 9, dans lequel le premier élément de positionnement (200) comprend en outre un ou plusieurs deuxièmes éléments de liaison (214), lesquels sont conçus pour l'établissement et le détachement d'une deuxième liaison amovible sans destruction entre le premier élément de positionnement (200) et le deuxième gabarit de perçage (221) dans l'espace buccal du patient, ou
dans lequel le deuxième gabarit de perçage (221) comprend le premier élément de positionnement (200), où le premier élément de positionnement (200) comprend des deuxièmes orifices d'accès (211) vers les troisièmes orifices de passage (225) du deuxième gabarit de perçage (221),
dans lequel les premier et deuxième gabarits de perçage (220, 221) comprennent le premier élément de positionnement (200), par exemple, conjointement.

11. Procédé selon l'une des revendications 6 à 7, dans lequel l'élaboration du deuxième gabarit de perçage (221) comprend en outre l'élaboration d'un deuxième élément de positionnement (201), où le deuxième élément de positionnement (201) est conçu en combinaison avec le premier élément de positionnement (200), pour fixer le deuxième gabarit de perçage (221) dans la deuxième position au moyen d'une pression, laquelle le patient exerce par une fermeture des maxillaires sur les premier et deuxième éléments de positionnement (200, 201), où la forme et les dimensions du deuxième élément de positionnement (201) sont déterminées de telle manière que les deux maxillaires, lors de l'exercice de la pression sur les premier et deuxième éléments de positionnement (200, 201) sont disposés dans la position de détection et le deuxième gabarit de perçage (221) est disposé dans la deuxième position par rapport au maxillaire complémentaire,
dans lequel le deuxième élément de positionnement (201) comprend par exemple un ou plusieurs troisièmes éléments de liaison (205), lesquels sont conçus pour l'établissement et le détachement d'une troisième liaison amovible sans destruction entre le deuxième élément de positionnement (201) et le deuxième gabarit de perçage (221) dans l'espace buccal du patient.

12. Procédé selon l'une des revendications 6 à 11, dans lequel les deuxièmes positions d'implant des deuxièmes perçages d'implant, et/ou deuxièmes positions de fixation des deuxièmes perçages de fixation, sont déterminées respectivement moyennant la satisfaction à un ou plusieurs des premiers critères de position suivants : une extension des deuxièmes perçages d'implant et/ou des deuxièmes perçages de fixation respectivement à travers une paroi osseuse de structure du maxillaire complémentaire (121) avec une première résistance minimale prédéfinie, le maintien respectivement d'une première distance minimale prédéfinie des deuxièmes perçages d'implant et/ou des deuxièmes perçages de fixation par rapport à des nerfs principaux dans le maxillaire complémentaire, le maintien respectivement d'une deuxième distance minimale prédéfinie des deuxièmes perçages d'implant et/ou des deuxièmes perçages de fixation par rapport à des vaisseaux dans le maxillaire complémentaire, et/ou
dans lequel le deuxième gabarit de perçage (221) est muni respectivement d'un élément de guidage de perçage pour les troisièmes orifices de passage (225) et/ou les quatrièmes orifices de passage (227), lequel est conçu pour guider un outil de perçage lors de l'élaboration des deuxièmes perçages d'implant et/ou des deuxièmes perçages de fixation et/ou pour limiter une première profondeur de perçage des deuxièmes perçages d'implant et/ou des deuxièmes perçages de fixation, et/ou
dans lequel les premier et/ou deuxième éléments de positionnement (200 ; 201) pour les troisièmes orifices de passage (225) et/ou quatrièmes orifices de passage (227) sont respectivement munis d'un troisième élément de guidage de perçage, lequel est conçu pour guider l'outil de perçage lors de l'élaboration des deuxièmes perçages d'implant et/ou des deuxièmes perçages de fixation et/ou pour limiter la première profondeur de perçage des deuxièmes perçages d'implant et/ou des deuxièmes perçages de fixation, et/ou
dans lequel le deuxième élément de positionnement (201) comprend un élément de détection de la pression (216) pour vérifier si la pression exercée sur le deuxième élément de positionnement (201) dans la position de détection des deux maxillaires correspond à la pression de détection.

13. Procédé selon l'une des revendications précédentes, dans lequel les premières positions d'implant des premiers perçages d'implant et/ou premières positions de fixation des premiers perçages de fixation sont déterminées respectivement moyennant la satisfaction à un ou plusieurs des deuxièmes critères de position suivants : une extension des premiers perçages d'implant et/ou des premiers perçages de fixation respectivement à travers une paroi osseuse de structure de maxillaire (120) avec une deuxième résistance minimale prédéfinie, le maintien respectivement d'une troisième distance minimale prédéfinie des premiers perçages d'implant et/ou des premiers perçages de fixation par rapport à des nerfs principaux dans le maxillaire, le maintien respectivement d'une quatrième distance minimale prédéfinie des premiers perçages d'implant et/ou des premiers perçages de fixation par rapport à des vaisseaux dans le maxillaire, et/ou
dans lequel le premier gabarit de perçage (220) est muni respectivement d'un troisième élément de guidage de perçage pour les premiers orifices de passage (224) et/ou les deuxièmes orifices de passage (226), lequel est conçu pour guider un outil de perçage lors de l'élaboration des premiers perçages d'implant et/ou des premiers perçages de fixation et/ou pour limiter une deuxième profondeur de perçage des premiers perçages d'implant et/ou des premiers perçages de fixation, et/ou
dans lequel le premier élément de positionnement (200) pour les premiers orifices de passage (224) et/ou deuxièmes orifices de passage (226) est respectivement muni d'un quatrième élément de guidage de perçage, lequel est conçu pour guider l'outil de perçage lors de l'élaboration des premiers perçages d'implant et/ou des premiers perçages de fixation et/ou pour limiter la deuxième profondeur de perçage des premiers perçages d'implant et/ou des premiers perçages de fixation, et/ou
dans lequel les premières et/ou les deuxièmes données de structure (140 ; 141) sont détectées moyennant l'emploi d'un premier procédé de balayage, lors duquel il s'agit d'un des procédés suivants : un procédé de tomodensitométrie, d'un procédé de tomographie volumique ou d'un procédé de tomographie par résonance magnétique,
dans lequel les données de position de l'enregistrement d'occlusion (130) sont détectées par exemple moyennant l'emploi de marqueurs (134), lesquels sont compris dans l'enregistrement d'occlusion (130) et lesquels sont opaques pour le premier procédé de balayage, et/ou
dans lequel les premières et/u deuxièmes données de forme (155) sont détectées moyennant l'emploi d'un deuxième procédé de balayage, lors duquel il s'agit d'un des procédés suivants : un procédé optique, un procédé de tomodensitométrie, un procédé haptique.

14. Combinaison à base d'un premier gabarit de perçage (220) et d'un élément de positionnement (200) permettant la fixation d'un premier gabarit de perçage (220) sur une gencive (122) d'un maxillaire d'un patient dans une première position par rapport à un os de maxillaire (120) du maxillaire,
dans lequel l'élément de positionnement (200) est conçu pour fixer le premier gabarit de perçage (220) au moyen d'une pression, laquelle exerce le patient par une fermeture du maxillaire et du maxillaire complémentaire sur le premier élément de positionnement (200), dans la première position, permettant la mise en place de premiers perçages d'implant dans les maxillaires pour des premiers implants dentaires,
la combinaison étant élaborée moyennant l'emploi d'un procédé, lequel comprend :
• la détection de premières données de forme (154) d'une première empreinte (132) de la surface (124) de la gencive (122) du maxillaire moyennant l'emploi d'un enregistrement d'occlusion (130) disposé entre les deux maxillaires du patient, où les deux maxillaires se trouvent dans une position de détection l'un par rapport à l'autre, où la première empreinte (132) se trouve dans la première position par rapport à l'os de maxillaire (120) et où la première empreinte (132) définit une première surface de contact de gabarit (222) du premier gabarit de perçage (220) pour l'établissement d'un contact avec une surface (124) de la gencive (122) du maxillaire,
• la détection d'une pression de détection exercée sur l'enregistrement d'occlusion (130) disposé entre les deux maxillaires du patient,
• la détection de premières données de structure (140) de l'os de maxillaire (120) pendant que l'enregistrement d'occlusion (130) est disposé dans la position de détection entre les deux maxillaires,
• la détection de données de position de l'enregistrement d'occlusion (130) par rapport à l'os de maxillaire (120) pendant que l'enregistrement d'occlusion (130) est disposé dans la position de détection entre les deux maxillaires,
• l'élaboration d'un premier modèle numérique tridimensionnel (160) moyennant l'emploi des premières données de forme (154), de premières données de structure (140) et de données de position détectées, où le premier modèle (160) comprend la structure de l'os de maxillaire (140) et la surface (124) de la gencive (122) du maxillaire,
• l'établissement des premières positions d'implant permettant la mise en place des premiers perçages d'implant dans le maxillaire moyennant l'emploi du premier modèle (160),
• l'élaboration d'un deuxième modèle numérique tridimensionnel (170) du premier gabarit de perçage (220) et du premier élément de positionnement (200) moyennant l'emploi du premier modèle (160) et des premières positions d'implant établies, où la forme et les dimensions du premier élément de positionnement (200) sont déterminées de telle manière que les deux maxillaires, lors de l'exercice de la pression de détection sur le premier élément de positionnement (200), sont disposés sur le premier élément de positionnement dans la position de détection et le premier gabarit de perçage (220) est disposé dans la première position par rapport à l'os de maxillaire (120), où le premier gabarit de perçage (220) comprend la première surface de contact de gabarit (222) et des premiers orifices de passage (224), où les premiers orifices de passage (224) définissent respectivement une des premières positions d'implant,
dans lequel le deuxième modèle numérique tridimensionnel (170) est employé pour l'élaboration de l'élément de positionnement (200) et du premier gabarit de perçage (220),
dans lequel l'élément de positionnement (200) comprend un élément de détection de la pression (216) pour vérifier si une pression exercée dans la position de détection des deux maxillaires sur l'élément de positionnement (200) correspond à la pression de détection.

15. Combinaison selon la revendication 14, laquelle comprend en outre un deuxième gabarit de perçage (221), lequel comprend une deuxième surface de contact de gabarit (223) pour l'établissement d'un contact avec la surface du maxillaire complémentaire, où la deuxième empreinte (133) définit la deuxième surface de contact de gabarit (223), où la surface du maxillaire complémentaire est une surface de la gencive (125) du maxillaire complémentaire (123),
dans laquelle le deuxième gabarit de perçage (221) comprend en outre des troisièmes orifices de passage (225), où les troisièmes orifices de passage (225) définissent respectivement une deuxième position d'implant pour la mise en place des deuxièmes perçages d'implant.
